(19) Europäisches Patentamt
European Patent Office
Office européen des brevets

(11) **EP 4 303 322 A1**

(12) **EUROPEAN PATENT APPLICATION**
published in accordance with Art. 153(4) EPC


(43) Date of publication:
**10.01.2024 Bulletin 2024/02**

(21) Application number: **21928566.5**

(22) Date of filing: **05.03.2021**

(51) International Patent Classification (IPC):
***C12Q 1/6886*** *(2018.01)* ***G01N 33/68*** *(2006.01)*
***A61K 31/675*** *(2006.01)* ***A61P 35/00*** *(2006.01)*

(52) Cooperative Patent Classification (CPC):
**A61K 31/675; A61P 35/00; C12Q 1/6886; G01N 33/68**

(86) International application number:
**PCT/CN2021/079299**

(87) International publication number:
**WO 2022/183483 (09.09.2022 Gazette 2022/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**



(71) Applicant: **Ascentawits Pharmaceuticals, Ltd.**
**Shenzhen, Guangdong 518118 (CN)**

(72) Inventors:
- **XIE, Yanbin**
  **Shenzhen, Guangdong 518118 (CN)**
- **HAO, Jing**
  **Shenzhen, Guangdong 518118 (CN)**
- **WANG, Ning**
  **Shenzhen, Guangdong 518118 (CN)**
- **LIU, Nan**
  **Shenzhen, Guangdong 518118 (CN)**

(74) Representative: **Nederlandsch Octrooibureau**
**P.O. Box 29720**
**2502 LS The Hague (NL)**


Remarks:
The complete document including Reference Table(s) and the Sequence Listing(s) can be downloaded from the EPO website

(54) **PRIMER-PROBE COMPOSITION, KIT, AND DETECTION METHOD**


(57) The present invention relates to a primer-probe composition, a kit, and a detection method. The primer-probe composition is selected from one group of group (i) to group (ix), and the kit comprises the primer-probe composition. According to the present invention, the detection of AKRIC3 RNA content in an *ex vivo* sample of a patient can be achieved.


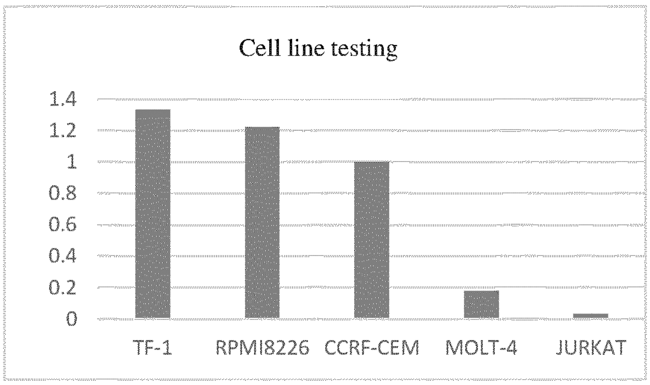


Fig.11

EP 4 303 322 A1

## Description

### TECHNICAL FIELD

The present invention relates to the field of molecular biology detection technology, especially to a primer-probe composition, a kit, and a detection method.

### BACKGROUND

Aldehyde-ketone reductase family 1 member C3 (AKR1C3) is an enzyme encoded by the AKR1C3 gene in humans. In various types of cancer, AKR1C3 is overexpressed at the protein level. Due to the high expression of AKR1C3 in tumors, compounds OBI-3424 (also referred as AST-3424 and TH-3424), OBI-3423 and OBI-2870 are designed to be specifically activated in tumors, but cannot be activated in normal cells expressing low level of AKR1C3 in order to achieve tumor-specific targeting. Compound OBI-3424 is currently being studied in multiple phase I clinical trials in the United States (NCT04315324 and NCT03592264) and China (CXHL1900137 and CXHL2000263) for treating more than 14 types of human cancers, including solid and hematological tumors.

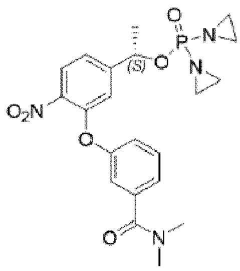

3424

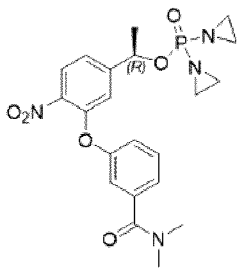

3423

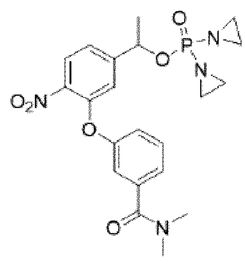

2870

Compound OBI-3424 is a novel prodrug dialkylating agent activated by AKR1C3. Studies have confirmed that compound OBI-3424 activation is AKR1C3-dependent, and its cytotoxicity and antitumor efficacy are highly correlated with the expression level of AKR1C3 enzyme. Compound OBI-3424 shows AKR1C3-dependent cytotoxicity *ex vivo* and antitumor activity *in vivo* in a variety of human cancer types, which supports the further development of compound OBI-3424 as an anticancer agent that can be used to treat different types of cancers. AKR1C3 is used as a biomarker to analyze the condition of cancer patients and further guides patients to choose compound OBI-3424 for treatment. Therefore, in practical application, the AKR1C3 enzyme content in an *ex vivo* sample of a patient can be detected, and then it is determined whether to administrate the compound OBI-3424 to patient for treatment according to the detection results.

In the prior art, Western blotting or immunochemical staining (IHC) is usually used to detect AKR1C3 enzyme content in an *ex vivo* sample of a patient. In the reference (Harvey D J, Singleton R S, Dachs G U, et al., The Bioreductive Prodrug PR-104A Is Activated under Aerobic Conditions by Human Aldo-Keto Reductase 1C3[J]. Cancer Research, 2010, 70(4): 1573), 2700 tumor tissue samples were counted and analyzed by IHC method, wherein AKR1C3 enzyme was highly expressed in liver cancer, gastric cancer, esophageal cancer and bladder cancer, while it was low expressed in small cell lung cancer, breast cancer, leukemia and prostate cancer. Therefore, it is theoretically feasible to use IHC method to detect the expression level of AKR1C3 enzyme in solid tumor tissue samples.

However, there are many types of cancer or tumor, patient with hematological cancer (leukemia) other than solid tumor cannot provide tissue samples, so it is not possible to directly use western blotting or immunochemical staining to detect the expression level of AKR1C3 enzyme in hematological cancer (leukemia) sample.

### SUMMARY OF THE INVENTION

The applicant's R&D team has found that the expression level of AKR1C3 enzyme and AKR1C3 RNA content of different solid tumor cancer cells are significantly correlated with the $IC_{50}$ value of AST-3424 inhibition on cancer cell proliferation, and the expression level of AKR1C3 enzyme and the AKR1C3 RNA content of different hematological cancer cell lines are significantly correlated with the $IC_{50}$ value of AST-3424 inhibition on cancer cell proliferation. Therefore, the enzyme content can also be predicted or characterized by detecting the ARKR1C3 RNA content, thereby guiding the drug administration.

However, there is no corresponding detection method for detecting AKR1C3 RNA content in the prior art.

Assuming that a corresponding detection method can be used to assess AKR1C3 RNA content, patient with higher AKR1C3 RNA content and most likely to respond to the prodrug thus can be selected to administrate compound OBI-3424 for achieving better cancer treatment effect.

In view of this, the purpose of the present invention is to provide a primer-probe composition, a kit, and a detection method, so that the detection of AKRIC3 RNA content in an *ex vivo* sample of a patient can be achieved with good accuracy, analytical specificity, precision and low detection limit by using the primer-probe composition, the kit, and the detection method.

For the above purpose, the first aspect of the present invention provides a primer-probe composition for the detection of AKR1 C3 RNA content in an *ex vivo* sample of a patient, selected from any one of the following groups:

(i) upstream primer AKR1C3-F1, downstream primer AKR1C3-R1 and probe AKR1C3-P1, wherein the nucleotide sequences of the upstream primer AKR1C3-F1, the downstream primer AKR1C3-R1 and the probe AKR1C3-P1 are shown as SEQ ID NO:1, SEQ ID NO:2 and SEQ ID NO:3, respectively;

(ii) upstream primer AKR1C3-F2, downstream primer AKR1C3-R2 and probe AKR1C3-P1, wherein the nucleotide sequences of the upstream primer AKR1C3-F2, the downstream primer AKR1C3-R2 and the probe AKR1C3-P1 are shown as SEQ ID NO:4, SEQ ID NO:5 and SEQ ID NO:3, respectively;

(iii) upstream primer AKR1C3-F2, downstream primer AKR1C3-R6 and probe AKR1C3-P1, wherein the nucleotide sequences of the upstream primer AKR1C3-F2, the downstream primer AKR1C3-R6 and the probe AKR1C3-P1 are shown as SEQ ID NO:4, SEQ ID NO:6 and SEQ ID NO:3, respectively;

(iv) upstream primer AKR1C3-F6, downstream primer AKR1C3-R2 and probe AKR1C3-P1, wherein the nucleotide sequences of the upstream primer AKR1C3-F6, the downstream primer AKR1C3-R2 and the probe AKR1C3-P1 are shown as SEQ ID NO:7, SEQ ID NO:5 and SEQ ID NO:3, respectively;

(v) upstream primer AKR1C3-F6, downstream primer AKR1C3-R6 and probe AKR1C3-P1, wherein the nucleotide sequences of the upstream primer AKR1C3-F6, the downstream primer AKR1C3-R6 and the probe AKR1C3-P1 are shown as SEQ ID NO:7, SEQ ID NO:6 and SEQ ID NO:3, respectively;

(vi) upstream primer AKR1C3-F5, downstream primer AKR1C3-R5 and probe AKR1C3-P2, wherein the nucleotide sequences of the upstream primer AKR1C3-F5, the downstream primer AKR1C3-R5 and the probe AKR1C3-P2 are shown as SEQ ID NO:8, SEQ ID NO:9 and SEQ ID NO: 10, respectively;

(vii) upstream primer AKR1C3-F3, downstream primer AKR1C3-R3 and probe AKR1C3-P1, wherein the nucleotide sequences of the upstream primer AKR1C3-F3, the downstream primer AKR1C3-R3 and the probe AKR1C3-P1 are shown as SEQ ID NO: 11, SEQ ID NO: 12 and SEQ ID NO:3, respectively;

(viii) upstream primer AKR1C3-F4, downstream primer AKR1C3-R3 and probe AKR1C3-P2, wherein the nucleotide sequences of the upstream primer AKR1C3-F4, the downstream primer AKR1C3-R3 and the probe AKR1C3-P2 are shown as SEQ ID NO: 13, SEQ ID NO: 12 and SEQ ID NO: 10, respectively;

(ix) upstream primer AKR1C3-F7, downstream primer AKR1C3-R7 and probe AKR1C3-P3, wherein the nucleotide sequences of the upstream primer AKR1C3-F7, the downstream primer AKR1C3-R7 and the probe AKR1C3-P3 are shown as SEQ ID NO: 14, SEQ ID NO: 15 and SEQ ID NO: 16, respectively.

In a preferred embodiment of the present invention, wherein the primer-probe composition is selected from any one of groups (iii), (iv) and (v);

more preferably, the primer-probe composition is selected from group (iv).

In a preferred embodiment of the present invention, wherein the 5'-end reporters of the probes AKR1C3-P1, AKR1C3-P2 and AKR1C3-P3 are FAM, and the 3'-end quenchers of the probes AKR1C3-P1, AKR1C3-P2 and AKR1C3-P3 are MGB.

Based on the same inventive concept, the second aspect of the present invention provides a kit comprising the primer-probe composition as described above.

In a preferred embodiment of the present invention, wherein the kit further comprises a primer-probe composition of reference gene;

preferably, the reference gene is ACTB.

In a preferred embodiment of the present invention, wherein the primer-probe composition of reference gene

comprises upstream primer ACTB-F1, downstream primer ACTB-R1 and probe ACTB-P1, and the nucleotide sequences of the upstream primer ACTB-F1, the downstream primer ACTB-R1 and the probe ACTB-P1 are shown as SEQ ID NO: 17, SEQ ID NO: 18 and SEQ ID NO: 19, respectively.

In a preferred embodiment of the present invention, wherein the 5'-end reporter of the probe ACTB-P1 is VIC, and the 3'-end quencher of the probe ACTB-P1 is BHQ1.

In a preferred embodiment of the present invention, wherein the kit as described above further comprises a polymerase mixture, and the polymerase mixture mainly comprises: DNA polymerase, $MgCl_2$, buffer and dNTPs; preferably, the polymerase mixture is KAPA PROBE FAST RT-PCR Master Mix(2x).

In a preferred embodiment of the present invention, wherein the kit as described above further comprises a reverse transcriptase mixture;

preferably, the reverse transcriptase mixture is Superscript VII,O MARSTER MIX.

In a preferred embodiment of the present invention, wherein the kit as described above further comprises a negative control and a positive control;

preferably, the negative control is nuclease-free water; and/or
preferably, the positive control is a reference with a known copy number.

Based on the same inventive concept, the third aspect of the present invention provides use of the primer-probe composition or the kit as described above in the preparation of a drug for treating cancers.

In a preferred embodiment of the present invention, wherein the AKR1C3 RNA content in an *ex vivo* sample of a patient is obtained by using the primer-probe composition or the kit as described above;
patient with AKR1C3 RNA content greater than or equal to the predetermined content is administrated with AKR1C3 activated anticancer drugs.

In a preferred embodiment of the present invention, wherein the AKR1C3 RNA content is obtained according to the ratio of AKR1C3 copy number/reference gene copy number;

preferably, the predetermined content is 0.0001~1;

more preferably, the predetermined content is 0.00011~0.5;

even more preferably, the predetermined content is 0.00013~0.05.

In a preferred embodiment of the present invention, wherein the *ex vivo* sample comprises blood sample, bone marrow sample, or tissue sample.

In a preferred embodiment of the present invention, wherein the AKR1C3 activated anticancer drug of course includes the AKR1C3 activated anticancer prodrug in the present invention, i.e., the compounds in the form of prodrugs are reduced under the catalysis of AKR1C3 in the biochemical environment in the cells to obtain cytotoxic toxins, thereby exerting toxic effect on cancer cells., see the following patent applications:

PCT/US2016/021581, Publication No. WO2016145092A1, corresponding to Chinese Application No. 2016800150788, Publication No. CN107530556A;

PCT/US2016/025665, Publication No. WO2016161342, corresponding to Chinese Application No. 2016800446081, Publication No. CN108290911A;

PCT/US2016/062114, Publication No. WO2017087428, corresponding to Chinese Application No. 2016800200132, Publication No. CN108136214A; and

PCT/NZ2019/050030, Publication No. WO2019190331, corresponding to Chinese Application No. 201980023423.6, Publication No. CN111918864A.

The compounds of the general formula and specific compounds disclosed in the above patent applications all belong to AKR1C3 activated anticancer prodrugs (compounds), and the above applications are hereby incorporated into the present patent application text in their entirety.

Broadly speaking, AKR1C3 activated anticancer drug should meet the following conditions:

In the presence of an AKR1C3 inhibitor (such as TH-3021 disclosed in the three patents above, or compound 36, i.e.,

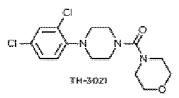

TH-3021

in Flanagan et al., Bioorganic and Medicinal Chemistry (2014), pp. 962-977), the inhibition effect detected of a compound on the proliferation of cancer cells is less than that of cancer cells in the absence of an AKR1C3 inhibitor (such as TH-3021 disclosed in the above three patents); and when the inhibition effect on cancer cell proliferation is quantified using the $IC_{50}$, then if the $IC_{50}$ detected of a compound on a certain cancer cell line in the presence of an AKR1C3 inhibitor is greater than that in the absence of an AKR1C3 inhibitor, then the compound can be determined to be an AKR1C3-activated anticancer drug.

preferably, the AKR1C3 activated anticancer drug is a compound selected from the compounds with the following structures:

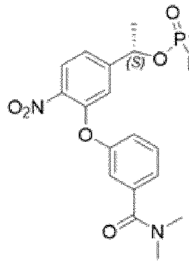
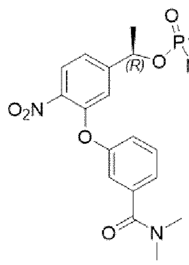
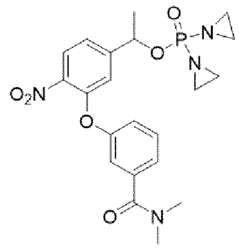
.

In a preferred embodiment of the present invention, wherein the cancers comprise:

lung cancer, non-small cell lung cancer, liver cancer, pancreatic cancer, breast cancer, gastric cancer, bone cancer, esophageal cancer, mastocarcinoma, prostate cancer, testicular cancer, colon cancer, ovarian cancer, bladder cancer, cervical cancer, hepatocellular carcinoma, melanoma, squamous cell carcinoma, basal cell carcinoma, adenocarcinoma, sweat gland carcinoma, sebaceous gland carcinoma, papillary carcinoma, papillary adenocarcinoma, renal cell carcinoma, cystic adenocarcinoma, cystic carcinoma, medullary carcinoma, bronchial carcinoma, osteocyte carcinoma, epithelial carcinoma, carcinoma of bile duct, choriocarcinoma, embryonal carcinoma, seminoma, Wilm's tumor, glioblastoma, astrocytoma, medulloblastoma, craniopharyngioma, ependymoma, pineal tumor, hemocytoblastoma, vocal cords neuroma, meningioma, neuroblastoma, optic neuroblastoma, retinoblastoma, neurofibroma, fibrosarcoma, fibroblastoma, fibroma, fibroadenoma, fibrochondroma, fibrocystoma, fibromyxoma, fibroosteoma, fibromyxosarcoma, fibropapilloma, myxosarcoma, myxocystoma, myxochondroma, myxochondrosarcoma, myxochondrofibrosarcoma, myxadenoma, myxoblastoma, liposarcoma, lipoma, lipoadenoma, lipoblastoma, lipochondroma, lipofibroma, lipoangioma, myxolipoma, chondrosarcoma, chondroma, chondromyoma, chordoma, choriocarcinoma, chorioepithelioma, chorioblastoma, osteosarcoma, osteoblastoma, osteochondrofibroma, osteochondrosarcoma, osteochondroma, osteocystoma, osteodentinoma, osteofibroma, fibrosarcoma of bone, angiosarcoma, hemangioma, angiolipoma, angiochondroma, hemangioblastoma, angiokeratoma, angioglioma, angioendothelioma, angiofibroma, angiomyoma, angiolipoma, angiolymphangioma, angiolipoleiomyoma, angiomyolipoma, angiomyoneuroma, angiomyxoma, angioreticuloma, lymphangiosarcoma, lymphogranuloma, lymphangioma, lymphoma, lymphomyxoma, lymphosarcoma, lymphangiofibroma, lymphocytoma, lymphoepithelioma, lymphoblastoma, peripheral T-cell lymphoma, nodular NK/T-cell lymphoma, endothelioma, endoblastoma, synovioma, synovial sarcoma, mesothelioma, connective tissue tumor, Ewing's tumor, leiomyoma, leiomyosarcoma, leiomyoblastoma, leiomyofibroma, rhabdomyoma, rhabdomyosarcoma, rhabdomyomyxoma, acute lymphatic leukemia, acute myelogenous leukemia, chronic disease cells, polycythemia, lymphoma, endometrial cancer, glioma, colorectal cancer, thyroid cancer, urothelial cancer or multiple myeloma;

preferably, the cancers comprise: ovarian cancer, cervical cancer, pancreatic cancer, breast cancer, colorectal cancer, esophageal cancer, stomach cancer, liver cancer, non-small cell lung cancer, prostate cancer, renal cell carcinoma, peripheral T-cell lymphoma, nodular NK/T-cell lymphoma, acute lymphoblastic leukemia or acute myelogenous leukemia.

Based on the same inventive concept, the fourth aspect of the present invention provides a method for detecting AKR1C3 RNA content, including the following steps:

(1) extracting RNA of an *ex vivo* sample to be detected and adding the extracted RNA to the reverse transcription system and the extracted RNA being reverse transcribed to synthesize cDNA;

(2) performing qPCR or digital PCR amplification with cDNA as template using the primer-probe composition or the kit as described above;

(3) obtaining AKR1C3 RNA content of *ex vivo* sample to be detected according to qPCR or digital PCR amplification results.

In a preferred embodiment of the present invention, wherein in step (1), the concentration of the extracted RNA is detected;

preferably, Qubit RNA HS Assay Kits are used to detect the concentration of the extracted RNA; and/or

the reverse transcription system comprises reverse transcriptase;

preferably, the mass-to-volume ratio of the extracted RNA and reverse transcriptase is (0.5~2):4, with the unit of μg/μL;

more preferably, the mass-to-volume ratio of the extracted RNA and reverse transcriptase is (1~1.8):4, with the unit of μg/μL;

even more preferably, the mass-to-volume ratio of the extracted RNA and reverse transcriptase is 2:4, with the unit of μg/μL.

In a preferred embodiment of the present invention, wherein in step (2), in qPCR reaction system, the primer-probe compositions selected from any one of groups (i) to (ix) is mixed with the primer-probe composition of reference gene;

preferably, in qPCR reaction system, the molar ratio of AKR1C3 upstream primer, AKR1C3 downstream primer and AKR1C3 probe is (2~10):(2~10):3;

more preferably, in qPCR reaction system, the molar ratio of AKR1C3 upstream primer, AKR1C3 downstream primer and AKR1C3 probe is (3~7):(3~7):3;

even more preferably, in qPCR reaction system, the molar ratio of AKR1C3 upstream primer, AKR1C3 downstream primer and AKR1C3 probe is 5:5:3; and/or, in qPCR reaction system,

preferably, the molar ratio of upstream primer ACTB-F1, downstream primer ACTB-R1 and probe ACTB-P1 is (2~10):(2~10):3;

more preferably, the molar ratio of upstream primer ACTB-F1, downstream primer ACTB-R1 and probe ACTB-P1 is (3~7):(3~7):3;

even more preferably, the molar ratio of upstream primer ACTB-F1, downstream primer ACTB-R1 and probe ACTB-P1 is 5:5:3; and/or, in qPCR reaction system,

preferably, the amount of AKR1C3 upstream primer, AKR1C3 downstream primer and AKR1C3 probe is the same as the amount of upstream primer ACTB-F1, downstream primer ACTB-R1 and probe ACTB-P1, respectively.

In a preferred embodiment of the present invention, wherein in step (2), in qPCR reaction system, dUTP, UNG enzyme, cDNA template and polymerase mixture are also added;

preferably, the volume of the polymerase mixture is (0.3~0.8) of the total volume of qPCR reaction system;

more preferably, the volume of the polymerase mixture is 0.5 of the total volume of qPCR reaction system.

In a preferred embodiment of the present invention, wherein in step (2), AKR1C3 and reference gene are

amplified by AKR1C3 digital PCR detection system and reference gene digital PCR detection system, respectively;

preferably, in AKR1C3 digital PCR detection system, the molar ratio of AKR1C3 upstream primer, AKR1C3 downstream primer and AKR1C3 probe is (5~15):(5~15):3;

more preferably, the molar ratio of AKR1C3 upstream primer, AKR1C3 downstream primer and AKR1C3 probe is (8~14):(8~14):3;

even more preferably, the molar ratio of AKR1C3 upstream primer, AKR1C3 downstream primer and AKR1C3 probe is 12:12:3; and/or, in the reference gene digital PCR detection system,

preferably, the molar ratio of upstream primer ACTB-F1, downstream primer ACTB-R1 and probe ACTB-P1 is (5~15):(5~15):3;

more preferably, the molar ratio of upstream primer ACTB-F1, downstream primer ACTB-R1 and probe ACTB-P1 is (8~14):(8~14):3;

even more preferably, the molar ratio of upstream primer ACTB-F1, downstream primer ACTB-R1 and probe ACTB-P1 is 12:12:3.

In a preferred embodiment of the present invention, wherein in step (3), the step for obtaining AKR1C3 RNA content of *ex vivo* sample to be detected according to qPCR or digital PCR amplification results includes:

(A) obtaining the copy numbers of AKR1C3 and reference gene *of ex vivo* sample to be detected according to qPCR or digital PCR amplification results, respectively;

(B) calculating the ratio of AKR1C3 copy number/reference gene copy number to obtain the AKR1C3 RNA content of *ex vivo* sample to be detected.

In a preferred embodiment of the present invention, wherein when the qPCR method is used, step (A) further includes:

($A_1$) plotting the standard curve of Ct value and initial copy number lg value of AKR1C3 and the standard curve of Ct value and initial copy number lg value of reference gene, respectively;

($A_2$) according to the standard curves, obtaining the copy numbers of AKR1C3 and reference gene of *ex vivo* sample to be detected by the detected Ct values of AKR1C3 and reference gene, respectively.

In a preferred embodiment of the present invention, wherein the above method is used to detect AKR1C3 RNA content in blood sample, bone marrow sample or tissue sample.

Based on the same inventive concept, the fifth aspect of the present invention provides a method for detecting the expression level of AKR1C3 enzyme, wherein the method is used to detect the AKR1C3 RNA content of *ex vivo* sample to be detected, and then the expression level of AKR1C3 enzyme of *ex vivo* sample to be detected is obtained according to the AKR1C3 RNA content of *ex vivo* sample to be detected.

In a preferred embodiment of the present invention, wherein there is a linear correlation between AKR1C3 RNA content *ex vivo* sample to be detected and the expression level of AKR1C3 enzyme *ex vivo* sample to be detected.

## BRIEF DESCRIPTION OF THE DRAWINGS

Fig.1 shows AKR1C3-dependent cytotoxicity of compound OBI-3424 *ex vivo;* the left figure shows the correlation between the expression level of AKR1C3 protein and OBI-3424 $IC_{50}$ in hepatocellular carcinoma cells; the middle figure shows the correlation between the expression level of AKR1C3 RNA and OBI-3424 $IC_{50}$ in hepatocellular carcinoma cells; and the right figure shows the correlation between the expression level of AKR1C3 RNA and OBI-3424 $IC_{50}$ in NSCLC cancer cells.

Fig.2 shows the cytotoxicity of compound OBI-3424 on leukemia cell lines; Fig.2a shows the cytotoxicity of compound

OBI-3424 on 6 types of B-ALL cell lines; Fig.2b shows the cytotoxicity of compound OBI-3424 on 7 types of T-ALL cell lines; Fig.2c shows the correlation between the expression level of AKR1C3 protein and the cell survival rate of 18 types of ALL PDX *ex vivo* at the concentration of 10 nmol/L of OBI-3424; and Fig.2d shows the correlation between the expression level of AKR1C3 protein and the cell survival rate of 18 types of ALL PDX *ex vivo* at the concentration of 100 nmol/L of OBI-3424.

Fig.3 shows the correlation between the expression level of AKR1C3 mRNA and the expression level of protein in leukemia cell lines, wherein Fig.3a shows the expression level of AKR1C3 mRNA of various ALL PDX detected by RNA-Seq analysis, Fig.3b shows the expression level of AKR1C3 protein of various ALL PDX detected by Western blotting, Fig.3c shows the correlation between the expression level of AKR1C3 RNA and the expression level of protein, and Fig.3d shows the correlation between the expression level of AKR1C3 RNA and the OBI-3424 $IC_{50}$.

Fig.4 shows the position information of the AKR1C3 primer-probe in present application.

Fig.5 shows the amplification results of 9 pairs of AKR1C3 primer-probes; wherein Fig.5a shows the amplification results of F1R1P1 primer-probe; Fig.5b shows the amplification results of F2R2P1 primer-probe; Fig.5c shows the amplification results of F2R6P1 primer-probe; Fig.5d shows the amplification results of F6R2P1 primer-probe; Fig.5e shows the amplification results of F6R6P1 primer-probe; Fig.5f shows the amplification results of F5R5P2 primer-probe; Fig.5g shows the amplification results of F3R3P1 primer-probe; Fig.5h shows the amplification results of F4R3P2 primer-probe; and Fig.5i shows the amplification results of F7R7P3 primer-probe.

Fig.6 shows the amplification results and standard curves of 3 pairs of AKR1C3 primer-probes; wherein Fig.6a shows the amplification results of F2R6P 1 primer-probe; Fig.6b shows the standard curve of F2R6P1 primer-probe (○ represents standard; E (amplification efficiency) =82.5%, $R^2$=0.987, Slope=-3.826, y-int (y-intercept) =51.961); Fig.6c shows the amplification results of F6R2P1 primer-probe; Fig.6d shows the standard curve of F6R2P1 primer-probe (○ represents standard; E (amplification efficiency) =96.5%, $R^2$=0.982, Slope=-3.408, y-int (y-intercept) =49.591); Fig.6e shows the amplification results of F6R6P1 primer-probe; and Fig.6f shows the standard curve of F6R6P1 primer-probe (○ represents standard; E (amplification efficiency) =98.0%, $R^2$=0.992, Slope=-3.371, y-int (y-intercept) =49.385).

Fig.7 shows the amplification result and standard curve of AKR1C3 plasmid; wherein Fig.7a shows the amplification result of AKR1C3 plasmid; and Fig.7b shows the standard curve of AKR1C3 plasmid (○ represents standards; E (amplification efficiency) =100.7%, $R^2$=0.999, Slope=-3.305, y-int (y-intercept) =42.336).

Fig.8 shows the amplification results of 4 types of reference gene primer-probes; wherein Fig.8a shows the amplification result of the reference gene GAP; Fig.8b shows the amplification result of the reference gene GOLGA1; Fig.8c shows the amplification result of the reference gene ACTB; and Fig.8d shows the amplification result of the reference gene HPRT1.

Fig.9 shows the Western blot result of AKR1C3 expression in different cell lines.

Fig.10 shows the results of detecting AKR1C3 expression in different cell lines using 4 types of reference genes; wherein Fig.10a shows the results of detecting AKR1C3 expression in different cell lines using the reference gene HPRT1; Fig.10b shows the results of detecting AKR1C3 expression in different cell lines using the reference gene GAP; Fig.10c shows the results of detecting AKR1C3 expression in different cell lines using reference gene ACTB; and Fig.10d shows the results of detecting AKR1C3 expression in different cell lines using the reference gene GOLGA1.

Fig.11 shows the results of different cell lines detected by AKR1C3-ACTB system.

Fig.12 shows the results of CCRF-CEM cell lines detected by AKR1C3-ACTB system; wherein Fig.12a shows the amplification result of AKR1C3 in CCRF-CEM cell lines; Fig.12b shows the standard curve of AKR1C3 in CCRF-CEM cell lines (target: AKR1C3; Eff (amplification efficiency) %=94.19%, $R^2$=0.998, Slope=-3.47, y-inter (y-intercept) =34.595); Fig.12c shows the amplification result of ACTB in CCRF-CEM cell lines; and Fig.12d shows the standard curve of ACTB in CCRF-CEM cell lines (target: ACTB; Eff (amplification efficiency) %=95.302%, $R^2$=0.991, Slope=-3.44, y-inter (y-intercept) =28.854).

Fig.13 shows the results of plasmids detected by AKR1C3-ACTB system; wherein Fig.13a shows the amplification

result of AKR1C3 plasmid; Fig.13b shows the standard curve of AKR1C3 plasmid (target: AKR1C3; Eff (amplification efficiency) %=101.853%, $R^2$=0.998, Slope=-3.278, y-inter (y-intercept) =38.048); Fig.13c shows the amplification result of ACTB plasmid; and Fig.13d shows the standard curve of ACTB plasmid (target: ACTB; Eff (amplification efficiency) %=94.134%, $R^2$=1, Slope=-3.471, y-inter (y-intercept) =40.821).

Fig.14 shows the qPCR detection result of Jurkat cell lines.

Fig.15 shows the digital PCR detection result of cell lines (FAM and VIC were detected simultaneously).

Fig.16 shows the results of AKR1C3 expression in 2 types of cell lines detected by digital PCR; wherein Fig.16a shows the result of AKR1C3 expression in CCRF-CEM cell lines detected by digital PCR; and Fig. 16b shows the result of AKR1C3 expression in MOLT-4 cell lines detected by digital PCR.

Fig.17 shows the results of ACTB expression in 2 types of cell lines detected by digital PCR; wherein Fig.17a shows the result of ACTB expression in CCRF-CEM cell line detected by digital PCR; and Fig.17b shows the result of ACTB expression in MOLT-4 cell line detected by digital PCR.

Fig.18 shows the results of plasmids detected by AKR1C3-ACTB system in actual samples testing; wherein Fig. 18a shows the amplification result of AKR1C3 plasmid; Fig. 18b shows the standard curve of AKR1C3 plasmid (target: AKR1C3; Eff (amplification efficiency) %=99.108%, $R^2$=0.996, Slope=-3.344, y-inter (y-intercept) =38.128); Fig.18c shows the amplification result of ACTB plasmid; and Fig.18d shows the standard curve of ACTB plasmid (target: ACTB; Eff (amplification efficiency) %=93.445%, $R^2$=0.998, Slope=-3.49, y-inter (y-intercept) =40.636).

Fig.19 is a histogram of FAM copy number/VIC copy number in actual samples.

Fig.20 shows the copy number results of 5-fold dilution of the cDNA of the cell lines detected with digital PCR.

## DETAILED DESCRIPTION OF THE INVENTION

It is necessary to indicate that, unless otherwise defined, technical terms or scientific terms used in one or more examples of the present specification shall have the ordinary meaning as understood by people having ordinary skill in the field to which the present disclosure belongs.

The experimental methods in the following examples are all conventional methods unless otherwise specified. The raw materials of the medicaments, the reagents and the like used in the following examples are all commercially available products unless otherwise specified.

"Administering" drug to patient refers to direct administration (which may be administered to patient by a medical professional or self-administered) and/or indirect administration, which may be an operation of making drug prescriptions. For example, physicians who instruct patient to self-administer drugs and/or provide patient with drug prescriptions will administer drugs to patient.

"Cancer" refers to leukemia, lymphoma, cancer and other malignant tumors (including solid tumors) that can spread locally by invasion and spread systemically by metastasis with potential unrestricted growth. Examples of cancers include (but are not limited to) cancers of adrenal gland, bone, brain, breast, bronchus, colon and/or rectum, gallbladder, head and neck, kidney, throat, liver, lung, nervous tissue, pancreas, prostate, accessory thyroid gland, skin, stomach and thyroid gland. Certain other examples of cancers include acute and chronic lymphocytic and granulocytic tumors, adenocarcinoma, adenoma, basal cell carcinoma, poorly differentiated cervical epithelium and carcinoma in situ, Ewing's sarcoma, epidermoid carcinoma, giant cell tumor, glioblastoma multiforme, hairy cell tumor, intestinal ganglion cell tumor, proliferative corneal nerve tumor, islet cell carcinoma, Kaposi's sarcoma, leiomyoma, leukemia, lymphoma, malignant carcinoid, malignant melanoma, malignant hypercalcemia, marfanoid habitus tumor, myeloid epithelial carcinoma, metastatic skin cancer, mucosal neuroma, myeloma, granuloma fungoide, neuroblastoma, osteosarcoma, osteogenic and other sarcomas, ovarian tumors, pheochromocytoma, true erythrocytosis, primary brain tumor, small cell lung cancer, squamous cell carcinoma of both ulcerative and papillary types, hyperplasia, seminoma, soft tissue sarcoma, retinoblastoma, rhabdomyosarcoma, renal cell tumor, local skin lesion, reticulum cell sarcoma, and Wilm's tumor.

The terms "patient" and "individual" can be used interchangeably herein and refer to mammals in need of treatment for cancer. Generally, the patient is a human. Generally, the patient is a human diagnosed with cancer. In certain examples, "patient" or "individual" may refer to a non-human mammal used in screening, characterizing, and evaluating drugs and therapies, such as, a non-human primate, a dog, cat, rabbit, pig, mouse or a rat.

"Prodrug" refers to a compound that, after administration, is metabolized or otherwise converted to a biologically active or more active compound (or drug) with respect to at least one property. A prodrug, relative to the drug, is modified

chemically in a manner that renders it, relative to the drug, less active or inactive, but the chemical modification is such that the corresponding drug is generated by metabolic or other biological processes after the prodrug is administered. A prodrug may have, relative to the active drug, altered metabolic stability or transport characteristics, fewer side effects or lower toxicity, or improved flavor (for example, see the reference Nogrady, 1985, Medicinal Chemistry A Biochemical Approach, Oxford University Press, New York, pages 388-392, incorporated herein by reference). A prodrug may be synthesized using reactants other than the corresponding drug.

"Solid tumor" refers to solid tumors including (but not limited to) metastatic tumor in bone, brain, liver, lung, lymph node, pancreas, prostate, skin and soft tissue (sarcoma).

"Treatment of" a condition or patient refers to taking steps to obtain beneficial or desired results, including clinical results. For purposes of this invention, beneficial or desired clinical results include, but are not limited to, alleviation or improvement of one or more symptoms of cancer; diminishment of extent of disease; delay or slowing of disease progression; alleviation, palliation, or stabilization of the disease state; or other beneficial results. Treatment of cancer may, in some cases, result in partial response or stable disease.

"Tumor cells" refers to tumor cells of any appropriate species, e.g., mammalian such as murine, canine, feline, equine or human.

"Therapeutically effective amount" of a drug refers to an amount of a drug that, when administered to a patient with cancer, will have the intended therapeutic effect, e.g., alleviation, amelioration, palliation or elimination of one or more manifestations of cancer in the patient. A therapeutic effect does not necessarily occur by administration of one dose, and may occur only after administration of a series of doses. Thus, a therapeutically effective amount may be administered in one or more administrations.

In describing optically active compounds, the prefixes R and S are used to represent the absolute configuration of the molecule around its chiral center. (+) and (-) are used to represent the optical activity of the compound, i.e., the direction in which the plane of polarization rotates through the optically active compound. The prefix (-) represents that the compound is levorotatory, i.e., the compound rotates the direction of the plane of polarization to left or anticlockwise. The prefix (+) represents that the compound is dextrorotary, i.e. the compound rotates the direction of the plane of polarization to right or clockwise. However, the optical signs (+) and (-) are independent of the absolute configurations of R and S of the molecule.

The structure of compounds OBI-3424 (also referred to as AST-3424 and TH-3424), OBI-3423 and OBI-2870 are shown below:

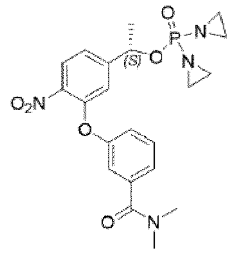

3424

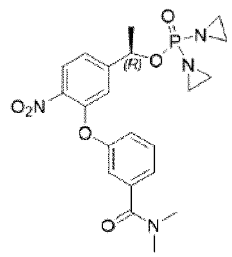

3423

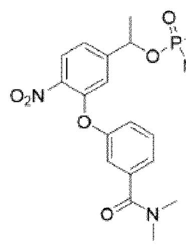

2870

wherein OBI-3424 is an S-enantiomer, OBI-3423 is an R-enantiomer, and OBI-2870 is a racemic mixture of OBI-3424 and OBI-3423 at a ratio of 1:1.

Previous studies have shown that compounds OBI-3424, OBI-3423 and OBI-2870 have good therapeutic effects on a variety of human cancers, especially compound OBI-3424 has good therapeutic effects on solid tumors and hematological malignancies; wherein the therapeutic effects of compound OBI-3424 on solid tumors (including liver cancer, hepatocellular carcinoma (HCC), non-small cell lung cancer, melanoma, prostate cancer, mastocarcinoma, esophageal cancer, renal cancer, gastric cancer, colon cancer, cerebral cancer, bladder cancer, cervical cancer, ovarian cancer, head and neck cancer, endometrial cancer, pancreas cancer, sarcomatoid carcinoma and rectal cancer and the like.) are recited in patent CN108290911A; and the therapeutic effects of compound OBI-3424 on hematological malignancies (including B lineage acute lymphoblastic leukemia (B-ALL) and T lineage acute lymphoblastic leukemia (T-ALL)) are recited in patent WO2019/062919A1.

AKR1C3 is overexpressed at the protein level in many types of cancer, especially in liver cancer, gastric cancer, renal cancer, CRPC and non-small cell lung cancer. Due to the high expression of AKR1C3 in tumors, compound OBI-3424 is designed to be specifically activated in tumors, but compound OBI-3424 cannot be activated in normal cells expressing low level of AKR1C3 to achieve tumor-specific targeting. OBI-3424 is developed as a highly potent DNA alkylation prodrug selectively activated by AKR1C3. In the presence of NADPH, OBI-3424 is reduced from AKR1C3 to

an intermediate which is spontaneously hydrolyzed to OBI-2660, and OBI-2660 is a DNA dialkylating agent similar to thioTEPA (N,N',N"-triethylenethiophosphoramide) which leads to DNA cross-linking at N7 (or O6) position of guanine, and subsequently leads to cell death, as shown below.

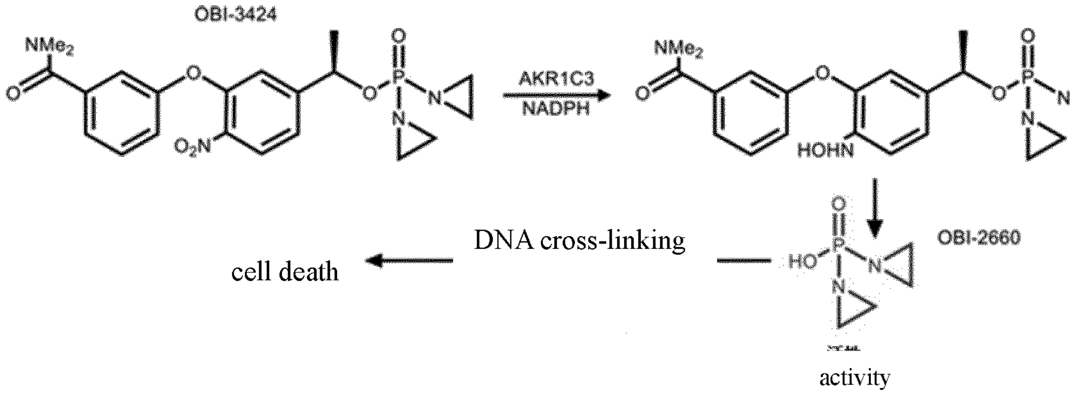


Further studies made by the applicant have confirmed that compound OBI-3424 activation is AKR1C3-dependent, and its cytotoxicity and antitumor efficacy are highly correlated with the expression level of AKR1C3 protein. Compound OBI-3424 shows AKR1C3-dependent cytotoxicity *ex vivo* and antitumor activity *in vivo* in a variety types of human cancer, which supports the further development of compound OBI-3424 as an anticancer agent that can be used to treat different types of cancer. AKR1C3 is used as a biomarker to analyze the condition of cancer patients and further guides patients to choose compound OBI-3424 for treatment.

Example 1 of the present application has confirmed that AKR1C3 enzyme and AKR1C3 RNA content in solid tumors are linearly correlated with the amount after mathematical transformation for $IC_{50}$ value of compound OBI-3424 on cancer cells and the linear correlation coefficient is high enough; Examples 2 and 3 have confirmed that AKR1C3 enzyme and AKR1C3 RNA content in hematological tumors are linearly correlated with the amount after mathematical transformation for $IC_{50}$ value of compound OBI-3424 on cancer cells, and the linear correlation coefficient is high enough. It is well known to those skilled in the art that the therapeutic effect of a drug on a patient can be directly confirmed only by association with $IC_{50}$ value; in theory and in practice, the lower the $IC_{50}$ value, the better the efficacy of a drug on a patient. All of the experimental data of examples 1-3 detected a relevant biometric value that can be objectively measured to correlate the $IC_{50}$ value, and then predicted the efficacy of a drug on a specific cancer patient and achieved targeted drug delivery (in fact, it is best to directly obtain the tumor tissue of a specific patient and then detect the $IC_{50}$ value under the condition of cancer cell survival. This is the best, most intuitive and accurate, but it is not feasible in practice, so only relevant biometric values that can be objectively measured can be detected to correlate the $IC_{50}$ value.). After extensive detections, the present application has confirmed that AKR1C3 enzyme and AKR1C3 RNA content in solid tumors and hematological tumors are not only linearly correlated with the $IC_{50}$ value of compound OBI-3424 on cancer cells, but also the linear correlation coefficient is high enough, which is enough to illustrate that AKR1C3 enzyme and AKR1C3 RNA content in solid and hematological tumors are highly correlated with the $IC_{50}$ value of compound OBI-3424 on cancer cells. Therefore, in practical application, AKR1C3 enzyme content and AKR1C3 RNA content *of ex vivo* sample of a patient can be detected, and then compound OBI-3424 can be administrated to a patient for treatment according to the detection results.

In the prior art, Western blotting or immunochemical staining is usually used to detect AKR1C3 enzyme content, however, there is no corresponding detection method for detecting AKR1C3 RNA content in the prior art. Assuming that a corresponding assay can be used to assess AKR1C3 RNA content, patient with higher AKR1C3 RNA content and most likely to respond to the prodrug thus can be selected to administrate compound OBI-3424 for achieving better cancer treatment effect.

Based on the above purpose, based on real-time fluorescence quantitative PCR technology (qPCR), the present invention adopts RNA reverse transcription reaction, polymerase chain reaction and Taqman probe technology to detect the expression of AKR1C3 gene, and establishes a method and the corresponding kit of human AKR1C3 gene expression which can be applied to LDT (Laboratory developed test) system, finally they can be used for the drug detection of clinical trial samples companion diagnostics.

Basic principles for designing primer and probe: 1) length of primer-probe: the length of each primer is 15-30 bases. 2) GC% in the primer-probe is required to range from 30 to 80%, and Tm value of primer ranges from between 55 to 60°C. 3) base pairing of the primer-probe itself and the 3' end between primers should be avoided as much as possible. 4) more than 6 consecutive base pairs of the primer-probe itself and between primer should be avoided as much as possible. 5) the length of the target fragment amplified by upstream and downstream primers ranges from 50

to 180bp, and the probe is located between the upstream and downstream primers, as close as possible to the upstream primer, and the use of guanine should be avoided at the 5' end of the probe. When the annealing temperature of the probe is too low, LNA probe or MGB probe is considered for design. 6) The selected primers and probes should be designed in the conserved region of AKR1C3 gene which can specifically detect the expression of AKR1C3 gene.

Technical principles: RNA reverse transcription reaction, polymerase chain reaction and Taqman probe technology are used to design specific primers and probes directing AKR1C3 gene expression. The target sequence is amplified by PCR using specific primers, and the Taqman probe bound to the template is cut by Taq enzyme (5'→3' exonuclease activity), the reporter is separated from the quencher, generates and accumulates fluorescent signals. The real-time amplification curve can be obtained according to the relationship between the fluorescent signals and the number of amplification cycles, and then the detection for the expression level of AKR1C3 RNA can be achieved.

The present invention designs 9 pairs of primers and probes for 3 target regions of AKR1C3 (exons 2-3, 4-5 and 5-6, respectively). The position information of the primer-probes can be seen in Fig.1. Beacon Designer 8.12 software is used to assist the evaluation of primer quality which is in line with the basic principles for designing primer-probe, and Blast tool in NCBI database is used to ensure that each pair of primer-probe specifically amplified human gene sequences without common SNP sites.

The first aspect of the present invention provides a primer-probe composition, selected from any one of the following groups:

(i) upstream primer AKR1C3-F1, downstream primer AKR1C3-R1 and probe AKR1C3-P1, wherein the nucleotide sequences of the upstream primer AKR1C3-F1, the downstream primer AKR1C3-R1 and the probe AKR1C3-P1 are shown as SEQ ID NO:1, SEQ ID NO:2 and SEQ ID NO:3, respectively;

(ii) upstream primer AKR1C3-F2, downstream primer AKR1C3-R2 and probe AKR1C3-P1, wherein the nucleotide sequences of the upstream primer AKR1C3-F2, the downstream primer AKR1C3-R2 and the probe AKR1C3-P1 are shown as SEQ ID NO:4, SEQ ID NO:5 and SEQ ID NO:3, respectively;

(iii) upstream primer AKR1C3-F2, downstream primer AKR1C3-R6 and probe AKR1C3-P1, wherein the nucleotide sequences of the upstream primer AKR1C3-F2, the downstream primer AKR1C3-R6 and the probe AKR1C3-P1 are shown as SEQ ID NO:4, SEQ ID NO:6 and SEQ ID NO:3, respectively;

(iv) upstream primer AKR1C3-F6, downstream primer AKR1C3-R2 and probe AKR1C3-P1, wherein the nucleotide sequences of the upstream primer AKR1C3-F6, the downstream primer AKR1C3-R2 and the probe AKR1C3-P1 are shown as SEQ ID NO:7, SEQ ID NO:5 and SEQ ID NO:3, respectively;

(v) upstream primer AKR1C3-F6, downstream primer AKR1C3-R6 and probe AKR1C3-P1, wherein the nucleotide sequences of the upstream primer AKR1C3-F6, the downstream primer AKR1C3-R6 and the probe AKR1C3-P1 are shown as SEQ ID NO:7, SEQ ID NO:6 and SEQ ID NO:3, respectively;

(vi) upstream primer AKR1C3-F5, downstream primer AKR1C3-R5 and probe AKR1C3-P2, wherein the nucleotide sequences of the upstream primer AKR1C3-F5, the downstream primer AKR1C3-R5 and the probe AKR1C3-P2 are shown as SEQ ID NO:8, SEQ ID NO:9 and SEQ ID NO: 10, respectively;

(vii) upstream primer AKR1C3-F3, downstream primer AKR1C3-R3 and probe AKR1C3-P1, wherein the nucleotide sequences of the upstream primer AKR1C3-F3, the downstream primer AKR1C3-R3 and the probe AKR1C3-P1 are shown as SEQ ID NO: 11, SEQ ID NO: 12 and SEQ ID NO:3, respectively;

(viii) upstream primer AKR1C3-F4, downstream primer AKR1C3-R3 and probe AKR1C3-P2, wherein the nucleotide sequences of the upstream primer AKR1C3-F4, the downstream primer AKR1C3-R3 and the probe AKR1C3-P2 are shown as SEQ ID NO: 13, SEQ ID NO: 12 and SEQ ID NO: 10, respectively;

(ix) upstream primer AKR1C3-F7, downstream primer AKR1C3-R7 and probe AKR1C3-P3, wherein the nucleotide sequences of the upstream primer AKR1C3-F7, the downstream primer AKR1C3-R7 and the probe AKR1C3-P3 are shown as SEQ ID NO: 14, SEQ ID NO: 15 and SEQ ID NO: 16, respectively.

The present invention performed AKR1C3 amplification by using the above 9 groups of primer-probe compositions, and selected groups (iii), (iv) and (v) from the 9 groups of primer-probe compositions as candidate primer-probe compositions according to the amplification efficiency and specificity. In order to determine the best primer-probe composition, further AKR1C3 amplification was performed in different samples by using primer-probe composition from

groups (iii), (iv) and (v), respectively. According to the amplification efficiency, the primer-probe composition of group (iv) (upstream primer AKR1C3-F6, downstream primer AKR1C3-R2 and probe AKR1C3-P1) was finally determined as the best primer-probe composition of the present invention.

It should be noted that the 9 groups of primer-probe compositions of the present invention can be used not only for the detection of AKR1C3 RNA content by qPCR method, but also for the detection of AKR1C3 RNA content by digital PCR method. The steps of the detection of AKR1C3 RNA content by qPCR method and digital PCR method are described in detail below.

In a preferred embodiment of the present invention, wherein the 5'-end reporters of probes AKR1C3-P1, AKR1C3-P2 and AKR1C3-P3 are FAM, and the 3'-end quenchers of probes AKR1C3-P1, AKR1C3-P2 and AKR1C3-P3 are MGB.

The preferred probe for the implementation of the present invention is the probe labeled according to the TaqMan system. The TaqMan system is available from the manufacturer, Life Technologies Inc. According to the TaqMan system, oligonucleotide probes that are specifically designed to hybridize to the amplified target DNA are covalently linked to a reporter at 5' end and to a quencher at 3' end. Examples of appropriate reporters for use in TaqMan system include 6-carboxyfluorescein (FAM) or tetrachlorofluorescein (TET). The typical quencher is minor groove binder (MGB). The principle of this TaqMan system is that the quencher inhibits the fluorescence of the reporter as long as the position of the quencher and the reporter are very close to each other on the probe. Once the oligonucleotide probe hybridizes to the target DNA during qPCR amplification, it will be degraded by Taq polymerase because the enzyme makes that the oligonucleotide primer extends along the DNA corresponding to the target DNA. This degradation releases the reporter and the quencher, and the quencher becomes no longer very close to the reporter, thus enabling the reporter to emit its fluorescence, which can then be detected and measured by appropriate tools typically integrated in qPCR equipment (i.e. thermal cyclers).

Based on the same inventive concept, the second aspect of the present invention provides a kit comprising the primer-probe composition as described above.

In a preferred embodiment of the present invention, wherein the kit as described above further comprises a primer-probe composition of reference gene; and the primer-probe composition of the reference gene comprises upstream primer ACTB-F1, downstream primer ACTB-R1 and probe ACTB-P1, and the nucleotide sequences of the upstream primer ACTB-F1, the downstream primer ACTB-R1 and the probe ACTB-P1 are shown as SEQ ID NO: 17, SEQ ID NO: 18 and SEQ ID NO: 19, respectively.

When using qPCR technology to measure the expression level of gene, the absolute expression level of the target gene is not often directly measured, but the expression levels of the target gene and the reference gene are measured, respectively. Specifically, the expression level of the reference gene as a standard is taken to measure the relative expression level of the target gene, and then the relative expression levels between samples are compared. When the relative quantitative method is used to measure the expression level of gene, the Ct value obtained by qPCR is an exponential relationship, not a linear relationship. Therefore, the Ct value cannot be directly used for statistical analysis methods that require normal distribution of data, such as t test and analysis of variance (ANOVA) method. Therefore, the original Ct value should be converted to $2^{-Ct}$ first to makes the data reach a linear relationship before statistical analysis. After the data are obtained by the relative quantitative method, the comparative CT value method ($2^{-\Delta\Delta Ct}$ method) is often used for data analysis. This method is based on two assumptions: ① the amplification efficiency is 100%, i.e., the amount of products in each PCR cycle is doubled, which can be solved by the verification of amplification efficiency; ② there are appropriate reference genes to correct the error of the loading quantity of samples. According to the formula, calculating the fold change $=2^{-\Delta\Delta Ct}$; $\Delta\Delta$Ct=(Ct of target gene - Ct of reference gene) of treatment group - (Ct of target gene - Ct of reference gene) of control group. According to the calculation formula, in relative quantitative method, the relative expression of the target gene cannot be calculated without the Ct value of the reference gene, suggesting that the reference gene plays a very important role.

The reference gene refers to a known reference gene whose expression level is not affected by the study conditions and can be expressed consistently among multiple samples, and the expression level of this gene can be used to accurately quantify the loading of the initial material. Since the expression level of housekeeping gene is less affected by environmental factors and can be continuously expressed in almost all tissues and various growth stages of an organism, housekeeping gene is usually used as a reference gene in experiments.

According to the reference (DONG Enni, LIANG Qing, LI Li, et al., The Selection Of Reference Gene in Real-time Quantitative PCR [J]. China Animal Industry, 49(11):92-96. DOI:10.3969/j.issn.0258-7033.2013.11.025) and the reference (ZHAO Wen-Jing, XU Jie, BAO Qiuhua, et al., Selection of Reference Genes for Real-time Quantitative PCR [J]. Microbiology China, 2010(12): 1825-1829. DOI:CNKI:SUN:WSWT.0.2010-12-019), the reference genes used in q-PCR are shown in the following table:

| Species | Sample Type | Reference Gene | |
|---|---|---|---|
| | | Good stability | Poor stability |
| Human | Blood (pulmonary tuberculosis patient) | HuPO | GAPDH, β-actin, HPRT |
| | Peripheral blood mononuclear cell | HuPO, HPRT | GAPDH, β-actin, EF-1-α |
| | Lung cancer tissue | GUSB, β2-M | β2-Microglobulin, GAPDH |
| | Liver cancer tissue | HMBS, C-TBP | UBC, HPRT, 18SrRNA |
| Mouse | Brain (olfactory bulb, cerebellum, cortex, hypothalamus, hippocampus, brainstem and corpus striatum) | GAPDH, HPRTl, β-actin, Aequorin | NT-3 |
| | Small intestine | SDHA, HRPTl | ARBP, ACTB, GAPDH, B2M |
| | Liver (immunostimulation) | ACTB, GAPDH | B2M, SDHA, HPRTl, ARBP |
| | Mammary tissue | GAPDH, HPRT1 | ARBP, ACTB, SDHA, B2M |
| Pig | Piglet (heart, liver, spleen, lung, kidney, brain, muscle, uterus, large intestine and intestinal lymphoid tissue) | HMBS, HPRT1, RPL4 | ACTB, GAPDH, SDHA, TBP1, B2M, YWHAZ |
| Bovine | Endometrial tissue | SUZ12 | GAPDH |
| Sheep | Blood | SDHA, YWHAZ | GAPDH, PGK1 |
| Goat | Preantral follicle | UBQ, β-actin | 18SrRNA |
| | Embryo fibroblast (Newcastle disease infected) | 18SrRNA, GAPDH, SHDA | ACTB, HPRTl, HMBS |
| Chicken | Blood (Pre-inflammatory phase) | β-actin | HPRT, GAPDH |
| Goose | Gosling (liver, kidney, heart, muscle and ovary) | GAPDH, HPRT1 | 28SrRNA, TUB, SDH, ACT, 18SrRNA |

As for reference genes, 4 types of reference genes: HPRT1 (hypoxanthine phosphoribosyl transferase), GAP (glyceraldehyde-3-phosphate dehydrogenase), ACTB (also known as β-actin) and GOLGA1 (Golgi protein, specifically Golgi autoantigen A1) were screened out in present invention. The primer-probe compositions corresponding to each of 4 types of reference genes (as shown in Table 1) were used and detected with the cell line CCRF-CEM to evaluate the fluorescence intensity and amplification. Different samples (including healthy blood samples and bone marrow samples of leukemia patients) and cell lines with different expression levels (including cell lines with high AKR1C3 expression and cell lines with low AKR1C3 expression) were further detected to verify the expression stability and whether the samples and cell lines with high and low expression levels can be distinguished. The detected results showed that cell lines RPMI8226 and CCRF-CEM with high expression levels can be better distinguished from cell line Jurkat with low expression level by using ACTB as reference as compared to using other three reference genes as references. In healthy people, the values remained relatively low level, while high expression level of AKR1C3 was detected in bone marrow RNA samples of leukemia patients. ACTB was selected as the reference gene based on the result of amplification curve. Accordingly, the primer-probe composition of the reference gene in the above kit comprises upstream primer ACTB-F1, downstream primer ACTB-R1 and probe ACTB-P1.

In a preferred embodiment of the present invention, wherein the 5'-end reporter of the probe ACTB-P1 is VIC (green fluorescent protein), and the 3'-end quencher of the probe ACTB-P1 is BHQ1 (Black Hole Quencher 1).

The best AKR1C3 primer-probe composition and reference ACTB primer-probe composition finally determined after screening are shown in Table 1.

Table 1 Sequence information table of AKR1C3 using primer-probes

| Name | Sequence | Marker | Description of Source |
|---|---|---|---|
| AKR1C3-F1 | CTCAACAAGCCAGGACTCAAGTACAAGC | | |

(continued)

| Name | Sequence | Marker | Description of Source |
|---|---|---|---|
| AKR1C3-F2 | ACAAGCCAGGACTCAAGTACAA | | |
| AKR1C3-F3 | CAGTGTGAAGAGAGAAGACATATTCTACA | | |
| AKR1C3-F4 | GCAGATGGCAGTGTGAAGAG | | |
| AKR1C3-F5 | TGGCAGTGTGAAGAGAGRAGAC | | |
| AKR1C3-F6 | GATGATCCTCAACAAGCCAGGA | | |
| AKR1C3-F7 | CACCAACAGATGAAAATGGAAAAGTAA | | |
| AKR1C3-R1 | ACTTGCAGAAATCTAGCAATTTACTCCGGTTG | | |
| AKR1C3-R2 | AATCTAGCAATTTACTCCGGTTGAAATAC | | |
| AKR1C3-R3 | CAAGGCTGGTCGGACCAA | | |
| AKR1C3-R5 | GGTCAACATAGTCCAATTGAGCT | | |
| AKR1C3-R6 | ACTTGCAGAAATCTAGCAATTTACTCC | | |
| AKR1C3-R7 | GGTTGAAGTTTGACACCCCAA | | |
| AKR1C3-P1 | CTGCAACCAGGTAGAATGTCA | 5'FAM, 3'MGB | |
| AKR1C3-P2 | CTTTGGTCCACTTTTCATCGAC | 5'FAM, 3'MGB | |
| AKR1C3-P3 | TTGACATAGTGGATCTCTGTACCA | 5'FAM, 3'MGB | |
| ACTB-F1 | CACCTTCTACAATGAGCTGCG | | |
| ACTB-R1 | GTCTCAAACATGATCTGGGTCATC | | |
| ACTB-P1 | CCTCGGTCAGCAGCACGGGGT | 5'VIC, 3'BHQ1 | |
| HPRT1-F1 | CAGCCTCAACATCCTGCACT | | from reference 1 |
| HPRT1-R1 | CACACAATAGCTCTTCAGTCTG | | from reference 1 |
| HPRT1-P1 | TGACCTTGATTTATTTTGCATACC | | from reference 2 |
| GAP-F1 | AGGCTGGGGCTCATTTGC | | from reference 3 |
| GAP-R1 | GTG CTCAGTGTAGCCCAGGATG | | from reference 3 |
| GAP-Pl | GAGCCACACAAACACCAG | | from reference 4 |
| GOLGA1-F1 | upstream nucleic acid fragment of Golgi autoantigen A1 (GOLGA1) gene | | nucleic acid sequence was not determined |
| GOLGA1-R1 | downstream nucleic acid fragment of Golgi autoantigen A1 (GOLGA1) gene | | nucleic acid sequence was not determined |
| GOLGA1-P1 | probe nucleic acid fragment of Golgi autoantigen A1 (GOLGA1) gene | | nucleic acid sequence was not determined |

Reference 1: corresponding nucleic acid sequences disclosed in Wu, X., Blackburn, P., Tschumper, R. et al. TALEN-mediated genetic tailoring as a tool to analyze the function of acquired mutations in multiple myeloma cells. Blood Cancer Journal 4, e210 (2014). https://doi.org/10.1038/bcj.2014.32.

Reference 2: HPRT1 nucleic acid sequences disclosed in WO2018123764A1.

Reference 3: corresponding nucleic acid sequences disclosed in Werner Kempf, Marshall E. Kadin, Ann M. Dvorak, Carol C. Lord, Günter Burg, Norman L. Letvin, Igor J. Koralnik, Endogenous retroviral elements, but not exogenous retroviruses, are detected in CD30-positive lymphoproliferative disorders of the skin, Carcinogenesis, Volume 24, Issue 2, February 2003, Pages 301-306, https://doi.org/10.1093/carcin/24.2.301.

Reference 4: corresponding nucleic acid sequences disclosed in Stecker C, Johann A, Herzberg C, Averhoff B, Gottschalk G. Complete nucleotide sequence and genetic organization of the 210-kilobase linear plasmid of Rhodococcus erythropolis BD2. J Bacteriol. 2003;185(17):5269-5274. doi:10.1128/jb.185.17.5269-5274.2003.

All the sequences without source indicated were new sequences or newly developed or selected sequences for the kit.

In a preferred embodiment of the present invention, wherein the kit as described above further comprises a polymerase mixture, and the polymerase mixture mainly comprises: DNA polymerase, $MgCl_2$, buffer and dNTPs; preferably, the polymerase mixture is selected as KAPA PROBE FAST RT-PCR Master Mix(2x), which has been used

in the laboratory for a long time and has been repeatedly verified to have good amplification efficiency.

In a preferred embodiment of the present invention, wherein the kit as described above further comprises a reverse transcriptase mixture;

preferably, the reverse transcriptase mixture is Superscript VII,O MARSTER MIX.

In each PCR reaction, it must be detected and analyzed together with positive control and negative control (NC, nuclease-free water). In a preferred embodiment of the present invention, wherein the kit as described above further comprises a negative control and a positive control;

preferably, the negative control (NC) is nuclease-free water; and/or

preferably, the positive control is a reference with a known copy number.

The kit as described above may further comprises the elements necessary to perform qPCR, for example, reagents, as known to those skilled in the art. In addition to primer pairs and probes, the kit may further comprises one or more enzymes (Taq polymerase) or reagents used in qPCR reactions. The enzyme can be present in a lyophilized form or in a suitable buffer. In addition, the kit may comprises all the additional elements necessary to perform qPCR, such as buffer, extraction reagent, enzyme, pipette, plate, nucleic acid, filter paper, gel material, transfer material, autoradiography equipment, instructions (recording relevant operation methods), etc.

Based on the same inventive concept, the third aspect of the present invention provides use of the primer-probe composition or the kit as described above in the preparation of a drug for treating cancers.

In a preferred embodiment of the present invention, wherein the AKR1C3 RNA content in an *ex vivo* sample of a patient is obtained by using the primer-probe composition or the kit as described above;

patient with AKR1C3 RNA content greater than or equal to the predetermined content are administrated AKR1C3 activated anticancer drugs.

Accordingly, the present invention provides a method for treating a patient suffering from cancer, wherein the method includes:

obtaining the AKR1C3 RNA content in *ex vivo* sample of a patient by using the primer-probe composition or the kit as described above;

administering AKR1C3 activated anticancer drugs to a patient with AKR1C3 RNA content greater than or equal to the predetermined content; preferably, a therapeutically effective amount of compounds OBI-3424, OBI-3423 and OBI-2870 is administrated to the patient.

In a preferred embodiment of the present invention, wherein the AKR1C3 RNA content is obtained according to the ratio of AKR1C3 copy number/reference gene copy number, for example, the ratio of AKR1C3 copy number/ACTB copy number is calculated, if the ratio of AKR1C3 copy number/ACTB copy number > X, then it is determined that the expression of AKR1C3 is high, and the patient will be given a therapeutically effective amount of compounds OBI-3424, OBI-3423 and OBI-2870; if the ratio of AKR1C3 copy number/ACTB copy number ≤ X, then it is determined that the expression of AKR1C3 is low, and the patient will not be given a therapeutically effective amount of compounds OBI-3424, OBI-3423 and OBI-2870, but other cancer drugs can be given to the patient.

The value of predetermined content X needs to be determined by detecting a large number of samples. According to a large number of clinical trial samples and through statistical methods, the inventors finally determined that the predetermined content X is 0.0001~1; more preferably, the predetermined content X is 0.00011~0.5; even more preferably, the predetermined content X is 0.00013~0.05; in particular preferably, the predetermined content X is 0.00014~0.015. For example, the predetermined content X is 0.0001~0.0005, 0.0001~0.001, 0.0001~0.005, 0.0001~0.01, 0.0001~0.05, 0.0001~0.1, 0.0001~0.5, 0.0001~0.9, 0.00011~0.0005, 0.00011~0.001, 0.00011~0.005, 0.00011~0.01, 0.00011~0.05, 0.00011~0.1, 0.00011~0.5, 0.00011~1, 0.00013~0.0005, 0.00013~0.001, 0.00013~0.01, 0.00013~0.05, 0.00013~0.1, 0.00013~0.5, 0.00013~1, 0.0005~0.001, 0.0005~0.005, 0.0005~0.01, 0.0005~0.05, 0.0005~0.1, 0.0005~0.5, 0.0005~1, 0.00091~0.001, 0.00091~0.05, 0.00091~0.1, 0.00091~0.5, 0.00091~1, 0.001~0.005, 0.001~0.01, 0.001~0.05, 0.001~0.1, 0.001~0.5, 0.001~1, 0.005~0.01, 0.005~0.05, 0.005~0.1, 0.005~0.5, 0.005~1, 0.01~0.05, 0.01~0.1, 0.01~0.5, 0.01~1, 0.05~0.1, 0.05~0.5 or 0.05~1; more preferably, the predetermined content X is 0.0001, 0.00011, 0.00012, 0.00013, 0.00014, 0.00015, 0.000156, 0.00016, 0.000163, 0.000165, 0.00017, 0.000177, 0.00018, 0.000181, 0.00019, 0.000195, 0.0002, 0.000201, 0.000216, 0.000221, 0.00023, 0.000239, 0.00025, 0.00029, 0.0003, 0.00035, 0.0004, 0.00045, 0.0005, 0.00054, 0.0006, 0.00065, 0.0007, 0.00075, 0.0008, 0.00085, 0.0009, 0.00095, 0.001, 0.0015, 0.00177, 0.002, 0.00238, 0.0025, 0.00266, 0.00296, 0.003, 0.00315, 0.0035, 0.004, 0.0045, 0.005, 0.0055, 0.006, 0.0065, 0.00679, 0.007, 0.00710, 0.0075, 0.00778, 0.00791, 0.008, 0.0085, 0.009, 0.00939, 0.0095, 0.01, 0.011, 0.012, 0.01207, 0.013, 0.01365, 0.014, 0.015, 0.016, 0.017, 0.018, 0.019, 0.02, 0.025, 0.03, 0.035,

0.04, 0.045, 0.05, 0.055, 0.06, 0.065, 0.07, 0.075, 0.08, 0.085, 0.09, 0.095, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95 or 1.

Based on the same inventive concept, the fourth aspect of the present invention provides a method for detecting AKR1C3 RNA content, including the following steps:

(1) extracting RNA of an *ex vivo* sample to be detected and adding the extracted RNA to the reverse transcription system and the extracted RNA being reverse transcribed to synthesize cDNA;

(2) performing qPCR or digital PCR amplification with cDNA as template using the primer-probe composition or the kit as described above;

(3) obtaining AKR1C3 RNA content of *ex vivo* sample to be detected according to qPCR or digital PCR amplification results.

Methodological principle of the method for detecting AKR1C3 RNA content of the present invention: firstly, the sample RNA is reverse transcribed into cDNA, and then the cDNA is amplified by specific primers and probes, wherein the AKR1C3 gene and reference gene are indicated by FAM and VIC signals, respectively; and the quantitative expression levels of AKR1C3 gene in peripheral blood RNA sample is calculated by samples and quality control products. At the same time, VIC signal in AKR1C3 reaction solution is used to monitor the sample quality.

In the present invention, two methods of qPCR and digital PCR are used to conduct a comparative experiment at the same time under the condition of sufficient samples. The same set of primer-probe is used for digital PCR and qPCR. Firstly, the temperature gradient of qPCR is used to optimize the primer-probe and the most appropriate temperature and primer-probe composition is selected for the digital PCR platform for quantitative reference materials and as a comparative method.

The detection process of digital PCR mainly includes two parts, i.e., PCR amplification and fluorescence signal analysis. Before PCR reaction, samples are divided into tens of thousands of units (reaction chambers) so that only a single DNA molecule is present in each unit. The amplification procedure and system of amplification stage of digital PCR are basically the same as those of ordinary PCR, but different from traditional qPCR method, digital PCR adopts direct counting method for quantitative analysis, that is, after the end of PCR amplification, if fluorescence signal (product) is present, marked as 1, and if no fluorescence signal (product) is present, marked as 0. A reaction unit with a fluorescent signal contains at least one copy of the target molecule. Theoretically, in the case of the concentration of target DNA in the sample is very low, the number of reaction units with fluorescence signal is equal to the copy number of target DNA molecule. However, under normal circumstances, the reaction units of digital PCR may contain two or more target molecules, which can be calculated by Poisson distribution.

$$p = \frac{\lambda^k}{k!}e^{-\lambda}$$

In the above formula, where $\lambda$ is the average copy number (concentration) of target DNA molecules contained in each reaction unit and *p* is the probability of *k* copies of target DNA molecules contained in each reaction unit under certain $\lambda$ conditions. $\lambda$ is determined by the dilution coefficient *m* of sample solution, with $\lambda$=cm, where c is the original copy number (concentration) of the sample. When k=0 (without the target DNA molecule), the above formula can be simplified as $p=e^{-\lambda}=e^{-cm}$, *p* can be regarded as the ratio of the number of reaction units without fluorescence signal to the total number of reaction units, i.e,

$$\frac{n-f}{n} = e^{-cm}$$

where *n* is the total number of reaction units and *f* is the number of reaction units with fluorescence signal. Take the logarithm (ln) of both sides of the above formula to obtain

$$cm = \ln(1 - \frac{f}{n})$$

The initial copy number (concentration) of sample can be obtained from the total number of reaction units in the digital PCR and the number of units with fluorescent signals, as well as the dilution coefficient of sample. The quantitative method of digital PCR does not depend on the cyclic threshold of the amplification curve, so it will not affected by the amplification efficiency and does not need to use the standard curve. It has good accuracy and reproducibility, and absolute quantitative analysis can be achieved.

In the present invention, the specific reaction system of digital PCR differs from the specific reaction system of qPCR in that: in qPCR reaction system, a group of primer-probe composition selected from groups (i) to (ix) is mixed with the primer-probe composition of reference gene ACTB; whereas in digital PCR reaction system, due to the large difference in the expression levels of AKR1C3 and ACTB in samples and cell lines, the maximum difference in Ct may reach 10 Ct values. Therefore, the copy number of VIC may be much larger than that of FAM during the digital PCR detection. In view of this, a group of primer-probe composition selected from groups (i) to (ix) is not mixed with the primer-probe composition of reference gene ACTB, but AKR1C3 digital PCR detection system and reference gene digital PCR detection system are used for the amplification of AKR1C3 and reference genes, respectively.

PCR amplification results are affected by the changes in various factors of reaction system (including primer concentration, probe concentration, etc.) and amplification procedures. In order to obtain the best amplification efficiency and the least nonspecific products, the present application determines the reaction system and amplification procedure of digital PCR and qPCR based on years of research and development experience. In combination with the selection of probe-primers, the correlation coefficient of standard curve is ensured to be greater than or equal to 0.98 and the E value (amplification efficiency) is between 85% and 110%.

In a preferred embodiment of the present invention, wherein in step (3), the step for obtaining AKR1C3 RNA content *of ex vivo* sample to be detected according to qPCR or digital PCR amplification results includes:

(A) obtaining the copy numbers of AKR1C3 and reference gene *of ex vivo* sample to be detected according to qPCR or digital PCR amplification results, respectively;

(B) calculating the ratio of AKR1C3 copy number/reference gene copy number to obtain the AKR1C3 RNA content of *ex vivo* sample to be detected.

Wherein when the qPCR method is used, step (A) further includes:

($A_1$) plotting the standard curve of Ct value and initial copy number lg value of AKR1C3 and the standard curve of Ct value and initial copy number lg value of reference gene, respectively;

($A_2$) according to the standard curves, obtaining the copy numbers of AKR1C3 and reference gene *of ex vivo* sample to be detected by the detected Ct values of AKR1C3 and reference gene, respectively (which can be calculated automatically by software).

Another difference between digital PCR and qPCR is that the copy numbers of AKR1C3 and reference gene of the *ex vivo* sample to be detected are obtained by qPCR according to the standard curve, while the copy numbers of AKR1C3 and reference gene of the *ex vivo* sample to be detected can be directly obtained by digital PCR according to the amplification result, without drawing the standard curve.

The Ct value of target gene AKR1C3 and the Ct value of reference gene should be interpreted. The Ct value of target gene AKR1C3 is the Ct value corresponding to the amplification signal (FAM signal) of target gene AKR1C3. The Ct value of reference gene is the Ct value corresponding to the amplification signal (VIC signal) of reference gene. The threshold values of FAM and VIC are set during the exponential amplification stage.

In a preferred embodiment of the present invention, wherein the method is used to detect AKR1C3 RNA content in blood samples, bone marrow samples, or tissue samples. Digital PCR and qPCR of the present invention can not only detect blood samples or bone marrow samples, but also detect tissue samples of solid tumors, except that the pre-processing process (such as RNA extraction process) is different. For example, as for blood samples, RNA extraction can be performed directly using the Qiagen made-up extraction kit; as for bone marrow samples or tissue samples, the mortar can be pre-cooled with liquid nitrogen, the bone marrow samples or tissue samples are ground to powder in the mortar, the dried tissue powder is placed in a 1.5 ml EP tube, and then the RNA extraction kit is used for extraction.

The present application has conducted a performance test on the above kit and detection method, and the test results are as follows:

1. minimum detection limit: the minimum detection limit was determined by gradient dilution of low-expression cell lines, which was used as the sample to be detected and repeated 10 times, and the detection results were 100% consistent;

2. precision: repeated detection by different time, instruments and experimenters, with CV value < 15%;

3. positive coincidence rate: the known positive samples and the standard curve were detected by qPCR, and the detection results were 100% consistent, E value of the standard curve was between 90%-110%, and $R^2$ was ≥0.98;

4. negative coincidence rate: the known negative samples were detected by qPCR, and the consistent detection rate of detection results was 100%;

5. different loading template amount: 200ng and 10ng RNA of known negative and positive samples were selected respectively as the starting amounts of reverse transcription to verify the range of loading amounts, and the consistent detection rate of detection results was 100%;

6. analysis specificity: two concentrations of negative references were selected, and each concentration level was repeated for three times, with a negative rate of 100%;

7. actual sample detection: 8 normal peripheral blood RNA samples were selected for actual sample detection, and the coincidence rate of detection results was 100%.

Through the performance verification of minimum detection limit, precision, negative coincidence rate, positive coincidence rate, template amount, analytical specificity and actual sample detection, all of the 7 performances met the verification standards. The performance of the detection method met the requirements of clinical application and can be used for clinical sample detection.

Based on the same inventive concept, the fifth aspect of the present invention provides a method for detecting the expression level of AKR1C3 enzyme, wherein the method is used to detect the AKR1C3 RNA content of *ex vivo* sample to be detected, and then the expression level of the AKR1C3 enzyme of *ex vivo* sample to be detected is obtained according to the AKR1C3 RNA content *of ex vivo* sample to be detected.

In a preferred embodiment of the present invention, wherein there is a linear correlation between AKR1C3 RNA content *ex vivo* sample to be detected and the expression level of AKR1C3 enzyme *ex vivo* sample to be detected.

In the prior art, administration of compounds OBI-3424, OBI-3423 and OBI-2870 are usually judged based on whether the AKR1C3 reductase content is equal to or greater than the predetermined content, and example 4 of the present invention proves that the expression level of AKR1C3 enzyme is highly correlated with the expression level of AKR1C3 RNA. Therefore, in practical application, the AKR1C3 enzyme content can be calculated according to AKR1C3 RNA content measured by the above method, and then administration of compounds OBI-3424, OBI-3423 and OBI-2870 are judged based on whether the AKR1C3 enzyme content reaches the predetermined content.

The technical embodiments provided by the present invention are further described in the following examples. The following examples are only used to illustrate the invention and do not limit the protection scope of the present invention.

The synthetic method for the AKR1C3 activated anticancer prodrug OBI-3424 (also referred to as AST-3424 or TH-3424) used in the following examples can be seen in WO2017087428A1 or CN108290911A.

Example 1. Correlation between the level of AKR1C3 protein and the level of RNA with $IC_{50}$ value in liver cancer cell lines and non-small cell lung cancer (NSCLC) cell lines

1. Experimental materials and methods

1.1 Cell lines

All human cancer cell lines were from American Type Culture Collection (ATCC, Manassas, VA) or Japanese Collection of Research Bioresources (JCRB, Osaka, Japan) or Cobioer Biosciences (NanJing, China).

1.2 *Ex vivo* proliferation assay method

Exponentially growing cells were inoculated. After 24h, the test compound OBI-3424 was added. After addition of the test compound OBI-3424, plates were incubated in a standard tissue incubator at 37°C for the indicated hours. At the end of the experiment, viable cells were detected using the CellTiter Glo (CTG) assay kit or AlamarBlue. Drug concentrations ($IC_{50}$) resulting in 50% growth inhibition relative to untreated control were calculated using XI,fit (IDBS, Boston, MA) or Prism 6 (GraphPad, San Diego, CA).

1.3 Western blotting

Human cell extract was prepared and protein concentration was determined. Proteins were detected using antibodies that recognize human AKR1C3 and tubulin or β-actin. Odyssey Laser Imaging System and software (LI-COR Biosciences, Lincoln, NE) or UVP ChemStudio imaging system and Vision Works software (Analytik Jena AG) were used to scan and quantify the band densities of AKR1C3 and tubulin or β-actin, and the ratio of AKR1C3 to tubulin or β-actin was calculated.

2. Experimental results

After exposing the hepatocellular carcinoma cell lines to compound OBI-3424 for 96h and exposing the NSCLC cell lines to compound OBI-3424 for 72h, the $IC_{50}$ value was determined by *ex vivo* proliferation assay, and the expression of AKR1C3 protein in hepatocellular carcinoma cell lines was determined by Western blotting (tubulin was used as loading control). The expression data of AKR1C3 RNA in the hepatocellular carcinoma cell lines and in the NSCLC cell lines were from CrownBio database (https://db.crownbio.com/Crownbio/OncoExpress_Login.aspx). The results were showed in Tables 2 and 3.

Table 2. Cytotoxicity of OBI-3424 to a group of human hepatocellular carcinoma cell lines after 96h of exposure

| hepatocellular carcinoma cell line | Normalized AKRIC3 expression | The expression level of AKR I C3 protein | The expression level of AKR I C3 RNA (Log2 FPKM) | 3424 $IC_{50}$ (nM) |
|---|---|---|---|---|
| SNU-475 | 1.22 | High | 9.19 | 15 |
| SNU-449 | 1.19 | High | 8.3 | 45 |
| C3A | 0.95 | High | 4.58 | 5 |
| SNU-387 | 0.64 | Medium | 6.42 | 103 |
| PLC/PRF/5 | 0.63 | High | 4.66 | 167 |
| HLE | 0.3 | High | 4.36 | 113 |
| HuCCTI | 0.21 | Low | 0.19 | 696 |
| SNU-182 | 0.17 | Low | -0.05 | >1000 |
| HLF | 0.15 | Low | -1.69 | >1000 |
| SK-HEP-1 | 0.13 | Low | -1.39 | >1000 |
| SNU-398 | 0.08 | Low | -1.33 | >1000 |

Table 3. Cytotoxicity of OBI-3424 to a group of non-small cell lung cancer (NSCLC) cell lines after 72h of exposure

| NSCLC cell line | The expression level of AKR1C3 RNA (Log2 FPKM) | 3424 $IC_{50}$ (nM) |
|---|---|---|
| NCI-H1944 | 11.06 | 2.3 |
| NCI-H2228 | 9.25 | 0.21 |
| NCIH1755 | 9 | 8.2 |
| NCI-H1563 | 8.61 | 2.5 |
| NCI-H2110 | 8.23 | 1.1 |
| NCI-H1792 | 8.07 | 4.5 |
| CAL12T | 3.86 | 29.1 |
| NCIH2106 | 2.68 | >1000 |
| NCI-H23 | -1.98 | >1000 |
| NCI-H522 | -1.88 | >1000 |

As shown in Tables 2 and 3, the hepatocellular carcinoma cell lines with high AKR1C3 expression at both protein and RNA levels were more sensitive to OBI-3424, where $IC_{50}$ values were in the low nanomolar range. On the other hand, cells expressing low AKR1C3 were less sensitive to OBI-3424, where $IC_{50}$ values were higher than 1000 nM. Similarly, NSCLC cells also showed AKR1C3-dependent cytotoxic properties after exposing NSCLC cell to compound OBI-3424 for 72h (Table 3). 3424 $IC_{50}$ in hepatocytes was highly correlated with the level of AKR1C3 protein ($R^2$=0.71,

Fig.1, left), 3424 $IC_{50}$ in hepatocytes was highly correlated with the expression level of AKR1C3 RNA ($R^2$=0.87, Fig.1, middle), and 3424 $IC_{50}$ in NSCLC was highly correlated with the expression level of AKR1C3 RNA ($R^2$=0.80, FIG. 1, right). These results showed that 3424-mediated cytotoxicity in hepatocyte lines and NSCLC cell lines was highly correlated with the expression level of AKR1C3.

Example 2. Correlation between the level of AKR1C3 protein and $IC_{50}$ value in leukemia cell lines

1. Experimental materials and methods

1.1 Cell lines and PDX *ex vivo* studies

All cell lines were purchased from HD Biosciences. All experimental work was conducted under the approval of the respective institutional review board and the animal ethics committee of each institution, and the use of human relevant tissue samples was in accordance with the ethical and relevant legal regulations of the location of the experiments. Serial PDX previously established in 20-25 g female non-obese/SCID (NOD.CB17-Prkdcscid/SzJ, NOD/SCID) or NOD/SCID/IL2 receptor γ-negative (NOD.Cg-$Prkdc^{scid}$ $Il2rg^{tm1Wj1}$/SzJAusb, NSG) was used in the experiments, as described elsewhere (Lock RB, Liem N, Farnsworth ML, Milross CG, Xue C, Tajbakhsh M, et al. The nonobese diabetic/severe combined immunodeficient (NOD/SCID) mouse model of childhood acute lymphoblastic leukemia reveals intrinsic differences in biologic characteristics at diagnosis and relapse. Blood 2002; 99:4100-8.). The development of lentivirus-transduced ALL-11 PDX [empty vector (EV) and AKR1C3 overexpression] has been described previously (Jamieson SM, Gu Y, Manesh DM, El-Hoss J, Jing D, Mackenzie KL, et al. A novel fluorometric assay for aldo-keto reductase 1C3 predicts metabolic activation of the nitrogen mustard prodrug PR-104A in human leukaemia cells. Biochem Pharmacol 2014; 88:36-45.).

1.2 *Ex vivo* cytotoxicity assay

Leukemia cell lines were suspended in RPMI medium supplemented with FBS (Biosera), while ALL PDX cells were cultured in QBSF medium (Quality Biological Inc.,) supplemented with Flt-3 ligand (20 ng/mL, BioNovus Life Sciences) or IL7 (10-20 ng/mL, Jomar Life Research). Cells were inoculated according to the optimal cell density and incubated for 3h or overnight (37°C, 5%$CO_2$). PDX cells and leukemia cell lines were treated with OBI-3424 (10 mmol/L - 1 pmol/L) or medium control for 48 or 72h, respectively. Viability was determined using Alamar Blue reduction assay or Cell Titer-Glo Luminescent Cell Viability Assay (Promega). The half-maximal inhibitory concentration ($IC_{50}$) was used to calculate the interpolation of nonlinear regression curves by GraphPad Prism 7 software.

1.3 Western blotting

Cryopreserved leukemia cells were thawed and lysed in RIPA lysis buffer, and protein concentrations were quantified by BCA assay. Each sample was loaded with 20 μg of protein lysate in NuPAGE 4-12% Bis-Tris protein gel, then electrophoresed at 120V and transferred to polyvinylenedifluoride membrane at 30V for 1h. Mouse anti-AKR1C3 (#A6229, Sigma-Aldrich, St. Louis, MO) or rabbit anti-actin primary antibody (#A2066, Sigma-Aldrich) was used, followed by horseradish peroxidase conjugated anti-mouse or anti-rabbit IgG secondary antibody (GE Healthcare, Buckingham, UK) respectively to probe the membrane. Immobilon Western chemiluminescent HRP substrate (Merck Millipore, Billerica, MA) was used to detect conjugated secondary antibodies by quantifying the signals in the BioRad Chemidoc touch imaging system.

2. Experimental results

The *ex vivo* cytotoxicity of AKR1C3-related OBI-3424 was observed in 11 types of T-ALL cell lines, one B-ALL cell line transfected with granulocyte-colony stimulating factor, and one BCP-ALL cell line. The expression levels of AKR1C3 protein was determined by Western blot analysis. The *ex vivo* cytotoxicity of OBI-3424 was determined using CellTiter-Glo assay and calculated as 50% maximum inhibitory concentration ($IC_{50}$).

*Ex vivo* cytotoxicity demonstrated by OBI-3424, $IC_{50}$ ranged from 3.0 to 30.0 nM in 6 types of cell lines expressing high (strong) levels of AKR1C3. As for cell lines with medium AKR1C3 expression levels, $IC_{50}$ ranged from 3.0 to 84.0 nM (Table 4).

Table 4. AKR1C3-dependent *ex vivo* cytotoxicity of OBI-3424 in ALL cell lines

| Leukemia | Cell line | AKR1C3 expression | $IC_{50}$(nM) |
|---|---|---|---|
| T-ALL (child) | PF-382 | Strong | 15.0 |
| T-ALL (child) | SUP-T1 | Strong | 3.0 |
| T-ALL (child) | TALL-1 | Strong | 30.0 |
| T-ALL (19 years old) | MOLT-4 | Strong | 10.0 |
| T-ALL (child) | CCRF-CEM | Strong | 3.7 |
| T-ALL (child) | Jurkat | Medium | 40.0 |
| T-ALL (child) | Jurkat, Clone E6-1 | Medium | 24.0 |
| T-ALL (child) | NOMO-1 | Medium | 11.0 |
| T-ALL (Jurkat mutant) | P116 | Medium | 84.0 |
| B-ALL (transfected with G-CSF) | GR-ST | Strong | 9.9 |
| BCP-ALL | Reh | Medium | 3.0 |

Note: G-CSF= granulocyte-colony stimulating factor; BCP-ALL= B-cell precursor ALL

In order to evaluate the potential anti-leukemia activity of OBI-3424, *ex vivo* cytotoxicity assays were performed on a variety of leukemia cell lines. It proved that OBI-3424 can treat T-ALL, B-ALL, acute myeloid leukemia, acute promyelocytic leukemia (APL) and erythroleukemia. OBI-3424 showed potent cytotoxicity, in particular against cell lines derived from T-ALL (T-lineage acute lymphoblastic leukemia) with high AKR1C3 expression, where $IC_{50}$ values were in the low nmol/L range (see Table 5). The difference in $IC_{50}$ values between cell lines with high/medium AKR1C3 expression and low expression was statistically significant (P=0.0016).

Table 5. *Ex vivo* cytotoxicity of OBI-3424 to leukemia cell lines after exposing OBI-3424 to leukemia cell lines for 72h

| Cell line | AKRIC3 expression* | IC50(nM) | Disease characteristic |
|---|---|---|---|
| CCRF-CEM | High | 3.7 | T-ALL |
| KG-1 | High | 153 | Erythroleukemia (59 years old) |
| MOLT-4 | Medium | 10 | T-ALL(19 years old) |
| NOMO-1 | Medium | 11 | T-ALL (adult) |
| PF-382 | Medium | 15 | T-ALL (pediatric) |
| SUP-T1 | Medium | 3 | T-ALL (pediatric) |
| TALL-1 | Medium | 30 | T-ALL (pediatric) |
| GR-ST | Medium | 9.9 | B-ALL (transfected with G-CSF) |
| TF-1 | Medium | 2.8 | Erythroleukemia (35 years old) |
| Jurkat | Low | 40 | T-ALL (pediatric) |
| Jurkat, clone E6-1 | Low | 24 | T-ALL |
| P116 | Low | 84 | Jurkat mutant |
| P30/OHK | Low | >1μM | T-ALL (pediatric) |
| HEL | Low | 224 | Erythroleukemia (30 years old) |
| Reh | Low | 3 | ALL (non-T, non-B) |
| HL-60 | Low | 52.6 | APL (36 years old) |
| HL-60, clone 15 | Low | 87 | APL (36 years old) |
| K-562 | Low | >1μM | CML (53 years old) |
| ATN-1 | Low | >1μM | T-ALL (adult) |
| Mono-Mac-6 | Low | 29.8 | AML-M5 (64 years old) |
| THP-1 | Low | >1μM | AML (pediatric) |
| Kasumi-1 | Low | >1μM | AML |
| P31/FUJ | Low | >1μM | AML |

AKR1C3 expression was evaluated by western blotting and relative to control β-tubulin expression: High, AKR1C3/β-tubulin>5.0; medium, AKR1C3/β-tubulin is 2.0-5.0; low, AKR1C3/β-tubulin<2.0.

Similar to the results obtained from leukemia cell lines, OBI-3424 exerted potent cell-killing effects on all leukemia cell lines. Fig.2a showed the cytotoxicity of OBI-3424 against 6 types of B-ALL cell lines, and Fig.2b showed the cytotoxicity of OBI-3424 against 7 types of T-ALL cell lines. It can be seen from Figs.2a and 2b that AKR1C3-dependent activation of OBI-3424 is a DNA alkylation reagent. In addition, as shown in Fig.2c, at a concentration of 10 nmol/L of OBI-3424,, AKR1C3 protein expression showed a significant negative correlation with the cell survival rate *ex vivo* of 18 types of ALL PDX (r=-0.53, P=0.023). Similarly, as shown in Fig.2d, at a concentration of 100 nmol/L of OBI-3424, AKR1C3 protein expression showed a significant negative correlation with the cell survival rate *ex vivo* of 18 types of ALL PDX (r=-0.56, P=0.015).

Example 3. Correlation between the level of AKR1C3 RNA and $IC_{50}$ value in leukemia cell lines

After exposing the hematological cancer cell lines to compound OBI-3424 for 72h, the $IC_{50}$ value was determined by *ex vivo* proliferation assay. The expression data of AKR1C3 RNA in hematological cancer cell lines was from CrownBio database (https://db.crownbio.com/Crownbio/OncoExpress_Login.aspx). The results were showed in Table 6.

Table 6. Cytotoxicity of AST-3424 to a group of human hematological cancer cell lines after 72h of exposure

| Hematological cancer cell line | The expression level of AKR1C3 RNA (Log2 FPKM) | $IC_{50}$(nM) |
|---|---|---|
| CCRF-CEM | 5.9566 | 1.7 |
| SUP-T 1 | 5.1574 | 3.9 |
| PF-382 | 4.0971 | 6 |
| TF-1 | 5.5893 | 16.8 |
| KG-1 | 2.6621 | 153 |
| P31/FUJ | -1.2529 | 1000 |
| Kasumi-1 | -1.6608 | 1000 |
| TALL-1 | 4.0126 | 24 |
| HEL | 2.1127 | 224 |
| THP-1 | -0.4299 | 1000 |
| Jurkat | 1.1763 | 51.1 |

The expression levels of AKR1C3 RNA (Log2 FPKM) and $IC_{50}$ (nM) in Table 6 above were plotted as a curve graph, as shown in Fig.3d. A curve fitting between $IC_{50}$ and the algebraic transformation value Log2 FPKM of AKR1C3 RNA expression level was performed to obtain the corresponding curve fitting formula. The correlation coefficient $R^2$=0.7889, which shows that the expression levels of AKR1C3 RNA have a significant linear correlation with the $IC_{50}$ values.

Example 4. Correlation between the level of AKR1C3 protein and the level of AKR1C3 RNA in leukemia cell lines

1. Experimental materials and methods

1.1 Cell lines and PDX *ex vivo* studies

All cell lines were purchased from HD Biosciences. All experimental work was conducted under the approval of the respective institutional review board and the animal ethics committee of each institution. Serial PDX previously established in 20-25 g female non-obese/SCID (NOD.CB17-Prkdcscid/SzJ, NOD/SCID) or NOD/SCID/IL2 receptor γ-negative (NOD.Cg-$Prkdc^{scid}$ $Il2rg^{tm1Wj1}$/SzJAusb, NSG) was used in the experiments, as described elsewhere (Lock RB, Liem N, Farnsworth ML, Milross CG, Xue C, Tajbakhsh M, et al. The nonobese diabetic/severe combined immunodeficient (NOD/SCID) mouse model of childhood acute lymphoblastic leukemia reveals intrinsic differences in biologic characteristics at diagnosis and relapse. Blood 2002; 99:4100-8.). The development of lentivirus-transduced ALL-11 PDX [empty vector (EV) and AKR1C3 overexpression] has been described previously (Jamieson SM, Gu Y, Manesh DM, El-Hoss J, Jing D, Mackenzie KL, et al. A novel fluorometric assay for aldo-keto reductase 1C3 predicts metabolic activation of the nitrogen mustard prodrug PR-104A in human leukaemia cells. Biochem Pharmacol 2014; 88:36-45.).

1.2 Western blotting

Cryopreserved leukemia cells were thawed and lysed in RIPA lysis buffer, and protein concentrations were quantified by BCA assay. Each sample was loaded with 20 μg of protein lysate in NuPAGE 4-12% Bis-Tris protein gel, then electrophoresed at 120V and transferred to polyvinylenedifluoride membrane at 30V for 1h. Mouse anti-AKR1C3 (#A6229, Sigma-Aldrich, St. Louis, MO) or rabbit anti-actin primary antibody (#A2066, Sigma-Aldrich) was used, followed by horseradish peroxidase conjugated anti-mouse or anti-rabbit IgG secondary antibody (GE Healthcare, Buckingham, UK) respectively to probe the membrane. Immobilon Western chemiluminescent HRP substrate (Merck Millipore, Billerica, MA) was used to detect conjugated secondary antibodies by quantifying the signals in the BioRad Chemidoc touch imaging system.

1.3 RNA-Seq analysis

In order to analyze AKR1C3 expression in aspirates from primary patients, the patients were divided into B-ALL and T-ALL and their related subgroups. Paired end readings were mapped by STAR (Dobin A, Davis CA, Schlesinger F, Drenkow J, Zaleski C, Jha S, et al. STAR: ultrafast universal RNA-seq aligner. Bioinformatics 2013;29:15-21.) to GRCh37 human genome reference via a recommended round-trip mapping pipeline with default parameters, and Picard MarkDuplicates module was used to mark the duplication rate. Gene annotation files were downloaded from Ensembl (http://www.ensembl.org/) for STAR localization and subsequent evaluation of gene expression levels. In order to evaluate gene expression profiles, the count of readings of annotated genes was invoked by HTSeq (Anders S, Pyl PT, Huber W. HTSeq-a Python framework to work with highthroughput sequencing data. Bioinformatics 2015;31:166-9.) and processed by the DESeq2 R program package (Anders S, Huber W. Differential expression analysis for sequence count data. Genome Biol 2010;11:R106.) to normalize gene expression to a regularized $\log_2$ value.

In order to analysis PDX samples, Illumina paired end RNA-seq data were aligned to human genome assembly (construct hg38) using STAR with the quantMode parameters set to TranscriptomeSAM, and the alignment was converted to transcript coordinates. The alignment was run with rsem-calculate-expression commanded by RSEM (version 1.2.31) to calculate raw gene counts, TPM, FPKM, and isoform expression. All RNA-seq values were expressed as number of fragments per kilobase million (FPKM). The FPKM data were performed with $\log_2$ conversion.

2. Experimental results

The expression levels of AKR1C3 mRNA and protein in various types of ALL were detected by RNA-Seq analysis (Fig.3a) and Western blotting (Fig.3b). The analysis of the results confirmed that T-ALL (n=25) had significantly higher AKR1C3 expression as compared with B-ALL (n=65; P<0.0001). Moreover, there was a significant correlation between AKR1C3 mRNA and protein expression (r=0.58; P=0.0003; See Fig.3c).

Example 5. Design and screening of primers and probes

5.1 Experimental materials

5.1.1 Sample information

The cell lines used in this example were purchased from Nanjing Cobioer Biosciences Co., Ltd and National Infrastructure of Cell Line Resource; Leukemia samples were obtained from acute lymphoblastic leukemia patient volunteers (clinical) and normal samples were obtained from healthy volunteers (donors), and the volunteers had signed informed consent. Fresh blood samples were preserved in PAXGENE tubes and stored in a refrigerator at -20°C.

The collection, use and subsequent processing of all human samples were conducted in accordance with Chinese relevant regulatory regulations of the location of the experiments and in accordance with the relevant requirements of international ethics.

Table 7. Sample information table

| Sample type | Sample number | Sample type | Sample source |
|---|---|---|---|
| Normal sample (11 cases) | 1 | Peripheral blood | Donor |
| | 2 | Peripheral blood | Donor |
| | 3 | Peripheral blood | Donor |
| | 5 | Peripheral blood | Donor |
| | 6 | Peripheral blood | Donor |
| | 7 | Peripheral blood | Donor |
| | 8 | Peripheral blood | Donor |
| | 9 | Peripheral blood | Donor |
| | 10 | Peripheral blood | Donor |
| | 11 | Peripheral blood | Donor |
| | 12 | Peripheral blood | Donor |
| Cell line sample (5 cases) | TF-1 | Human erythroid leukemia cells | National Infrastructure of Cell Line Resource (NICR, Website of the platform: http://www.cellresource.cn/) |
| | CCRF-CEM | Human T-cell leukemia cells | National Infrastructure of Cell Line Resource |
| | RPMI8226 | Multiple myeloma cells | Nanjing Cobioer Biosciences (Website of the company: www.cobioer.com) |
| | MOLT-4 | Human acute T lymphoblastic leukemia cells | National Infrastructure of Cell Line Resource |
| | Jurkat | Human T-cell leukemia cells | Nanjing Cobioer Biosciences |
| Leukemia sample (10 cases) | GZJ | ALL peripheral blood | Clinical |
| | HCL | B-ALL peripheral blood | Clinical |
| | HXB | B-ALL peripheral blood | Clinical |
| | JRZ | B-ALL peripheral blood | Clinical |
| | LBJ | T-ALL peripheral blood | Clinical |
| | LSX | B-ALL peripheral blood | Clinical |
| | PSS | B-ALL peripheral blood | Clinical |
| | ZYQ | B-ALL peripheral blood | Clinical |
| | BZQ | B-ALL bone marrow | Clinical |
| | QQY | B-ALL bone marrow | Clinical |
| Plasmid | AKR1C3 | Plasmid (concentration: 16.7ng/ul, the length of the inserted element: 355bp, the total length of the plasmid: 3065bp) | Sangon Biotech (Shanghai) Co., Ltd. |
| | ACTB | Plasmid (concentration: 57.8ng/ul, the length of the inserted element: 500bp, the total length of the plasmid: 2606bp) | Sangon Biotech (Shanghai) Co., Ltd. |

5.1.2 Instrument information

Table 8. Instrument platform information

| Instrument | Manufacturer | Model |
|---|---|---|
| Fluorescence quantitative PCR instrument | Thermo Fisher | ABI7500 |
| Nucleic acid quantitative instrument | Life | Qubit 3.0 |
| Digital PCR instrument | Thermo Fisher | QuantStudio 3D Digital PCR |

5.1.3 Reagent information

Table 9. Reagent information

| Reagent | Manufacturer | Model | Batch number | Expiry date |
|---|---|---|---|---|
| QIAamp RNA Blood Mini Kit | Qiagen | 52304 | 166034744 | 13 months |
| PAXgene Blood RNA Kit-IVD | Qiagen | 762174 | 166011286 | 7 months |
| SuperScript™ VILO™ Master Mix | Thermo Fisher | 11755050 | 2200704 | 6 months |
| KAPA PROBE FAST RT-PCR Master Mix(2x) | KAPA | KK4702 | 0000100987 | 9 months |
| dUTP | TAKARA | 4020 | B301E | 12 months |
| UNG | TAKARA | 2820 | AK40810A | 12 months |

5.2 Nucleic acid extraction

The RNA extraction kit was Qiagen made-up extraction kit: PAXGENE BloodRNA Kit (catalog number: 762174, used for blood preserved in PAXGENE tubes) and Qiagen made-up extraction kit: QIAamp RNA Blood Mini Kit (catalog number: 52304, used for blood preserved in EDTA tubes).

According to the instructions, RNA extraction was performed on blood preserved in PAXgene tubes using an extraction and purification Kit (PAXgene Blood RNA Kit-IVD), and RNA extraction was performed on blood preserved in EDTA tubes using QIAamp RNABlood Mini Kit. Concentration detection was performed on the extracted RNA using the recommended RNA nucleic acid quantification kit (Qubit RNA HS Assay Kits, Thermo Fisher Scientific, Q32852).

5.3 Reverse Transcription

4 μL of AKR1C3 reverse transcriptase mixture (Superscript VII,O MARSTER MIX from Thermo Fisher (catalog number: 11755050)) was absorbed and added to 16 μL of RNA sample (replenish water to 16 μL if the volume was insufficient), slightly vortexed and mixed, and then centrifuged transiently. PCR tubes containing the reaction solution were placed in PCR instrument, the PCR program was set for the synthesis of cDNA.

5.4 Design and selection of AKR1C3 primers

9 pairs of primers and probes were designed for a total of 3 target regions of AKR1C3. The position information of primer-probes can be seen in Fig.4. AKR1C3 gene primers were designed and synthesized by Contract Research Organization (CRO).

5.5 Selection of polymerase and reverse transcriptase

Polymerase mixture is a key component of PCR success or failure and affects the efficiency of amplification. The main components of polymerase mixture comprise: polymerase, $MgCl_2$, buffer and dNTPs. The above components in commercially available polymerase mixture have been optimized to the best state in a certain proportion to make concentrated mother liquor. When using, the corresponding detection can be carried out as long as the polymerase buffer was added in proportion according to the multiplier. The polymerase KAPA PROBE FAST RT-PCR Master Mix (2x) (catalog number: KK4702) which has been repeatedly verified to have good amplification efficiency, was used in this example.

In each PCR reaction, it must be detected and analyzed together with positive reference and negative control (NC, nuclease-free water). qPCR detection process was as follows:

1) taking out AKR1C3 polymerase mixture, AKR1C3 reaction solution and references, mixing by shaking, transiently centrifuging for future use.

2) according to the number of samples to be detected and the total number of reaction tubes required for quality control, 11.1 μL of AKR1C3 qPCR polymerase mixture, 2.6 μL of AKR1C3 reaction mixture and 4.3 μL of nuclease-free water were respectively included in each test, which were mixed by vortex and transiently centrifuged, and then divided into qPCR eight-row tubes with 18 μL of each tube. (Note: both of the samples to be detected and the quality control need to be repeated twice, and the detection number of NTC should be≥1.).

3) adding cDNA of samples to be detected, quality control and references in corresponding qPCR eight-row tubes, respectively, covering eight-row caps, mixing by vortex and transiently centrifuging.

4) placing eight-row tubes into the sample tank of qPCR instrument and recording the order of placement, and setting the instrument amplification program according to the optimized reaction conditions for amplification.

5) after the completion of the experiment, wrapping the PCR reaction strips with 2 layers of PE gloves and treating them as biological waste. The caps of PCR tubes were forbidden to open to prevent contamination.

5.6 Selection of primer-probes

5.6.1 Screening test of AKR1C3 primer-probes

The above steps 5.2-5.5 were adopted, 9 pairs of primer-probes were amplified first, the amplification results were shown in Fig.5.

Total RNA was extracted from the peripheral blood of healthy volunteers, and cDNA was synthesized via reverse transcription. It can be seen from the amplification results that F1R1P1, F2R2P1, F2R6P1, F6R2P1, F6R6P1 and F3R3P1 were higher than other F5R5P2, F4R3P2 and F7R7P3 in terms of fluorescence intensity values.

Considering the specificity of the primer-probes, BLAST was performed on 9 pairs of primer, and F2R6P1, F6R2P1 and F6R6P1 displayed better specificity, and no nonspecific products appeared.

5.6.2 Actual sample test of AKR1C3 primer-probes

cDNA was obtained by reverse transcription with total RNA extracted from peripheral blood of healthy volunteers using 3 sets of primer-probes (F2R6P1, F6R2P1 and F6R6P1), and 6 samples were selected for testing. The results were shown in the following table.

Table 10. Actual sample test results of primer-probes

| Primer pair | F2R6 | | F6R2 | | F6R6 | |
|---|---|---|---|---|---|---|
| Sample number | FAM (Replicate 1) | FAM (Replicate 2) | FAM (Replicate 1) | FAM (Replicate 2) | FAM (Replicate 1) | FAM (Replicate 2) |
| 6 | 29.00 | 29.02 | 29.03 | 28.95 | 28.97 | 28.88 |
| 7 | 29.19 | 29.14 | 29.07 | 29.15 | 29.11 | 29.18 |
| 8 | 28.33 | 28.29 | 28.27 | 28.31 | 28.36 | 28.41 |
| 9 | 27.41 | 27.25 | 27.20 | 27.26 | 27.24 | 27.36 |
| 10 | 27.47 | 27.45 | 27.42 | 27.51 | 27.47 | 27.55 |
| 11 | 28.86 | 28.58 | 28.61 | 28.60 | 28.65 | 28.82 |
| 12 | 29.51 | 29.36 | 29.53 | 29.61 | 29.68 | 29.51 |
| NTC | N/A | N/A | N/A | N/A | N/A | N/A |

As can be seen from Table 10 that Ct value of the target gene AKR1C3 expression is relatively stable in the range of 27 to 30 in healthy population.

5.6.3 Amplification efficiency test of AKR1C3 primer-probes

cDNA was obtained by reverse transcription with RNA extracted from peripheral blood of one healthy volunteer using 3 sets of primer-probes (F2R6P1, F6R2P1 and F6R6P1) for amplification efficiency test. The concentration of cDNA was 100ng/μL, after 5-fold dilution, it was further diluted 25-fold, 125-fold and 625-fold, and there were a total of four concentration gradients for 5 to 625-fold, with two replicates for each concentration for qPCR testing. The standard curve and amplification efficiency were determined based on the linear relationship between Ct value and log value of initial concentration. The results were shown in Fig.6.

It can be seen from the results that the amplification efficiency of F6R2P1 and F6R6P1 was higher than that of F2R6P1, with greater than 95%. Therefore, F6R2P1 and F6R6P1 primer-probes were selected for subsequent tests.

RNA samples extracted from the blood of 4 healthy volunteers were selected for amplification efficiency test of F6R2P1 and F6R6P1, respectively. The results were shown in the following table:

Table 11. More sample standard curve tests of F6R2P1 and F6R6P1

| Primer-probe pair | F6R2P1 | | | | F6R6P1 | | | |
|---|---|---|---|---|---|---|---|---|
| Sample number | 1 | 2 | 3 | 5 | 1 | 2 | 3 | 5 |
| Target efficiency | 81.90% | 75.50% | 88.50% | 81.40% | 83.20% | 70.20% | 89.50% | 84% |
| $R^2$ (squared correlation coefficient of sample standard curve fitting) | 0.998 | 0.993 | 1.000 | 0.999 | 0.999 | 0.995 | 0.996 | 0.996 |

According to the results, it can be seen that there was little difference in the amplification efficiency of the 2 pairs of primer-probes, and there were slight fluctuations in different samples. However, $R^2$ of F6R2P1 was better, and the $R^2$ was equal to 1 in sample 3. Therefore, F6R2P1 was selected as the primer-probe of AKR1C3 in this kit.

5.6.4 Standard curve test of AKR1C3 plasmids

After dissolving in 40μL nuclease-free water, the AKR1C3 plasmid dry powder was diluted 10-fold to a concentration of 5.78ng/μL measured by Qubit. The plasmid stock solution was diluted by gradient, which was $10^5$, $10^6$, $10^7$ and $10^8$-fold, respectively, to obtain a total of 4 gradient concentrations of samples for qPCR test. Each gradient was repeated twice, and the standard curve of AKR1C3 was measured as shown in Fig.7.

As can be seen from the standard curve in Fig.7, the amplification efficiency of AKR1C3 primer-probe reached 100%, and $R^2$=0.999, suggesting the good amplification efficiency.

Example 6. Screening of reference genes as well as design and screening of corresponding primers and probes

As for the selection of reference genes, the references on leukemia gene expression ([1] Yanlan Wang, Yue Liu, Changhua Zhou et al. An AKR1C3-specific prodrug with potent antitumor activities against T-ALL. [J] .Leukemia&Lymphoma, 2020, Feb. DOI: 10.1080/10428194.2020.1728746; [2] Donya Moradi Manesh, Jad El-Hoss, Kathryn Evans et al. Growth factor AKR1C3 is a biomarker of sensitivity to PR-104 in preclinical models of T-cell acute lymphoblastic leukemiaas treatment targets in clinical oncology.[J]. BLOOD, 2015, 125: 1193-1201; [3] Yuantong Tian, Lijing Zhao, Haitao Zhang et al. AKR1C3 overexpression may serve as a promising biomarker for prostate cancer progression. [J]. Diagnostic Pathology, 2014, 9: 42.) were referred to, and in combination with the primer-probes previously used, 4 reference genes (HPRT1, GAP, ACTB and GOLGA1) were selected for testing with the cell line CCRF-CEM to evaluate their fluorescence intensity and amplification. The results were shown in Fig.8.

In terms of fluorescence intensity: ACTB was the highest, followed by HPRT1.

In terms of amplification curves: the amplification curve of GOLGA1 are not typical S-shaped, and the other three are typical S-shaped amplification curves.

In terms of Ct: the Ct values of GAP and ACTB were low and their expression levels were relatively high, while the Ct values of HPRT1 and GOLGA1 were high and their expression levels were relatively low.

Samples and cell lines were tested to verify their expression stability and whether high and low expression samples could be distinguished.

RNA numbers 5, 6 and 7 of blood samples from healthy volunteers, bone marrow RNA numbers BZQ and QQY of leukemia patients, and cell lines expressing AKR1C3 (RPMI8226, CCRF-CEM and Jurkat) were used in this experiment. According to the data and previous results, the cell lines RPMI8226 and CCRF-CEM were high-expression cell lines, and Jurkat was low-expression cell line (as shown in Fig.9).

ΔΔCt calculating method was adopted for the experimental results, i.e., ΔΔCt= unknown sample FAM Ct- unknown sample VIC Ct- (quality control FAM Ct- quality control VIC Ct), and CCRF-CEM was used as quality control in each group. The results were shown in Fig.10.

It can be seen from the results that ACTB can better distinguish high-expression cell lines RPMI8226 and CCRF-CEM from low-expression cell line Jurkat when ACTB was used as the reference gene compared with the other three reference genes. In healthy people, the values remained relatively low level, while high expression of AKR1C3 was detected in bone marrow RNA samples of leukemia patients. ACTB was selected as the reference gene based on the results of the amplification curve.

After ACTB was determined as the reference gene, 5 types of cell lines were tested, respectively, and the test results were shown in Fig. 11.

It can be seen from the results that the test results of the cell lines were consistent with the validation results of Western blot (Fig. 9), which proved the accuracy of the test results of AKR1C3-ACTB primer-probes.

Example 7. Study of qPCR reaction system and conditions

The quality of qPCR reaction system was a necessary condition for successful development and detection, and its main components must be optimized.

7.1 Determination of reaction system

The reverse transcription system was shown below:

Table 12. Reverse transcription system

| Reverse transcription system | Amount (20μL) |
|---|---|
| RNA | Up to 2ug |
| Reverse transcriptase | 4μL |
| Nuclease-free water | Replenish to 20μL |

The reverse transcription reaction conditions were shown below:

Table 13. Reverse transcription reaction conditions

| Reaction temperature | Reaction time |
|---|---|
| 25°C | 10min |
| 42 °C | 60min |
| 85 °C | 5min |
| 10 °C | Hold |

Polymerase mixture was the key component that affects PCR amplification efficiency. Contained components of the polymerase mixture KAPA PROBE FAST RT-PCR Master Mix (2x) used in this example have been optimized to the best state at a certain ratio to make concentrated mother liquor. When in use, the corresponding detection can be carried out as long as the polymerase buffer was added in proportion according to the multiplier. According to the instructions, the recommended amount was half of the reaction system, i.e., in the reaction system of 20μL, the amount should be 10μL. The specific system was shown in the following table:

Table 14. Preparation of AKR1C3 qPCR reaction system

| | qPCR reaction system | Amount (μL) |
|---|---|---|
| AKR1C3-F6 (10μM) | | 0.5 |

(continued)

| | qPCR reaction system | Amount (μL) |
|---|---|---|
| | AKR1C3-R2 | 0.5 |
| | ACKR1C3-P1 | 0.3 |
| | ACTB-F1 | 0.5 |
| | ACTB-R1 | 0.5 |
| | ACTB-P1 | 0.3 |
| | dUTP (2mM) | 1 |
| | UNG enzyme | 0.1 |
| cDNA template (Converted according to the amount of RNA) | | 20-50ng |
| | Polymerase mixture | 10 |
| | $H_2O$ | Replenish to 20 μl |

7.2 Optimization of qPCR reaction conditions

The reaction conditions used in this example were shown in the following table 15. The reaction conditions were tested as the preferred reaction conditions for RNA reaction after long-term verification in the early stage.

Table 15. Amplification reaction conditions

| System | The total volume was 20μl | | | |
|---|---|---|---|---|
| Collection | Fluorescence signal was collected by AKR1C3 gene-FAM channel. Fluorescence signal was collected by ACTB reference gene-VIC channel. Both signals were collected at the same time. | | | |
| Amplification procedure | Cycle number | Temperature (°C) | Time | Fluorescence signal collection |
| 1 | 1 cycle | 37°C | 3min | NO |
| 2 | 1 cycle | 95 °C | 3min | NO |
| 3 | 40 cycle | 95 °C | 15s | NO |
| | | 60 °C | 45s | YES |

Cycle number was one of the direct factors affecting the results of PCR, and the cycle number determined the degree of PCR amplification. The cycle number of qPCR used was usually between 30 and 45 cycles. The more cycles there were, the more significant the non-specific amplification was, while the less cycles there were, the more significant was the effect on detection sensitivity.

7.3 Standard curve test of AKR1C3-ACTB system

7.3.1 Standard curve test of cell lines

In optimized reaction system, the primer-probes of AKR1C3 and ACTB were mixed, and the test of standard curve was conducted to investigate the amplification efficiency of the primer-probes. CCRF-CEM was used as the cell line, and reverse transcription was carried out according to the reverse transcription system of section 7.1. After cDNA was obtained, four gradients of stock solution, 5, 25 and 125-fold dilution were obtained according to the 5-fold gradient dilution with two replicates for each concentration and the detection was performed by qPCR reaction system. The standard curve was obtained based on the linear relationship between the Ct value and initial copy number lg value, as shown in Fig. 12.

It can be seen from Fig. 12 that the amplification efficiency of AKR1C3 primer-probe was 94.2%, and that of ACTB primer-probe was 95.3%, both of them met the requirements. $R^2$ was greater than 0.99, suggesting that the linear relationship was good and met the requirements.

7.3.2 Standard curve test of plasmids

The AKR1C3 and ACTB plasmid dry powders used were dissolved in 40μL nuclease-free water. After diluting

10-fold, the concentrations were measured by Qubit to be 1.67ng/μL, 5.78ng/μL, respectively. The plasmid of AKR1C3 was diluted to $10^{-5}$, and the plasmid of ACTB was diluted to $10^{-3}$. 100μL of each plasmid was mixed, and then the gradient of 10, 100 and 1000-fold was diluted. Each gradient was repeated twice for qPCR test. The standard curve was made according to the linear relationship between Ct value and log value of initial concentration. The results were shown in Fig.13.

It can be seen from the result in the figures that the amplification efficiency of AKR1 C3 reached 101.9% and that of ACTB reached 94.1%, with $R^2$ greater than 0.99, meeting the requirements.

Example 8. Digital PCR test of AKR1C3-ACTB primer-probes

8.1 Digital PCR detection system and reaction conditions

A digital PCR test using AKR1C3-ACTB primer-probes was tried in this example. Since the expression levels of AKR1C3 and ACTB in samples and cell lines were quite different, the maximum Ct difference may reach 10 Ct values. Fig. 14 showed the qPCR detection diagram of Jurkat cell line with low expression.

Therefore, in digital PCR detection, the copy number of VIC will be much larger than that of FAM, as shown in Fig.15.

In view of this, AKR1C3 and ACTB were detected separately. Two cell lines, CCRF-CEM and MOLT-4, were taken as examples. After 2ug RNA was reverse transcribed, the transcribed product was diluted 5-fold, and the copy number of AKR1C3 was detected by digital PCR using the following system:

Table 16. AKR1C3 digital PCR detection system

| Reagent | Amount (μL) |
|---|---|
| AKR1C3-F6 (10uM) | 1.2 |
| AKR1C3-R2 | 1.2 |
| AKR1C3-P1 | 0.3 |
| 2×3D LIFE MIX | 7.5 |
| cDNA | 0.5 |
| $H_2O$ | 4.3 |

The reaction procedure of the digital PCR was shown in the following table:

Table 17. Digital PCR reaction conditions

| Cycle number | Procedure | Amount (μL) |
|---|---|---|
| 1 | 96°C | 10min |
| 39 | 60 °C | 2min |
| | 98°C | 30s |
| 1 | 60 °C | 3min |
| | 10 °C | ∞ |

The detection results were shown in Fig. 16, respectively.

As for the detection of ACTB, 5-fold dilution of cDNA was required, followed by 200-fold dilution, and then the copy number of ACTB was detected using the following system:

Table 18. ACTB digital PCR detection system

| Reagent | Amount (μL) |
|---|---|
| ACTB-F1 (10uM) | 1.2 |
| ACTB-R1 | 1.2 |
| ACTB-P1 | 0.3 |
| 2×3D LIFE MIX | 10 |
| cDNA | 0.5 |

(continued)

| Reagent | Amount (μL) |
| --- | --- |
| $H_2O$ | 4.3 |

The detection results were shown in Fig.17. It can be seen from the results that FAM copy number/VIC copy number of cell line CCRF-CEM was 0.0092, and FAM copy number/VIC copy number of cell line MOLT-4 was 0.00129.

8.2 Actual sample test and determination of calculation method

Sample No. 9 of healthy volunteer, 8 cases of leukemia patients (numbered as PSS, GZJ, HXB, HCL, ZYQ, LBJ, JRZ, LSX), and 5 types of cell lines (TF-1, CCRF-CEM, RPMI8226, MOLT-4, Jurkat) were used. The plasmid and concentration used in the standard curve were as described in step 7.3.2 of Example 7. The preparation method was as follows: the plasmid of AKR1C3 was diluted to $10^{-4}$ and the plasmid of ACTB was diluted to $10^{-2}$; 100μL of each plasmid was mixed, and then the gradient of 10, 100 and 1000-fold was diluted. The copy numbers were quantified by digital PCR. qPCR tests were performed jointly, with two replicates for each sample. The copy numbers of AKR1C3 and ACTB of each unknown sample can be obtained by standard curve, and the ratio of AKR1C3 copy number/ACTB copy number was used as the way to measure the expression of AKR1C3. The measured standard curve was shown in Fig. 18.

It can be seen from Fig. 18 that the amplification efficiency of AKR1C3 reached 99.1% and that of ACTB reached 93.4%, with $R^2$ greater than 0.99, meeting the requirements.

The sample test results were shown in the following table:

Table 19. Sample test results

| Sample Name | Target Name | $C_T$ | Quantity | The ratio of FAM/VIC copy number |
| --- | --- | --- | --- | --- |
| 9 | AKRIC3 | 25.87238503 | 4631 | 0.00394 |
| 9 | ACTB | 19.45120239 | 1176619 | |
| c-8226 | AKR1C3 | 24.11899948 | 15491 | 0.00939 |
| c-8226 | ACTB | 18.93831253 | 1650472 | |
| c-CCRF | AKRIC3 | 21.87590218 | 72603 | 0.00710 |
| c-CCRF | ACTB | 16.17395782 | 10227160 | |
| c-JUR | AKRIC3 | 28.83884048 | 600 | 0.00029 |
| c-JUR | ACTB | 18.57073402 | 2103491 | |
| c-MOLT-4 | AKRIC3 | 26.2293644 | 3621 | 0.00177 |
| c-MOLT-4 | ACTB | 18.61536598 | 2042448 | |
| c-TF-1 | AKRIC3 | 22.73562241 | 40163 | 0.01365 |
| c-TF-1 | ACTB | 18.06160355 | 2943309 | |
| GZJ | AKRIC3 | 20.59411812 | 175517 | 0.00266 |
| GZJ | ACTB | 13.34858036 | 65976364 | |
| HCL | AKRIC3 | 27.93593407 | 1118 | 0.00014 |
| HCL | ACTB | 16.5698204 | 7876216 | |
| HXB | AKRIC3 | 25.07105827 | 8041 | 0.00023 |
| HXB | ACTB | 14.28288174 | 35616992 | |
| JRZ | AKRIC3 | 27.13578415 | 1940 | 0.00054 |
| JRZ | ACTB | 17.76814651 | 3572138 | |
| LBJ | AKRIC3 | 24.13855743 | 15283 | 0.01207 |
| LBJ | ACTB | 19.34033203 | 1265921 | |

(continued)

| Sample Name | Target Name | $C_T$ | Quantity | The ratio of FAM/VIC copy number |
|---|---|---|---|---|
| LSX | AKRIC3 | 20.65554237 | 168247 | 0.00238 |
| LSX | ACTB | 13.24556828 | 70616680 | |
| PSS | AKRIC3 | 20.97867584 | 134680 | 0.00315 |
| PSS | ACTB | 14.00572395 | 42764056 | |
| ZYQ | AKRIC3 | 20.80658913 | 151625 | 0.00296 |
| ZYQ | ACTB | 13.72992897 | 51299136 | |

The results of the FAM copy number /VIC copy number were shown in Fig. 19.

As can be seen from Fig. 19, the detection results of cell lines were consistent with the results of Western blot of cell lines, which verified the accuracy of the calculation method. At the same time, samples with high AKR1C3 expression were detected in the samples of leukemia patients, as shown by LBJ in the figure. The 8 cases of patients were all patients who had received bone marrow transplantation.

Based on the studies of the above AKR1C3 primer-probe design, reaction system and reaction conditions, the detection method of AKR1C3 expression at RNA level was established, and certain sensitivity and accuracy were achieved.

Example 9. Performance test of the method for detecting AKR1C3 RNA content

According to examples 5-8, the best primer-probe composition, reference gene and corresponding primer-probe composition, reaction system and reaction conditions in qPCR method and digital PCR method were determined. Based on the above conditions, the performance test was performed in this example by combining qPCR method and digital PCR method, including sensitivity, specificity, repeatability, accuracy and the like.

The qPCR reaction process included the following steps:

(1) Sample preparation
The concentration of RNA should be more than 5ng/μL, 20~200ng RNA was taken and replenished to a certain volume with nuclease-free water;

(2) Reverse transcription
As shown in Table 12, the reverse transcription mixture was successively added to the above PCR tubes, and the mixture was mixed by vortex or by blowing with a pipettor. After short centrifugation, the mixture was placed on an ordinary PCR instrument. Reverse transcription was performed as shown in Table 13, and the reverse transcription product cDNA was used as the template of qPCR reaction system and could be stored at -20 °C.

(3) Preparation of primer working solution

Dry powders of primers and probes were dissolved in TE buffer for 2h at room temperature (or dissolved overnight at 4 °C). The concentration of primer working solution was 10μM. After dissolving, the primers and probes were mixed according to a certain proportion and combination. After mixing thoroughly, it was stored in refrigerator at -20 °C. The primer working solution can be stably stored at -20 °C for 1 year.

Before each use, the centrifuge tube containing DNA primer amplification mixture was shaken and mixed, then centrifuged transiently. According to the indicated quantity (20μL/reaction), the required amount for single-use was dispensed and reserved.

(4) Preparation of reaction system

Positive control product (STD), negative control product (Neg) and blank control product (NTC) should be used for quality control each time. The qPCR reaction system was prepared according to Table 14, mixed and centrifuged transiently. Then qPCR amplification was performed under the amplification conditions as shown in Table 15.

The corresponding meanings of the sample numbers involved in the following tests are: W1- standard with no AKRIC3 expression, P1- strong positive cell lines with AKR1C3 expression, P2- gradient of standards for AKR1C3-reference with different copy numbers, which were used to detect standard curves, P3- weak positive cell lines with AKR1C3 expression, S1- AKR1C3 standard with the lowest detection limit, R1- strong positive cell lines with AKR1C3 expression, R2-standard with no AKR1C3 expression.

9.1 Lowest detection limit

9.1.1 Detection method

RNA extracted from cell lines with low expression was selected for reverse transcription, and the amount of RNA was 2μg. cDNA after reverse transcription was diluted 25-fold as the lowest detection limit reference, the copy numbers of reference were determined by digital PCR, and each reference was repeated for 10 times.

9.1.2. Confirmation process and result of the copy number of lowest detection limit reference

1) preparation and packaging of PCR mix: 7.5 μL of PCR master mix (per assay), 3 μL of Primer mix, and the total amount of water required to be added were taken, mixed by vortex, centrifuged, and dispensed into PCR reaction tubes.

2) sample addition: 0.5μL of samples to be detected was added to the corresponding PCR reaction tube respectively, mixed by vortex, then centrifuged.

3) sample loading: the corresponding number of chips was prepared and 14.5μL of reaction liquid was taken for loading. Noted that do not touch the center of chip and the outer covers of chip during this process. The chip was pressed for at least 20 s. The mineral oil was added slowly and noted that do not spill the oil. The chip was sealed after adding mineral oil.

4) on-line testing: the chips were arranged on the shelf of digital PCR instrument and noted that the two-dimensional code and the number on the shelf should towards yourself.

5) chips reading: after the chips were removed from the special PCR instrument and placed on room temperature, the chips were put into the chip scanner to scan the fluorescence signal. The computer calculated the mutation ratio according to the fluorescence signal.

6) detection results:
copy number calculation: target copy number =copies (target) × 14.5/ loading amount.

Fig. 20 showed the test results of 5-fold dilution of cDNA in this cell line, which was 261.8 copies/μL, and the copy number of detection limit reference was 52.4 copies/μL.

9.1.3 qPCR verification results

Table 20. Detection results of detection limit reference

| Sample Name | Target Name | $C_T$ | CV | Quantity | Target Name | $C_T$ | CV | Quantity | FAM/VIC copy number |
|---|---|---|---|---|---|---|---|---|---|
| Jurkat | AKR1C3 | 31.97866 | | 73 | ACTB | 21.659 | | 303652 | 0.000239 |
| Jurkat | AKR1C3 | 32.12123 | | 66 | ACTB | 21.36 | | 372154 | 0.000177 |
| Jurkat | AKR1C3 | 32.34266 | | 56 | ACTB | 21.626 | | 310465 | 0.000181 |
| Jurkat | AKR1C3 | 32.17264 | | 63 | ACTB | 21.562 | | 324296 | 0.000195 |
| Jurkat | AKR1C3 | 31.96986 | 0.584% | 73 | ACTB | 21.531 | 0.688% | 331262 | 0.000221 |
| Jurkat | AKR1C3 | 32.4185 | | 53 | ACTB | 21.549 | | 327325 | 0.000163 |
| Jurkat | AKR1C3 | 31.81558 | | 81 | ACTB | 21.232 | | 405964 | 0.000201 |
| Jurkat | AKR1C3 | 32.25435 | | 60 | ACTB | 21.534 | | 330640 | 0.000181 |
| Jurkat | AKR1C3 | 31.99182 | | 72 | ACTB | 21.52 | | 333726 | 0.000216 |
| Jurkat | AKR1C3 | 32.19634 | | 62 | ACTB | 21.259 | | 398636 | 0.000156 |

As can be seen from Table 20, the lowest detection limit reference was detected by qPCR with 10 times repeat. The positive detection rate was 100%, and the lowest detection limit met the verification standard.

9.2 Precision

Precision referred to the degree of closeness between multiple parallel test results under the same conditions. The closer the test values were to each other, the higher the precision. Precision reflected the repeatability of the kit operation. In this example, negative references, precision references with weak expression, and a precision references with high expression were selected to determine the intra-batch precision. The precision performance of the kit was evaluated by calculating the negative coincidence rate of negative precision references, the coefficient of variation (CV) of the relative expression level of AKR1C3 for precision references with weak expression and high expression.

9.2.1 Detection method

In this experiment, 2 precision references were used as detection samples, 2 instruments, 2 operators and 3 verification dates were used, and each reference was detected 24 times. All negative references were negative, and the positive CV value was <15%.

$$\text{CV value} = (\text{standard deviation SD/ Mean}) \times 100\%$$

9.2.2 Experimental results

Table 21. AKR1C3 precision results

| Experimental date | Experimenter | Instrument model | Sample Name | Target Name | $C_T$ | $C_T$ CV | Target Name | $C_T$ | $C_T$ CV |
|---|---|---|---|---|---|---|---|---|---|
| 20201026 a.m. | A | 275053849 | R1 | AKR1C3 | 24.11411476 | 0.78% | ACTB | 19.088524 | 1.73% |
| 20201026 a.m. | A | 275053849 | R1 | AKR1C3 | 24.16817093 | | ACTB | 19.390919 | |
| 20201026 a.m. | A | 275053849 | R1 | AKR1C3 | 24.0476532 | | ACTB | 19.034349 | |
| 20201026 a.m. | A | 275053849 | R1 | AKR1C3 | 23.91945457 | | ACTB | 18.850056 | |
| 20201026 a.m. | B | 275051628 | R1 | AKR1C3 | 24.11411476 | | ACTB | 19.088524 | |
| 20201026 a.m. | B | 275051628 | R1 | AKR1C3 | 24.16817093 | | ACTB | 19.390919 | |
| 20201026 a.m. | B | 275051628 | R1 | AKR1C3 | 24.0476532 | | ACTB | 19.034349 | |
| 20201026 a.m. | B | 275051628 | R1 | AKR1C3 | 23.91945457 | | ACTB | 18.850056 | |
| 20201027 a.m. | A | 275053849 | R1 | AKR1C3 | 24.40969276 | | ACTB | 19.163565 | |
| 20201027 a.m. | A | 275053849 | R1 | AKR1C3 | 24.28497124 | | ACTB | 18.652203 | |
| 20201027 a.m. | A | 275053849 | R1 | AKR1C3 | 24.29683304 | | ACTB | 18.56831 | |
| 20201027 a.m. | A | 275053849 | R1 | AKR1C3 | 24.219944 | | ACTB | 18.605289 | |
| 20201023 p.m. | B | 275051628 | R1 | AKR1C3 | 23.9898243 | | ACTB | 19.041698 | |
| 20201023 p.m. | B | 275051628 | R1 | AKR1C3 | 24.13328171 | | ACTB | 19.094217 | |
| 20201023 p.m. | B | 275051628 | R1 | AKR1C3 | 23.93481636 | | ACTB | 18.961788 | |
| 20201023 p.m. | B | 275051628 | R1 | AKR1C3 | 23.92903328 | | ACTB | 19.025682 | |

(continued)

| Experimental date | Experimenter | Instrument model | Sample Name | Target Name | $C_T$ | $C_T$ CV | Target Name | $C_T$ | $C_T$ CV |
|---|---|---|---|---|---|---|---|---|---|
| 20201028 p.m. | B | 275053849 | R1 | AKR1C3 | 24.27304268 | | ACTB | 18.652338 | |
| 20201028 p.m. | B | 275053849 | R1 | AKR1C3 | 24.26483727 | | ACTB | 18.751188 | |
| 20201028 p.m. | B | 275053849 | R1 | AKR1C3 | 24.30420494 | | ACTB | 19.012062 | |
| 20201028 p.m. | B | 275053849 | R1 | AKR1C3 | 24.20526314 | | ACTB | 19.029068 | |
| 20201022 p.m. | A | 275051628 | R1 | AKR1C3 | 24.31328773 | | ACTB | 19.504038 | |
| 20201022 p.m. | A | 275051628 | R1 | AKR1C3 | 24.57431793 | | ACTB | 19.716539 | |
| 20201022 p.m. | A | 275051628 | R1 | AKR1C3 | 24.55712318 | | ACTB | 19.723076 | |
| 20201022 p.m. | A | 275051628 | R1 | AKR1C3 | 24.47789192 | | ACTB | 19.65189 | |

(continued)

| Experimental date | Experimenter | Instrument model | Sample Name | Target Name | $C_T$ | $C_T$ CV | Target Name | $C_T$ | $C_T$ CV |
|---|---|---|---|---|---|---|---|---|---|

| | | | | | | | |
|---|---|---|---|---|---|---|---|
| 20201026 a.m. | A | 275053849 | R2 | AKR1C3 | Undetermined | ACTB | 39.802425 |
| 20201026 a.m. | A | 275053849 | R2 | AKR1C3 | Undetermined | ACTB | Undetermined |
| 20201026 a.m. | A | 275053849 | R2 | AKR1C3 | Undetermined | ACTB | Undetermined |
| 20201026 a.m. | A | 275053849 | R2 | AKR1C3 | Undetermined | ACTB | 39.324535 |
| 20201026 a.m. | B | 275051628 | R2 | AKR1C3 | 39.69240952 | ACTB | Undetermined |
| 20201026 a.m. | B | 275051628 | R2 | AKR1C3 | Undetermined | ACTB | 39.547302 |
| 20201026 a.m. | B | 275051628 | R2 | AKR1C3 | Undetermined | ACTB | 39.802425 |
| 20201026 a.m. | B | 275051628 | R2 | AKR1C3 | Undetermined | ACTB | Undetermined |
| 20201027 a.m. | A | 275053849 | R2 | AKR1C3 | Undetermined | ACTB | Undetermined |
| 20201027 a.m. | A | 275053849 | R2 | AKR1C3 | Undetermined | ACTB | Undetermined |
| 20201027 a.m. | A | 275053849 | R2 | AKR1C3 | Undetermined | ACTB | Undetermined |
| 20201027 a.m. | A | 275053849 | R2 | AKR1C3 | Undetermined | ACTB | Undetermined |
| | | | | | NA | | NA |
| 20201023 p.m. | B | 275051628 | R2 | AKR1C3 | Undetermined | ACTB | Undetermined |
| 20201023 p.m. | B | 275051628 | R2 | AKR1C3 | Undetermined | ACTB | Undetermined |
| 20201023 p.m. | B | 275051628 | R2 | AKR1C3 | Undetermined | ACTB | 38.619129 |
| 20201023 p.m. | B | 275051628 | R2 | AKR1C3 | Undetermined | ACTB | Undetermined |
| 20201028 p.m. | B | 275053849 | R2 | AKR1C3 | Undetermined | ACTB | Undetermined |
| 20201028 p.m. | B | 275053849 | R2 | AKR1C3 | Undetermined | ACTB | 39.244194 |

The CV value of AKR1C3 Ct for 24 times of positive precision reference was 0.78%, and the CV value of ACTB CT was 1.73%. The 24 times of negative precision reference were all negative.

As can be seen from Table 21, the samples of precision and repeatability detection were detected by qPCR for 24 times, and the CV values were all <5%, suggesting that precision and repeatability met the verification standards.

9.3 Negative coincidence rate

9.3.1 Detection method

Negative references (AKR1C3 homologous plasmid AKR1C4, 9.06×$10^3$copies) were selected and detected by the kit for 3 times repeat to evaluate for false positives.

9.3.2 Experimental results

Table 22. qPCR detection results of negative references

| Well | Sample Name | Target Name | Reporter | $C_T$ | Target Name | Reporter | $C_T$ |
|---|---|---|---|---|---|---|---|
| A3 | W1 | AKR1C3 | FAM | Undetermined | ACTB | VIC | 39.62632 |
| B3 | W1 | AKR1C3 | FAM | Undetermined | ACTB | VIC | Undetermined |
| C3 | W1 | AKR1C3 | FAM | Undetermined | ACTB | VIC | Undetermined |

As can be seen from Table 22, the repeated detection results were all negative, and the negative coincidence rate was 100%, which met the verification standards.

9.4 Negative coincidence rate

9.4.1 Detection method

Two positive references (AKR1C3 cell lines verified by Western blot) were selected and detected by kit to evaluate the accuracy of the detection. AKR1C3 and ACTB plasmids (quantified by digital PCR) were used for gradient dilution to make standard curves, which were detected by kit to evaluate amplification efficiency and linear relationship.

9.4.2 Experimental results

Table 23. qPCR detection results of positive references

| Sample Name | Target Name | $C_T$ | $C_T$ Mean | $C_T$ SD | $C_T$ CV | Quantity | Target Name | $C_T$ | $C_T$ Mean | $C_T$ SD | $C_T$ CV | Quantity | FAM copy number / VIC copy number |
|---|---|---|---|---|---|---|---|---|---|---|---|---|---|
| P1 | AKR1C3 | 24.18057 | | | | 11074.48 | ACTB | 18.75349 | | | | 1423347 | 0.00778 |
| P1 | AKR1C3 | 24.15725 | 24.17021 | 0.011876 | 0.05% | 11254.97 | ACTB | 18.52806 | 18.68381 | 0.135132 | 0.72% | 1657402 | 0.00679 |
| P1 | AKR1C3 | 24.17281 | | | | 11134.24 | ACTB | 18.76988 | | | | 1407672 | 0.00791 |
| P3 | AKR1C3 | 29.13986 | | | | 355.8989 | ACTB | 18.15344 | | | | 2134538 | 0.00017 |
| P3 | AKR1C3 | 29.08711 | 29.04446 | 0.122427 | 0.42% | 369.154 | ACTB | 18.2114 | 18.18871 | 0.030965 | 0.17% | 2052591 | 0.00018 |
| P3 | AKR1C3 | 28.90641 | | | | 418.4136 | ACTB | 18.2013 | | | | 2066642 | 0.00020 |
| P2 | AKR1C3 | 22.36224 | 22.20885 | 0.21693 | | 44200 | ACTB | 15.31071 | 15.21909 | 0.129568 | | 15780000 | |
| P2 | AKR1C3 | 22.05546 | 22.20885 | 0.21693 | | 44200 | ACTB | 15.12747 | 13.21909 | 0.129568 | | 13780000 | |
| P2 | AKR1C3 | 23.30387 | 25.48958 | 0.023043 | | 4420 | ACTB | 18.49942 | 18.59612 | 0.136749 | | 1578000 | |
| P2 | AKR1C3 | 23.47328 | 25.48958 | 0.023043 | | 4420 | ACTB | 18.69281 | 18.59612 | 0.136749 | | 1578000 | |
| P2 | AKR1C3 | 28.66143 | 28.78446 | 0.173985 | | 442 | ACTB | 21.82707 | 21.93377 | 0.150898 | | 157800 | |
| P2 | AKR1C3 | 28.90748 | 28.78446 | 0.173985 | | 442 | ACTB | 22.04047 | 21.93377 | 0.150898 | | 157800 | |
| P2 | AKR1C3 | 32.32851 | 32.18291 | 0.205901 | | 44.2 | ACTB | 25.45004 | 23.47176 | 0.030727 | | 15780 | |
| P2 | AKR1C3 | 32.03732 | 32.18291 | 0.205901 | | 44.2 | ACTB | 25.49349 | 23.47176 | 0.030727 | | 15780 | |

As can be seen from Table 23, the selected positive references (cell lines verified by Western blot) were detected using the above detection method. P1 was a strong positive standard and the ratio of FAM copy number/VIC copy number was high, P3 was a weak positive standard and the ratio of FAM copy number/VIC copy number was low, and CV met the requirements. The detection accuracy of the method met the requirements.

9.5 Specificity

9.5.1 Detection method

2 concentrations of negative references (AKR1C3 homologous plasmid AKR1C2 with copy numbers of $1.14\times10^4$ and $1.14\times10^3$, respectively) were selected. Each concentration level was repeated for 3 times to evaluate the specificity.

9.5.2 Detection results

Table 24. qPCR detection results of specific references

| Dilution | Target Name | Reporter | $C_T$ | Target Name | Reporter | $C_T$ |
|---|---|---|---|---|---|---|
| $10^{-6}$ | AKR1C3 | FAM | Undetermined | ACTB | VIC | Undetermined |
| $10^{-6}$ | AKR1C3 | FAM | Undetermined | ACTB | VIC | Undetermined |
| $10^{-6}$ | AKR1C3 | FAM | Undetermined | ACTB | VIC | Undetermined |
| $10^{-7}$ | AKR1C3 | FAM | Undetermined | ACTB | VIC | Undetermined |
| $10^{-7}$ | AKR1C3 | FAM | Undetermined | ACTB | VIC | Undetermined |
| $10^{-7}$ | AKR1C3 | FAM | Undetermined | ACTB | VIC | Undetermined |

2 gradients of the negative references were repeated for 3 times, and the results were all negative. The specificity of kit test met the requirements.

9.6 Actual sample detection

9.6.1 Detection method

8 normal blood samples were selected for detection, and the loading amount was 40ng cDNA of RNA reverse transcription. Each sample was detected twice, and the accuracy of FAM copy number /VIC copy number ratio was calculated.

9.6.2 Detection results

The detection results were shown in the following table:

Table 25. qPCR detection results of normal blood samples

| Sample Name | Target Name | $C_T$ | $C_T$ Mean | $C_T$ SD | $C_T$ CV | Quantity | Target Name | $C_T$ | $C_T$ Mean | $C_T$ SD | $C_T$ CV | Quantity | FAM copy number / VIC copy number |
|---|---|---|---|---|---|---|---|---|---|---|---|---|---|
| 1 | AKR1C3 | 29.19695 | 29.18378 | 0.018623 | 0.06% | 342.0897 | ACTB | 17.85256 | 17.7661 | 0.122284 | 0.69% | 2615471 | 0.00013 |
| 1 | AKR1C3 | 29.17061 | | | | 348.3924 | ACTB | 17.67963 | | | | 2939487 | 0.00012 |
| 3 | AKR1C3 | 27.59087 | 27.47804 | 0.159567 | 0.58% | 1041.478 | ACTB | 17.17691 | 17.13873 | 0.054002 | 0.32% | 4127791 | 0.00025 |
| 3 | AKR1C3 | 27.36521 | | | | 1217.828 | ACTB | 17.10054 | | | | 4346273 | 0.00028 |
| 6 | AKR1C3 | 28.04806 | 28.00367 | 0.062777 | 0.22% | 738.603 | ACTB | 18.27402 | 18.05006 | 0.316733 | 1.75% | 1967602 | 0.00039 |
| 6 | AKR1C3 | 27.95928 | | | | 806.755 | ACTB | 17.82609 | | | | 2662646 | 0.00030 |
| 7 | AKR1C3 | 27.65212 | 27.55965 | 0.130763 | 0.47% | 998.1864 | ACTB | 16.47845 | 16.47723 | 0.001732 | 0.01% | 6615674 | 0.00015 |
| 7 | AKR1C3 | 27.46719 | | | | 1134.708 | ACTB | 16.476 | | | | 6626625 | 0.00017 |
| 8 | AKR1C3 | 26.84116 | 26.84425 | 0.004378 | 0.02% | 1751.268 | ACTB | 17.08492 | 17.04245 | 0.060062 | 0.35% | 4392362 | 0.00040 |
| 8 | AKR1C3 | 26.84735 | | | | 1743.768 | ACTB | 16.99998 | | | | 4651691 | 0.00037 |
| 10 | AKR1C3 | 25.80928 | 25.76475 | 0.062972 | 0.24% | 3580.959 | ACTB | 17.2306 | 17.18045 | 0.070927 | 0.41% | 3980807 | 0.00090 |
| 10 | AKR1C3 | 23.72022 | | | | 3808.989 | ACTB | 17.13029 | | | | 4259813 | 0.00089 |
| 11 | AKR1C3 | 27.16312 | 27.22639 | 0.089475 | 0.33% | 1400.954 | ACTB | 16.77766 | 16.76808 | 0.013548 | 0.08% | 5405273 | 0.00026 |
| 11 | AKR1C3 | 27.28966 | | | | 1283.304 | ACTB | 16.7585 | | | | 5475667 | 0.00023 |
| 12 | AKR1C3 | 27.55924 | 26.98829 | 0.807451 | 2.99% | 1064.568 | ACTB | 16.55035 | 16.75941 | 0.295644 | 1.76% | 6302104 | 0.00017 |
| 12 | AKR1C3 | 26.41733 | | | | 2349.339 | ACTB | 16.96846 | | | | 4751781 | 0.00049 |

8 normal blood samples were detected twice, the ratio of FAM copy number/VIC copy number was <0.001, and the detection CV was <5%. The accuracy of the kit met the requirements.

9.7 Template amount verification

200ng and 10ng RNA were used as the initial amount of reverse transcription, and the range of loading amount of bone marrow samples from 3 clinical leukemia patients was verified.

Table 26. qPCR detection results of negative and positive samples with different initiation amounts of reverse transcription

| Sample Name | Target Name | $C_T$ | Quantity | FAM copy number/VIC copy number |
|---|---|---|---|---|
| u8099-10ng | AKR1C3 | 27.94 | 1249 | 0.00300 |
| u8099-10ng | ACTB | 21.16 | 415745 | |
| u8099-10ng | AKR1C3 | 28.22 | 1032 | 0.00254 |
| u8099-10ng | ACTB | 21.19 | 405838 | |
| u8099-200ng | AKR1C3 | 2330 | 29525 | 0.00293 |
| u8099-200ng | ACTB | 16.48 | 10070348 | |
| u8099-200ng | AKR1C3 | 23.23 | 31032 | 0.00299 |
| u8099-200ng | ACTB | 16.43 | 10370063 | |
| x9375-10ng | AKR1C3 | 24.86 | 10204 | 0.01243 |
| x9375-10ng | ACTB | 20.16 | 820935 | |
| x9375-10ng | AKR1C3 | 24.87 | 10142 | 0.01478 |
| x9375-10ng | ACTB | 20.42 | 686215 | |
| x9375-200ng | AKR1C3 | 20.40 | 213177 | 0.00986 |
| x9375-200ng | ACTB | 15.36 | 21623626 | |
| x9375-200ng | AKR1C3 | 20.37 | 217756 | 0.00916 |
| x9375-200ng | ACTB | 15.22 | 23766040 | |
| c5233-10ng | AKR1C3 | 27.50 | 1689 | 0.00262 |
| c5233-10ng | ACTB | 20.51 | 645796 | |
| c5233-10ng | AKR1C3 | 27.48 | 1710 | 0.00304 |
| c5233-10ng | ACTB | 20.71 | 562264 | |
| c5233-200ng | AKR1C3 | 23.36 | 28377 | 0.00214 |
| c5233-200ng | ACTB | 16.07 | 13286656 | |
| c5233-200ng | AKR1C3 | 23.16 | 32533 | 0.00256 |
| c5233-200ng | ACTB | 16.13 | 12732719 | |

Conclusions: the detection results of 10ng and 200ng loading samples were consistent.

9.8 Verification Conclusions

Table 27. The summary table for verification conclusions

| Verification type | Verification item | Sample | Times of repeat | Standard | Detection result |
|---|---|---|---|---|---|
| Accuracy verification | Negative coincidence rate | W1 | 3 | All 3 results were negative | All 3 results were negative |
| | Positive reference | P1 | 3 | All 3 results were positive | All 3 results were positive |
| | | P2 | 3 | The amplification efficiency of standard curve≥90%, $R^2$>0.99 | The amplification efficiency of standard curve≥90%, $R^2$>0.99 |
| | | P3 | 3 | All 3 results were positive | All 3 results were positive |
| The lowest detection limit | The lowest detection limit | S1 | 10 | Detection rate was 100% | All 10 results were positive, and detection rate was 100% |
| Analysis specificity | Specificity | Negative reference ($10^{-6}$ dilution) | 3 | All 3 results were negative | All 3 results were negative |
| | | Negative reference ($10^{-7}$ dilution) | 3 | All 3 results were negative | All 3 results were negative |
| Precision | Precision | R1 | 24 | 2 instruments, 2 operators and 3 verification dates were used, and each reference was tested 24 times. All negative references were negative, and the CV value for positive references was<15% | The CV value of AKR1C3 Ct for 24 times of R1 was 0.78%, and the CV value of ACTB CT was 1.73%. The 24 times of R2 were all negative |
| | | R2 | 24 | | |
| Sample detection | Actual sample detection | 8 normal blood samples | 2 | CV<15% | CV<15%, FAM copy number/VIC copy number was at a low level |

Conclusions: through the performance verification of the lowest detection limit, precision, negative coincidence rate, positive coincidence rate, template amount, analytical specificity and actual sample detection, the seven performances all met the verification standards. The performance of this detection method met the requirements of clinical application, and can be used for clinical sample detection.

Example 10. Kit for detecting AKR1C3 RNA content (qPCR Method)

In this example, the kit for detecting AKR1C3 RNA content comprises:

1) qPCR polymerase mixture, wherein the qPCR polymerase mixture mainly comprises: DNA polymerase, $MgCl_2$, buffer and dNTPs (plus UNG enzyme and dUTP). Preferably, the polymerase mixture is KAPA PROBE FAST RT-PCR Master Mix(2x);

2) Reverse transcriptase mixture, preferably, the reverse transcriptase mixture is Superscript VII,O MARSTER MIX;

3) AKR1C3 reaction mixture, wherein the AKR1C3 reaction mixture comprises primer-probe composition for detecting AKR1C3 gene and primer-probe composition for detecting reference gene; wherein the primer-probe composition for detecting AKR1C3 gene was selected from any one of the groups (i) to (ix) as described above; more preferably, it was selected from one group of group (iii), (iv) and (v); even more preferably, it was selected from group (iv). The 5'-end reporter was FAM and the 3'-end quencher was MGB in the probe for detecting AKR1C3

gene. The primer-probe composition for detecting reference gene comprises upstream primer ACTB-F1, downstream primer ACTB-R1 and probe ACTB-P1, the nucleotide sequences of the upstream primer ACTB-F1, the downstream primer ACTB-R1 and the probe ACTB-P1 are shown as SEQ ID NO: 17, SEQ ID NO: 18 and SEQ ID NO: 19, respectively. The 5'-end reporter of the probe of reference gene was VIC, and the 3'-end quencher was BHQ1;

4) Negative control (NC), for example, nuclease-free water;

5) Positive control, for example, a reference with a known copy number;

6) Instructions recording relevant operation methods.

Example 11. Kit for detecting AKR1C3 RNA content (Digital PCR Method)

As described in example 8, separate detections were needed for AKR1C3 and ACTB in digital PCR method. Therefore, the kit in this example differs from the kit in example 10 in that the AKR1C3 reaction mixture only comprises primer-probe composition for detecting AKR1C3 gene, and does not comprise primer-probe composition for detecting reference gene ACTB. The kit in this example comprises both AKR1C3 reaction mixture (comprising a primer-probe composition for detecting AKR1C3 gene) and reference gene ACTB reaction mixture (comprising a primer-probe composition for detecting reference gene ACTB), the remaining components being the same.

As described in example 10 or 11, the kit for detecting AKR1C3 RNA content was used together with AKR1C3 activated anticancer drugs, usually screening patients. The use of the kit makes it easier for medical personnel to detect in different laboratories using a uniform detection kit standard operation procedure (SOP) before deciding to administer drugs to patients. In this way, the AKR1C3 detection results obtained by the same reagent and same procedure can match the recommended detection results for a specific cancer in the drug instructions of the AKR1C3 activated cancer drug.

The specific operation method of the kit is recorded in the instructions, i.e., the specific operating conditions in the above instructions. Optionally or as a preferred embodiment, the instructions also provide a predetermined amount X value for the AKR1C3 activated anticancer drug for different cancer (tumor) types. For example, as for B lineage acute lymphoblastic leukemia (B-ALL) and T lineage acute lymphoblastic leukemia (T-ALL), the predetermined X value was 0.00014 to 0.015, and the ratio of AKR1C3 copy number/ACTB copy number of an *ex vivo* peripheral blood sample from a patient detected by the above kit was 0.0092. According to statistics, the ratio of AKR1C3 copy number/ACTB copy number of B-ALL patients using AKR1C3 activated anticancer drugs should not be less than 0.00014. Therefore, doctors can prescribe AKR1C3 activated anticancer drugs for this patient. Another example, as for T lineage acute lymphoblastic leukemia (T-ALL), the ratio of AKR1C3 copy number/ACTB copy number of an *ex vivo* peripheral blood sample from a patient detected by the above kit was 0.00012. According to statistics, the ratio of AKR1C3 copy number/ACTB copy number of T-ALL patients using AKR1C3 activated anticancer drugs should not be less than 0.00014. Therefore, doctors should not prescribe AKR1C3 activated anticancer drugs for this patient.

Example 12. Detection method for AKR1C3 RNA content and the use of the kit for detecting AKR1C3 RNA content in preparation of a drug for treating cancers

The ratio of AKR1C3 copy number/ACTB copy number of an *ex vivo* peripheral blood sample from a B-ALL patient detected by the detection method for AKR1C3 RNA content established in examples 5-9 or the kit for detecting AKR1C3 RNA content established in example 10 or 11 was 0.0092, greater than the predetermined content of 0.00014.

The B-ALL patient was administered with AKR1C3 activated anticancer drug.

Through the verification of existing experiments, AKR1C3 activated anticancer drugs selected from the following structure have better therapeutic effect.

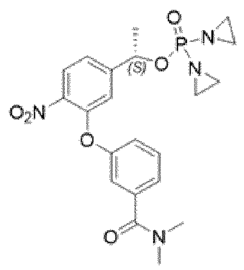
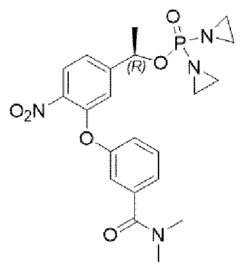
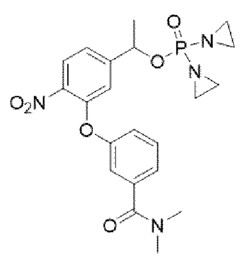
.

## Claims

1. A primer-probe composition, selected from any one of the following groups:

   (i) upstream primer AKR1C3-F1, downstream primer AKR1C3-R1 and probe AKR1C3-P1, wherein the nucleotide sequences of the upstream primer AKR1C3-F1, the downstream primer AKR1C3-R1 and the probe AKR1C3-P1 are shown as SEQ ID NO:1, SEQ ID NO:2 and SEQ ID NO:3, respectively;
   (ii) upstream primer AKR1C3-F2, downstream primer AKR1C3-R2 and probe AKR1C3-P1, wherein the nucleotide sequences of the upstream primer AKR1C3-F2, the downstream primer AKR1C3-R2 and the probe AKR1C3-P1 are shown as SEQ ID NO:4, SEQ ID NO:5 and SEQ ID NO:3, respectively;
   (iii) upstream primer AKR1C3-F2, downstream primer AKR1C3-R6 and probe AKR1C3-P1, wherein the nucleotide sequences of the upstream primer AKR1C3-F2, the downstream primer AKR1C3-R6 and the probe AKR1C3-P1 are shown as SEQ ID NO:4, SEQ ID NO:6 and SEQ ID NO:3, respectively;
   (iv) upstream primer AKR1C3-F6, downstream primer AKR1C3-R2 and probe AKR1C3-P1, wherein the nucleotide sequences of the upstream primer AKR1C3-F6, the downstream primer AKR1C3-R2 and the probe AKR1C3-P1 are shown as SEQ ID NO:7, SEQ ID NO:5 and SEQ ID NO:3, respectively;
   (v) upstream primer AKR1C3-F6, downstream primer AKR1C3-R6 and probe AKR1C3-P1, wherein the nucleotide sequences of the upstream primer AKR1C3-F6, the downstream primer AKR1C3-R6 and the probe AKR1C3-P1 are shown as SEQ ID NO:7, SEQ ID NO:6 and SEQ ID NO:3, respectively;
   (vi) upstream primer AKR1C3-F5, downstream primer AKRIC3-R5 and probe AKR1C3-P2, wherein the nucleotide sequences of the upstream primer AKR1C3-F5, the downstream primer AKR1C3-R5 and the probe AKR1C3-P2 are shown as SEQ ID NO:8, SEQ ID NO:9 and SEQ ID NO: 10, respectively;
   (vii) upstream primer AKR1C3-F3, downstream primer AKR1C3-R3 and probe AKR1C3-P1, wherein the nucleotide sequences of the upstream primer AKR1C3-F3, the downstream primer AKR1C3-R3 and the probe AKR1C3-P1 are shown as SEQ ID NO: 11, SEQ ID NO: 12 and SEQ ID NO:3, respectively;
   (viii) upstream primer AKR1C3-F4, downstream primer AKR1C3-R3 and probe AKR1C3-P2, wherein the nucleotide sequences of the upstream primer AKR1C3-F4, the downstream primer AKR1C3-R3 and the probe AKR1C3-P2 are shown as SEQ ID NO: 13, SEQ ID NO: 12 and SEQ ID NO: 10, respectively;
   (ix) upstream primer AKR1C3-F7, downstream primer AKR1C3-R7 and probe AKR1C3-P3, wherein the nucleotide sequences of the upstream primer AKR1C3-F7, the downstream primer AKR1C3-R7 and the probe AKR1C3-P3 are shown as SEQ ID NO: 14, SEQ ID NO: 15 and SEQ ID NO: 16, respectively.

2. The primer-probe composition according to claim 1, wherein the primer-probe composition is selected from any one of groups (iii), (iv) and (v);
   preferably, the primer-probe composition is selected from group (iv).

3. The primer-probe composition according to claim 1 or 2, wherein the 5'-end reporters of probes AKR1C3-P1, AKR1C3-P2 and AKR1C3-P3 are FAM, and the 3'-end quenchers of probes AKR1C3-P1, AKR1C3-P2 and AKR1C3-P3 are MGB.

4. A kit comprising the primer-probe composition according to any one of claims 1 to 3.

5. The kit according to claim 4, wherein the kit further comprises a primer-probe composition of a reference gene;
   preferably, the reference gene is ACTB.

6. The kit according to claim 4 or 5, wherein the primer-probe composition of reference gene comprises upstream primer ACTB-F1, downstream primer ACTB-R1 and probe ACTB-P1, and the nucleotide sequences of the upstream primer ACTB-F1, the downstream primer ACTB-R1 and the probe ACTB-P1 are shown as SEQ ID NO: 17, SEQ ID NO: 18 and SEQ ID NO: 19, respectively.

7. The kit according to any one of claims 4-6, wherein the 5'-end reporter of probe ACTB-P1 is VIC, and the 3'-end quencher of probe ACTB-P1 is BHQ1.

8. The kit according to any one of claims 4-7, wherein the kit further comprises a polymerase mixture, and the polymerase mixture mainly comprises: DNA polymerase, $MgCl_2$, buffer and dNTPs;
   preferably, the polymerase mixture is KAPA PROBE FAST RT-PCR Master Mix(2x).

9. The kit according to any one of claims 4-8, wherein the kit further comprises a reverse transcriptase mixture;

preferably, the reverse transcriptase mixture is Superscript VII,O MARSTER MIX.

**10.** The kit according to any one of claims 4-9, wherein the kit further comprises a negative control and a positive control;

preferably, the negative control is nuclease-free water; and/or
preferably, the positive control is a reference with a known copy number.

**11.** Use of the primer-probe composition according to any one of claims 1-3 or the kit according to any one of claims 4-10 in the preparation of a drug for treating cancers.

**12.** The use according to claim 11, wherein the AKR1C3 RNA content in an *ex vivo* sample of a patient is obtained by using the primer-probe composition according to any one of claims 1-3 or the kit according to any one of claims 4-10; the patient with AKR1C3 RNA content greater than or equal to the predetermined content is administrated with AKR1C3 activated anticancer drugs.

**13.** The use according to claim 11 or 12, wherein the AKR1C3 RNA content is obtained according to the ratio of AKR1C3 copy number/reference gene copy number;

preferably, the predetermined content is 0.0001~1;
more preferably, the predetermined content is 0.00011-0.5;
even more preferably, the predetermined content is 0.00013~0.05.

**14.** The use according to any one of claims 11-13, wherein the *ex vivo* sample comprises blood sample, bone marrow sample, or tissue sample.

**15.** The use according to any one of claims 11-14, wherein the AKR1C3 activated anticancer drug is a compound selected from the compounds with the following structures:

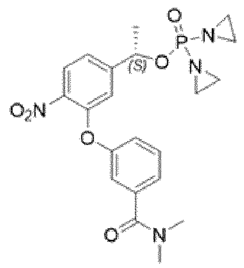 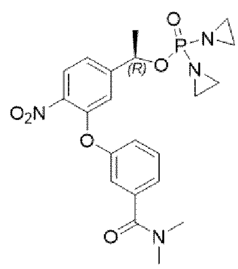 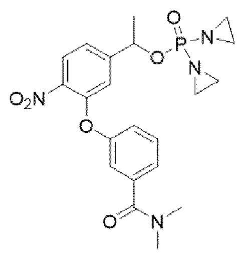 .

**16.** The use according to any one of claims 11-15, wherein the cancers comprise:

lung cancer, non-small cell lung cancer, liver cancer, pancreatic cancer, breast cancer, gastric cancer, bone cancer, esophageal cancer, mastocarcinoma, prostate cancer, testicular cancer, colon cancer, ovarian cancer, bladder cancer, cervical cancer, hepatocellular carcinoma, melanoma, squamous cell carcinoma, basal cell carcinoma, adenocarcinoma, sweat gland carcinoma, sebaceous gland carcinoma, papillary carcinoma, papillary adenocarcinoma, renal cell carcinoma, cystic adenocarcinoma, cystic carcinoma, medullary carcinoma, bronchial carcinoma, osteocyte carcinoma, epithelial carcinoma, carcinoma of bile duct, choriocarcinoma, embryonal carcinoma, seminoma, Wilm's tumor, glioblastoma, astrocytoma, medulloblastoma, craniopharyngioma, ependymoma, pineal tumor, hemocytoblastoma, vocal cords neuroma, meningioma, neuroblastoma, optic neuroblastoma, retinoblastoma, neurofibroma, fibrosarcoma, fibroblastoma, fibroma, fibroadenoma, fibrochondroma, fibrocystoma, fibromyxoma, fibroosteoma, fibromyxosarcoma, fibropapilloma, myxosarcoma, myxocystoma, myxochondroma, myxochondrosarcoma, myxochondrofibrosarcoma, myxadenoma, myxoblastoma, liposarcoma, lipoma, lipoadenoma, lipoblastoma, lipochondroma, lipofibroma, lipoangioma, myxolipoma, chondrosarcoma, chondroma, chondromyoma, chordoma, choriocarcinoma, chorioepithelioma, chorioblastoma, osteosarcoma, osteoblastoma, osteochondrofibroma, osteochondrosarcoma, osteochondroma, osteocystoma, osteodentinoma, osteofibroma, fibrosarcoma of bone, angiosarcoma, hemangioma, angiolipoma, angiochondroma, hemangioblastoma, angiokeratoma, angioglioma, angioendothelioma, angiofibroma, angiomyoma, angiolipoma, angiolymphangioma, angiolipoleiomyoma, angiomyolipoma, angiomyoneuroma, angiomyxoma, angioreticuloma, lymphangiosarcoma, lymphogranuloma, lymphangioma, lymphoma, lymphomyxoma, lymphosarcoma, lymphangiofibroma, lymphocytoma, lymphoepithelioma, lymphoblastoma, peripheral T-cell lymphoma, nodular NK/T-cell lymphoma, endothelioma, endoblastoma, synovioma, synovial sarcoma, mesothelioma, connective tissue tumor, Ewing's tumor, leiomyoma, leiomyosarcoma, leiomyoblastoma, leiomyofibroma, rhabdomyoma, rhabdomyosarcoma, rhabdomyomyxoma, acute lymphatic leukemia, acute myelogenous leukemia, chronic disease cells, polycythemia, lymphoma, endometrial cancer, glioma, colorectal cancer, thyroid cancer, urothelial cancer or multiple myeloma;

preferably, the cancer comprises: ovarian cancer, cervical cancer, pancreatic cancer, breast cancer, colorectal cancer, esophageal cancer, gastric cancer, hepatocellular carcinoma, non-small cell lung cancer, prostate cancer, renal cell carcinoma, peripheral T-cell lymphoma, nodular NK/T-cell lymphoma, acute lymphatic leukemia or acute myelogenous leukemia.

**17.** A method for detecting AKR1C3 RNA content, comprising the following steps:

(1) extracting RNA of an ex vivo sample to be detected and adding the extracted RNA to the reverse transcription system and the extracted RNA being reverse transcribed to synthesize cDNA;
(2) performing qPCR or digital PCR amplification with cDNA as template using the primer-probe composition any one of claims 1-3 or the kit according to any one of claims 4-10;
(3) obtaining the AKR1C3 RNA content of *ex vi*vo sample to be detected according to qPCR or digital PCR amplification results.

**18.** The method according to claim 17, wherein in step (1), the concentration of the extracted RNA is detected;

preferably, Qubit RNA HS Assay Kits are used to detect the concentration of the extracted RNA; and/or
the reverse transcription system comprises reverse transcriptase;
preferably, the mass-to-volume ratio of the extracted RNA and reverse transcriptase is (0.5~2):4, with the unit of μg/μL;
more preferably, the mass-to-volume ratio of the extracted RNA and reverse transcriptase is (1~1.8):4, with the unit of μg/μL;
even more preferably, the mass-to-volume ratio of the extracted RNA and reverse transcriptase is 2:4, with the unit of μg/μL.

**19.** The method according to claim 17 or 18, wherein in step (2), in qPCR reaction system, the primer-probe composition selected from any one of groups (i) to (ix) is mixed with the primer-probe composition of reference gene;

preferably, in qPCR reaction system, the molar ratio of AKR1C3 upstream primer, AKR1C3 downstream primer and AKR1C3 probe is (2~10):(2~10):3;
more preferably, in qPCR reaction system, the molar ratio of AKR1C3 upstream primer, AKR1C3 downstream primer and AKR1C3 probe is (3~7):(3~7):3;
even more preferably, in qPCR reaction system, the molar ratio of AKR1C3 upstream primer, AKR1C3 downstream primer and AKR1C3 probe is 5:5:3; and/or, in qPCR reaction system,
preferably, the molar ratio of upstream primer ACTB-F1, downstream primer ACTB-R1 and probe ACTB-P1 is (2~10):(2~10):3;
more preferably, the molar ratio of upstream primer ACTB-F1, downstream primer ACTB-R1 and probe ACTB-P1 is (3~7):(3~7):3;
even more preferably, the molar ratio of upstream primer ACTB-F1, downstream primer ACTB-R1 and probe ACTB-P1 is 5:5:3; and/or, in qPCR reaction system,
preferably, the amount of AKR1C3 upstream primer, AKR1C3 downstream primer and AKR1C3 probe is the same as the amount of upstream primer ACTB-F1, downstream primer ACTB-R1 and probe ACTB-P1, respectively.

**20.** The method according to any one of claims 17-19, wherein in step (2), in qPCR reaction system, dUTP, UNG enzyme, cDNA template and polymerase mixture are also added;

preferably, the volume of the polymerase mixture is (0.3~0.8) of the total volume of qPCR reaction system;
more preferably, the volume of the polymerase mixture is 0.5 of the total volume of qPCR reaction system.

**21.** The method according to any one of claims 17-20, wherein in step (2), AKR1C3 and reference gene are amplified by AKR1C3 digital PCR detection system and reference gene digital PCR detection system, respectively;

preferably, in AKR1C3 digital PCR detection system, the molar ratio of AKR1C3 upstream primer, AKR1C3 downstream primer and AKR1C3 probe is (5~15):(5~15):3;
more preferably, the molar ratio of AKR1C3 upstream primer, AKR1C3 downstream primer and AKR1C3 probe is (8~14):(8~14):3;
even more preferably, the molar ratio of AKR1C3 upstream primer, AKR1C3 downstream primer and AKR1C3 probe is 12:12:3; and/or, in reference gene digital PCR detection system,
preferably, the molar ratio of upstream primer ACTB-F1, downstream primer ACTB-R1 and probe ACTB-P1 is (5~15):(5~15):3;
more preferably, the molar ratio of upstream primer ACTB-F1, downstream primer ACTB-R1 and probe ACTB-P1 is (8~14):(8~14):3;
even more preferably, the molar ratio of upstream primer ACTB-F1, downstream primer ACTB-R1 and probe ACTB-P1 is 12:12:3.

**22.** The method according to any one of claims 17-21, wherein in step (3), the step for obtaining the AKR1C3 RNA content of *ex vivo* sample to be detected according to qPCR or digital PCR amplification results includes:

(A) obtaining the copy numbers of AKR1C3 and reference gene of *ex vivo* sample to be detected according to qPCR or digital PCR amplification results, respectively;
(B) calculating the ratio of AKR1C3 copy number/reference gene copy number to obtain the AKR1C3 RNA content of *ex vi*vo sample to be detected.

**23.** The method according to any one of claims 17-22, wherein when the qPCR method is used, step (A) further includes:

($A_1$) plotting the standard curve of Ct value and initial copy number lg value of AKR1C3 and the standard curve of Ct value and initial copy number lg value of reference gene, respectively;
($A_2$) according to the standard curves, obtaining the copy numbers of AKR1C3 and reference gene of *ex vi*vo sample to be detected by the detected Ct values of AKR1C3 and reference gene, respectively.

**24.** The method according to any one of claims 17-23, wherein the method is used to detect AKR1C3 RNA content in blood sample, bone marrow sample or tissue sample.

**25.** A method for detecting the expression level of AKR1C3 enzyme, wherein the method according to any one of claims 17-24 is used to detect the AKR1C3 RNA content of *ex vivo* sample to be detected, and then the expression level of AKR1C3 enzyme of *ex vivo* sample to be detected is obtained according to the AKR1C3 RNA content of *ex vivo* sample to be detected.

**26.** The method according to claim 25, wherein there is a linear correlation between AKR1C3 RNA content *ex vivo* sample to be detected and the expression level of AKR1C3 enzyme *ex vivo* sample to be detected.

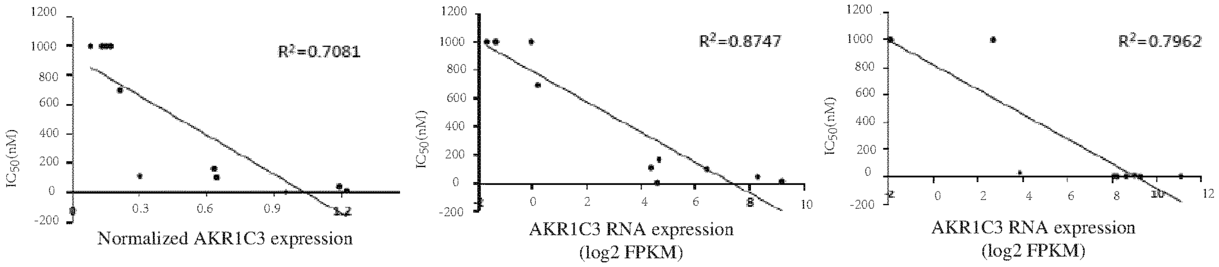
R²=0.7081
IC50(nM)
Normalized AKR1C3 expression
R²=0.8747
AKR1C3 RNA expression
(log2 FPKM)
R²=0.7962
AKR1C3 RNA expression
(log2 FPKM)

Fig.1

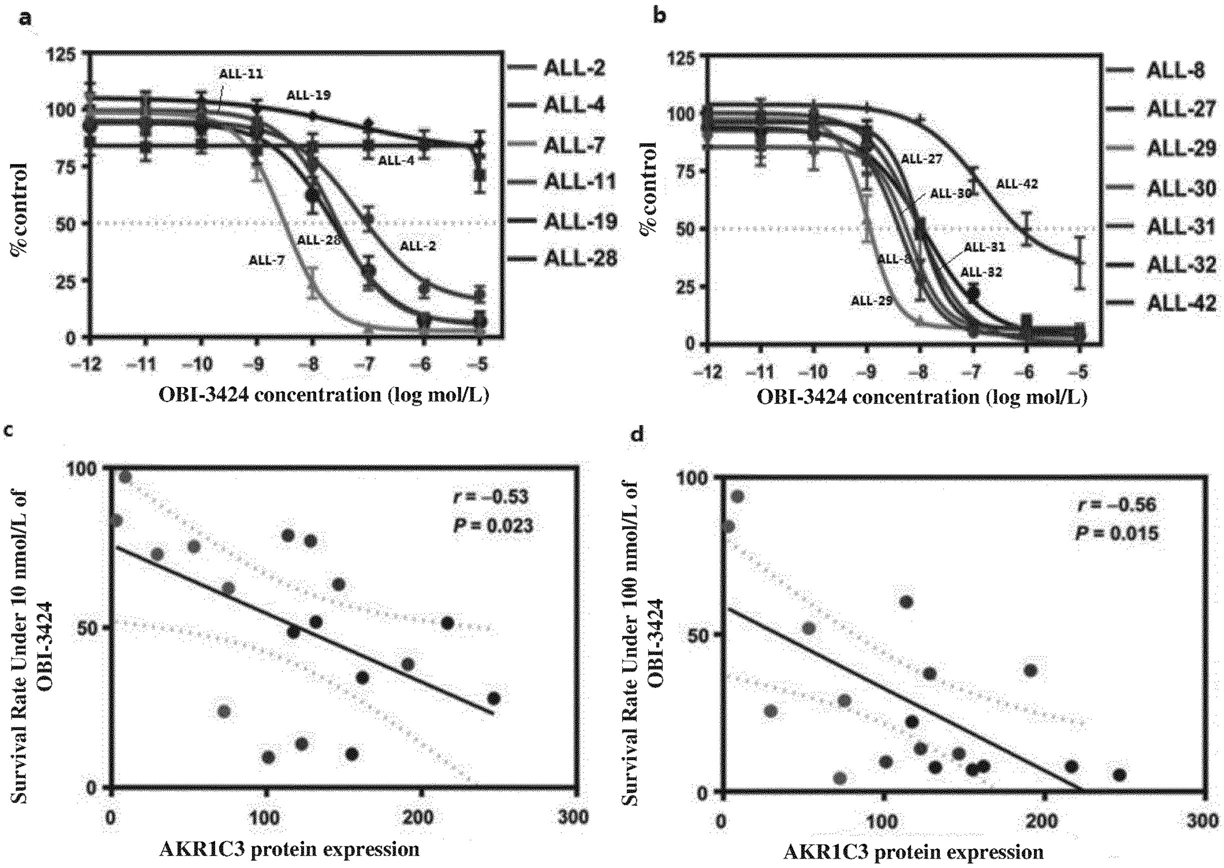
a
b
%control
OBI-3424 concentration (log mol/L)
ALL-2
ALL-4
ALL-7
ALL-11
ALL-19
ALL-28
ALL-8
ALL-27
ALL-29
ALL-30
ALL-31
ALL-32
ALL-42
c
d
Survival Rate Under 10 nmol/L of OBI-3424
Survival Rate Under 100 nmol/L of OBI-3424
AKR1C3 protein expression
r = -0.53
P = 0.023
r = -0.56
P = 0.015

Fig.2

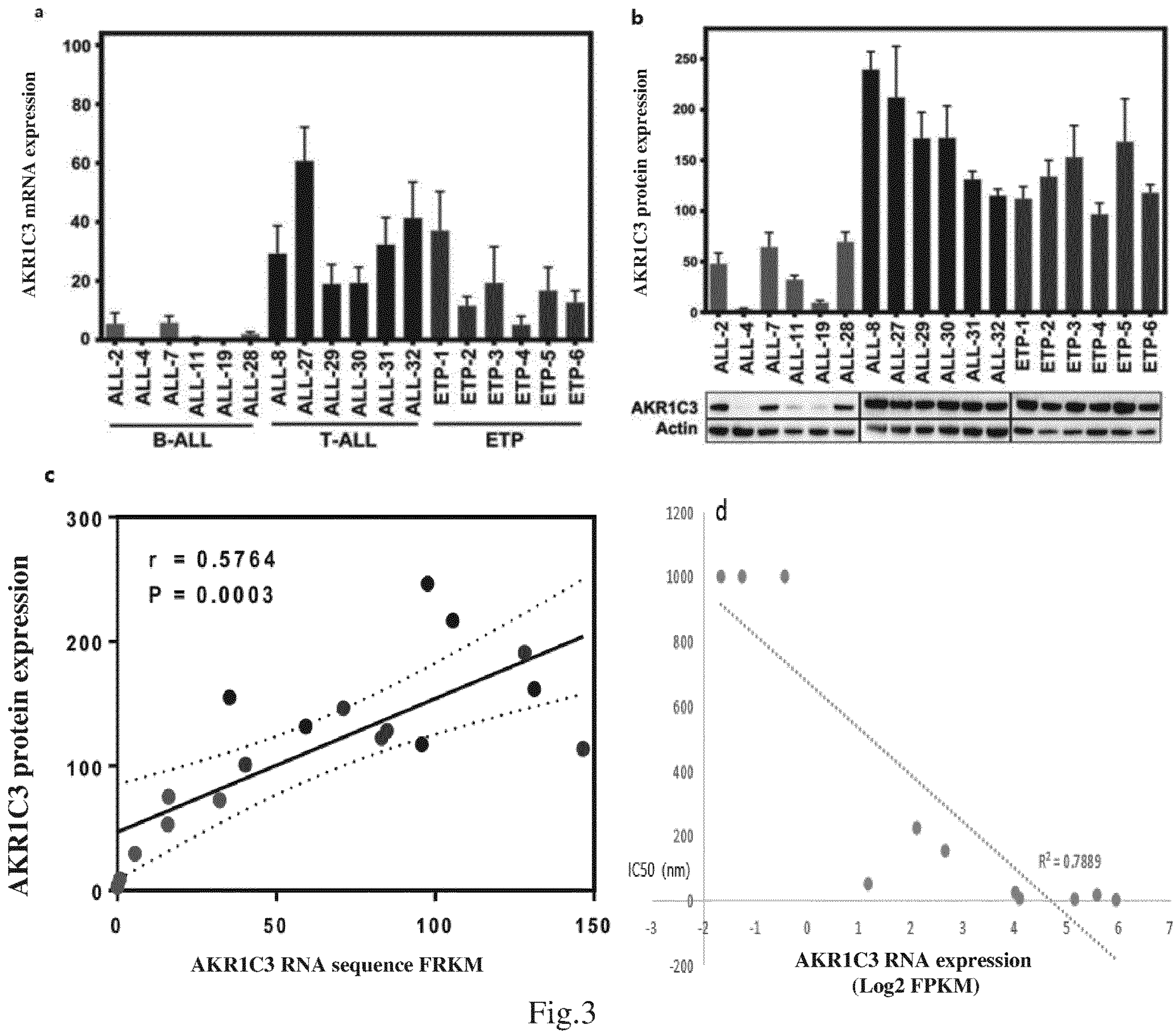
a
AKR1C3 mRNA expression
100
80
60
40
20
0
ALL-2
ALL-4
ALL-7
ALL-11
ALL-19
ALL-28
ALL-8
ALL-27
ALL-29
ALL-30
ALL-31
ALL-32
ETP-1
ETP-2
ETP-3
ETP-4
ETP-5
ETP-6
B-ALL
T-ALL
ETP
b
AKR1C3 protein expression
250
200
150
100
50
0
AKR1C3
Actin
c
AKR1C3 protein expression
300
200
100
0
r = 0.5764
P = 0.0003
50
150
AKR1C3 RNA sequence FRKM
d
1200
1000
800
600
400
200
IC50 (nm)
-200
$R^2 = 0.7889$
-3
-2
-1
1
2
3
4
5
6
7
AKR1C3 RNA expression
(Log2 FPKM)
Fig.3

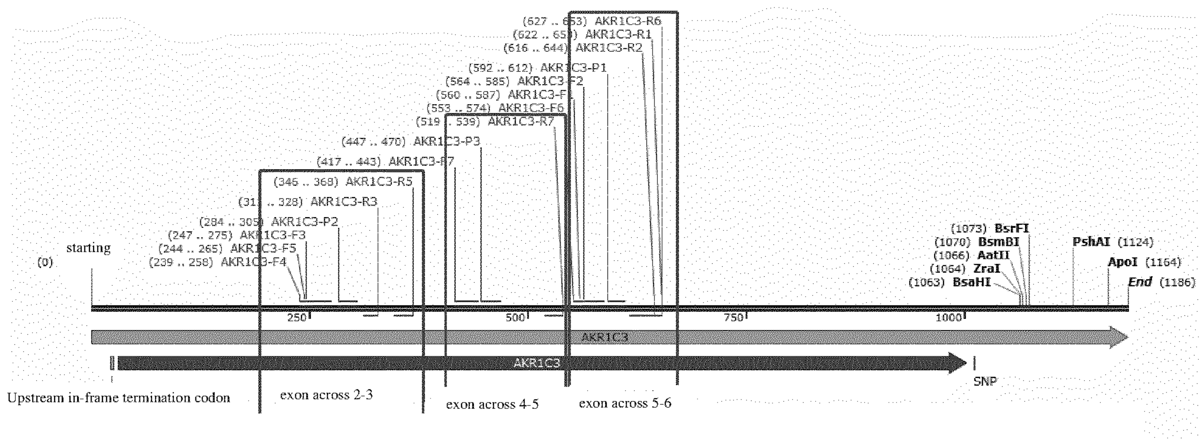
(627 .. 653) AKR1C3-R6
(622 .. 651) AKR1C3-R1
(616 .. 644) AKR1C3-R2
(592 .. 612) AKR1C3-P1
(564 .. 585) AKR1C3-F2
(560 .. 587) AKR1C3-F1
(553 .. 574) AKR1C3-F6
(519 .. 539) AKR1C3-R7
(447 .. 470) AKR1C3-P3
(417 .. 443) AKR1C3-F7
(346 .. 368) AKR1C3-R5
(311 .. 328) AKR1C3-R3
(284 .. 305) AKR1C3-P2
(247 .. 275) AKR1C3-F3
(244 .. 265) AKR1C3-F5
(239 .. 258) AKR1C3-F4
starting
(0)
(1073) BsrFI
(1070) BsmBI
(1066) AatII
(1064) ZraI
(1063) BsaHI
PshAI (1124)
ApoI (1164)
End (1186)
250
500
750
1000
AKR1C3
AKR1C3
SNP
Upstream in-frame termination codon
exon across 2-3
exon across 4-5
exon across 5-6
Fig.4

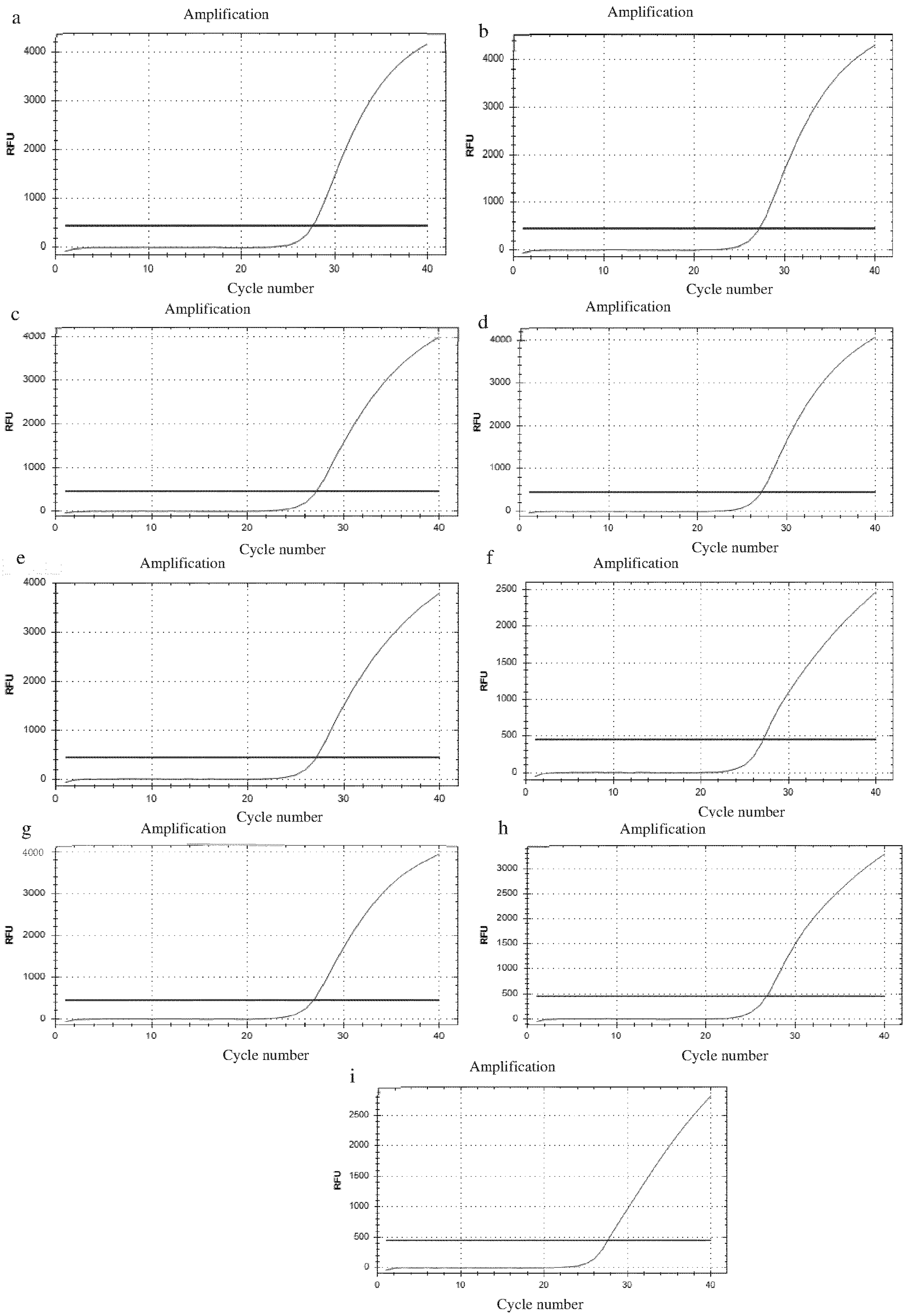
a
Amplification
RFU
Cycle number
b
Amplification
RFU
Cycle number
c
Amplification
RFU
Cycle number
d
Amplification
RFU
Cycle number
e
Amplification
RFU
Cycle number
f
Amplification
RFU
Cycle number
g
Amplification
RFU
Cycle number
h
Amplification
RFU
Cycle number
i
Amplification
RFU
Cycle number

Fig.5

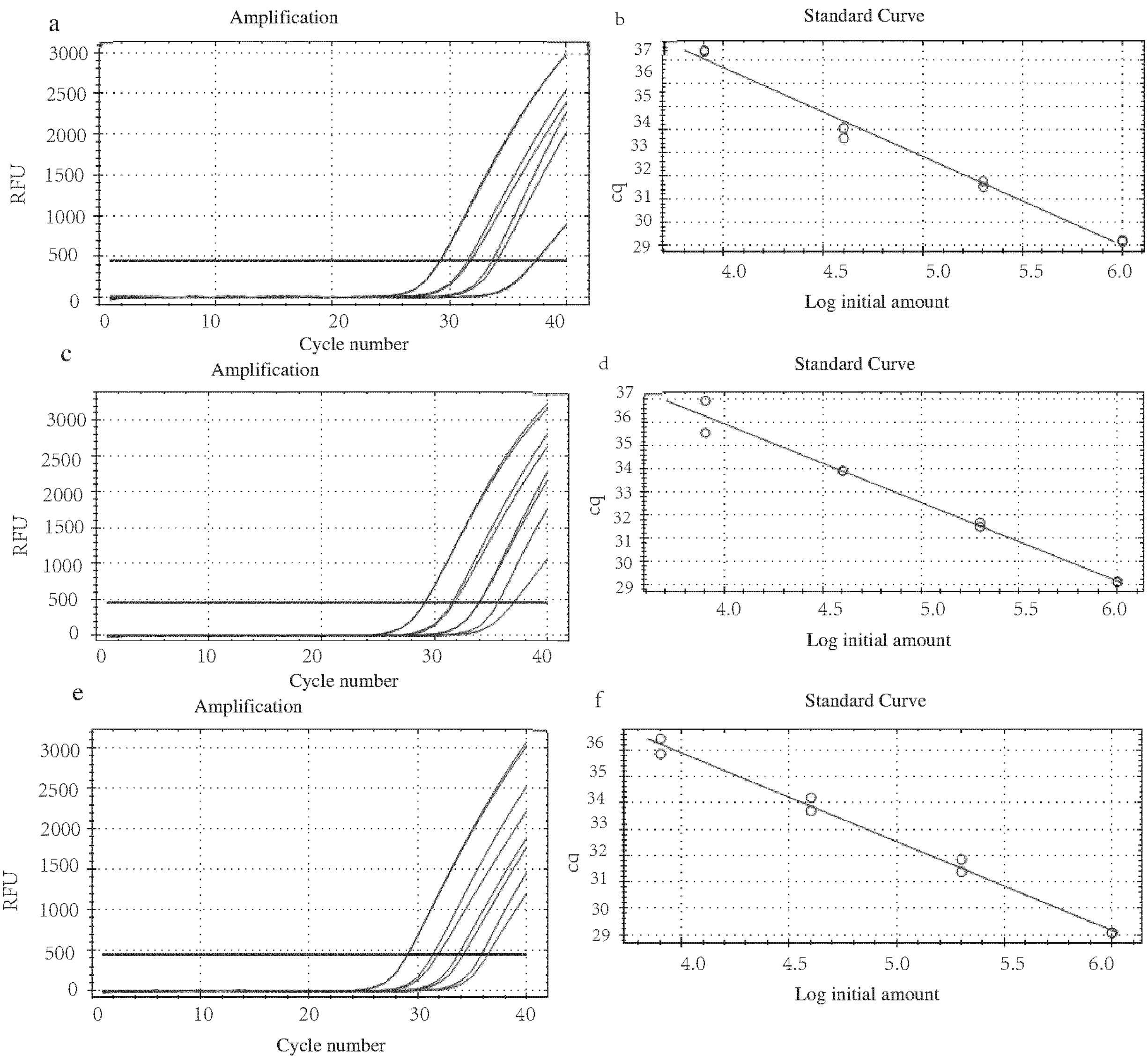
a
Amplification
RFU
3000
2500
2000
1500
1000
500
0
0
10
20
30
40
Cycle number
b
Standard Curve
cq
37
36
35
34
33
32
31
30
29
4.0
4.5
5.0
5.5
6.0
Log initial amount
c
Amplification
RFU
Cycle number
d
Standard Curve
cq
Log initial amount
e
Amplification
RFU
Cycle number
f
Standard Curve
cq
Log initial amount

Fig.6

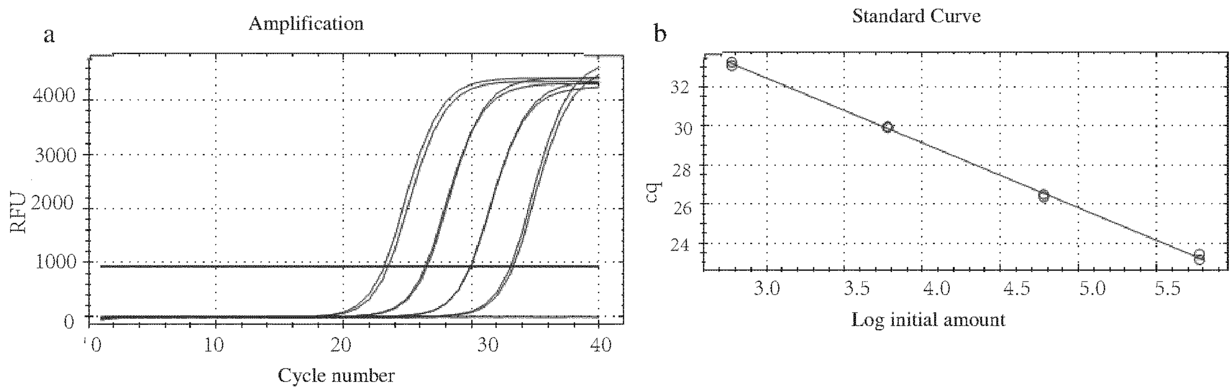
a
Amplification
RFU
4000
3000
2000
1000
0
0
10
20
30
40
Cycle number
b
Standard Curve
cq
32
30
28
26
24
3.0
3.5
4.0
4.5
5.0
5.5
Log initial amount

Fig.7

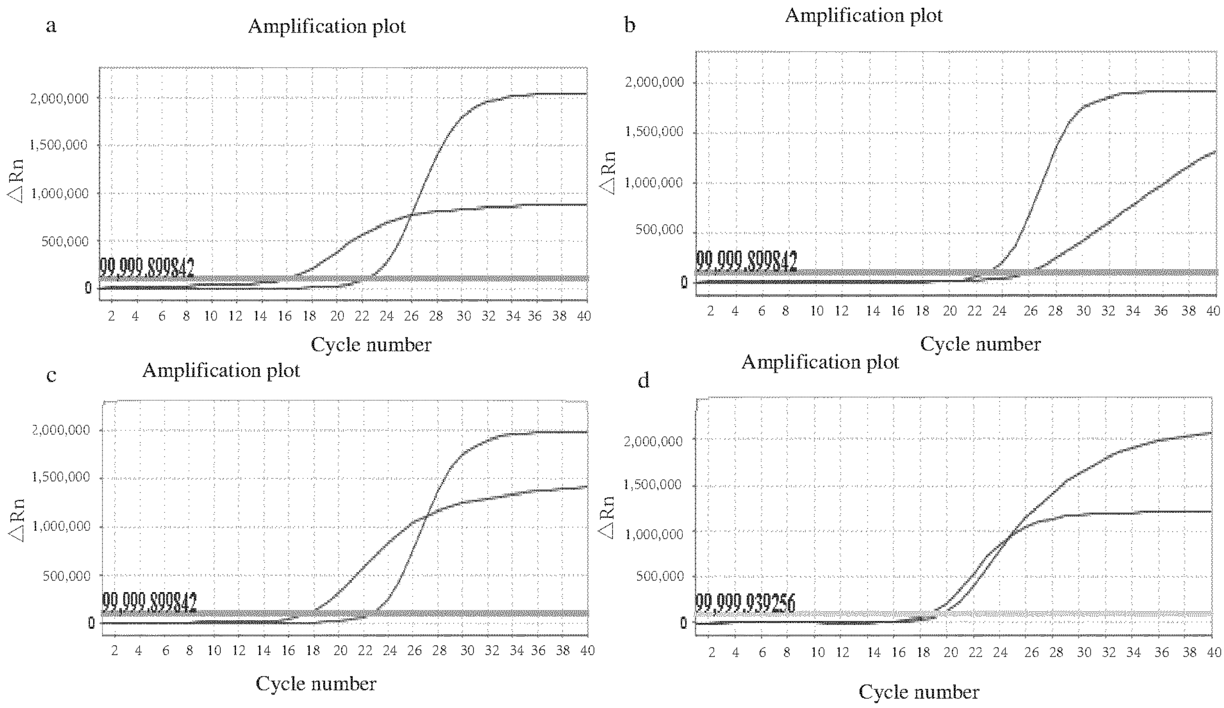
a
Amplification plot
△Rn
Cycle number
99,999.899842
b
Amplification plot
c
Amplification plot
d
Amplification plot
99,999.939256

Fig.8

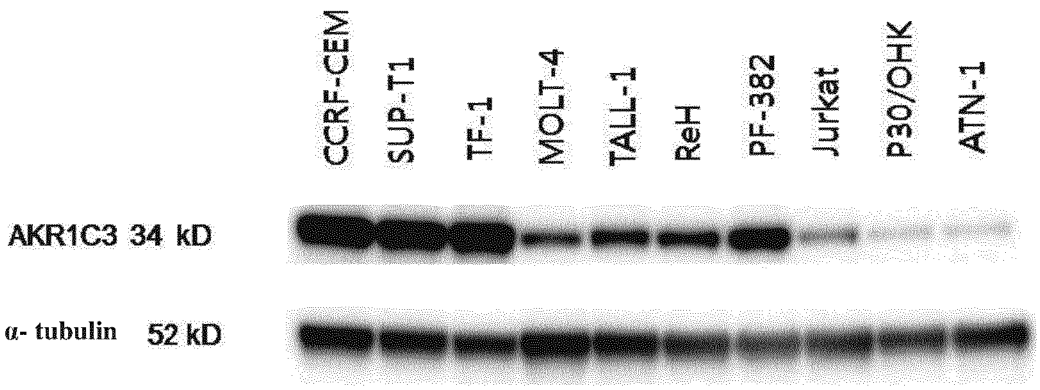
CCRF-CEM
SUP-T1
TF-1
MOLT-4
TALL-1
ReH
PF-382
Jurkat
P30/OHK
ATN-1
AKR1C3 34 kD
α- tubulin 52 kD

Fig.9

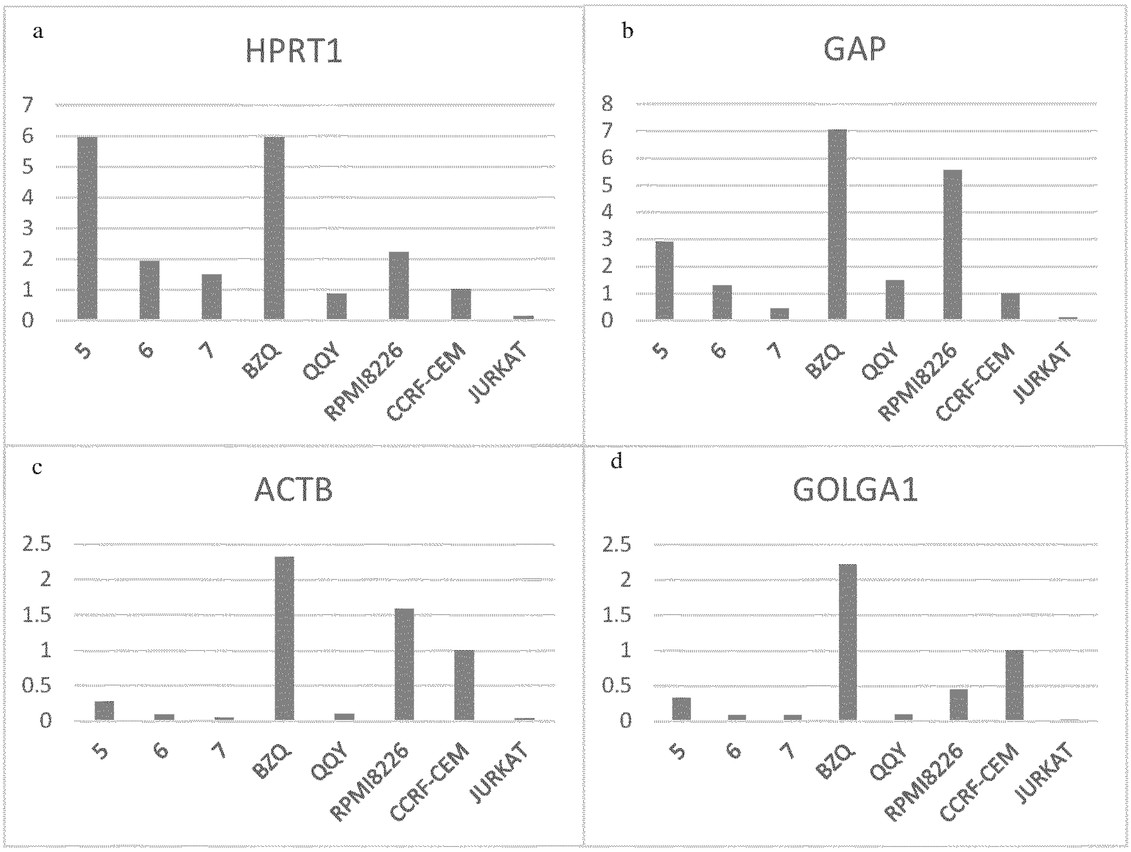
a
HPRT1
7
6
5
4
3
2
1
0
5
6
7
BZQ
QQY
RPMI8226
CCRF-CEM
JURKAT
b
GAP
8
7
6
5
4
3
2
1
0
5
6
7
BZQ
QQY
RPMI8226
CCRF-CEM
JURKAT
c
ACTB
2.5
2
1.5
1
0.5
0
5
6
7
BZQ
QQY
RPMI8226
CCRF-CEM
JURKAT
d
GOLGA1
2.5
2
1.5
1
0.5
0
5
6
7
BZQ
QQY
RPMI8226
CCRF-CEM
JURKAT

Fig.10

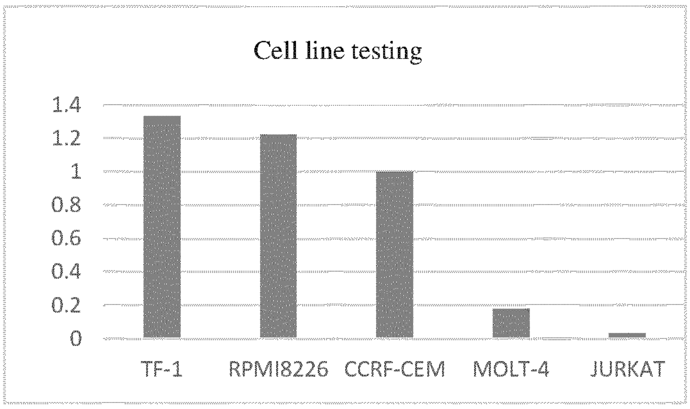
Cell line testing
1.4
1.2
1
0.8
0.6
0.4
0.2
0
TF-1
RPMI8226
CCRF-CEM
MOLT-4
JURKAT

Fig.11

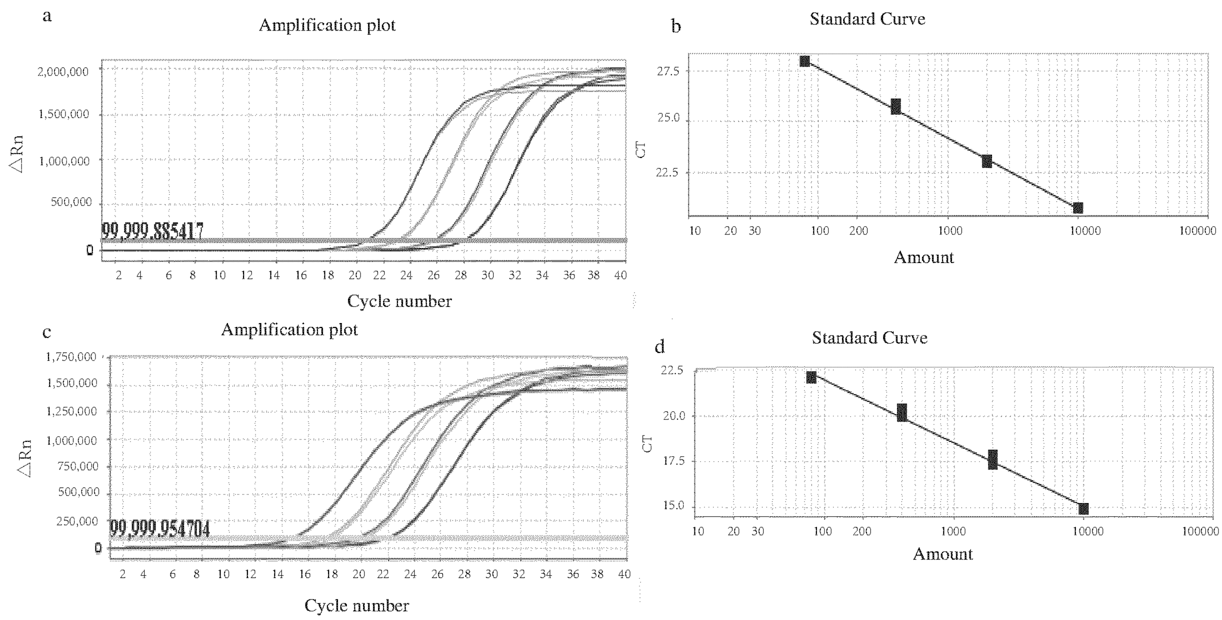
a
Amplification plot
ΔRn
Cycle number
99,999.885417
b
Standard Curve
CT
Amount
c
Amplification plot
ΔRn
Cycle number
99,999.954704
d
Standard Curve
CT
Amount

Fig.12

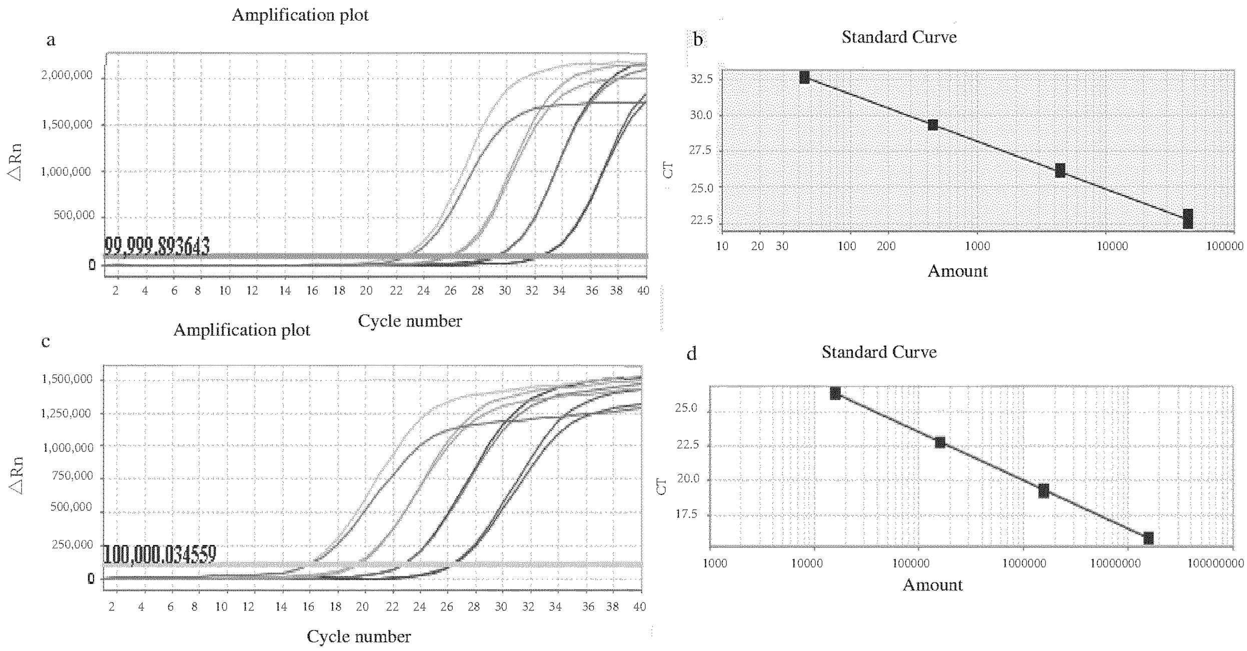
a
Amplification plot
ΔRn
Cycle number
99,999.893643
b
Standard Curve
CT
Amount
c
Amplification plot
ΔRn
Cycle number
100,000.034559
d
Standard Curve
CT
Amount

Fig.13

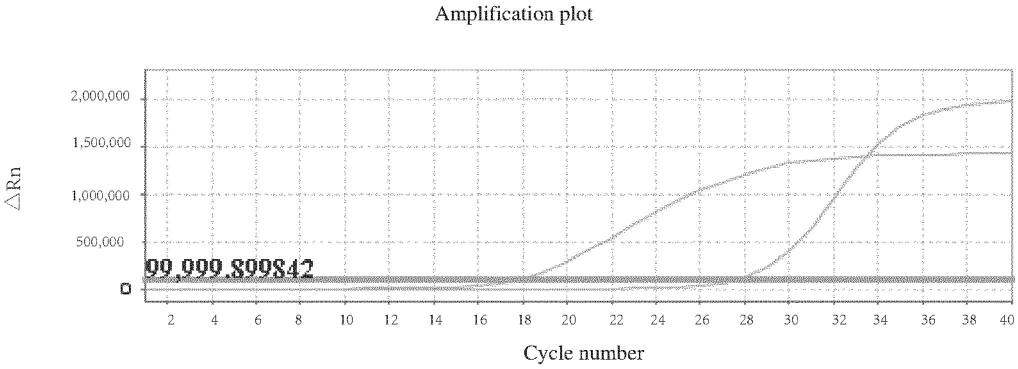
Amplification plot
2,000,000
1,500,000
1,000,000
500,000
0
99,999.899842
△Rn
2
4
6
8
10
12
14
16
18
20
22
24
26
28
30
32
34
36
38
40
Cycle number

Fig.14

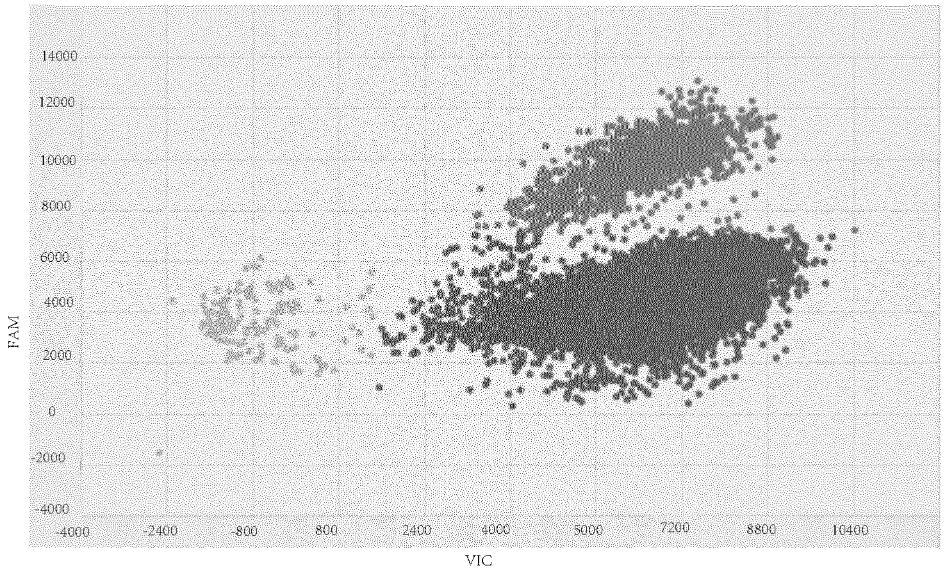
14000
12000
10000
8000
6000
4000
2000
0
-2000
-4000
FAM
-4000
-2400
-800
800
2400
4000
5000
7200
8800
10400
VIC

Fig.15

a

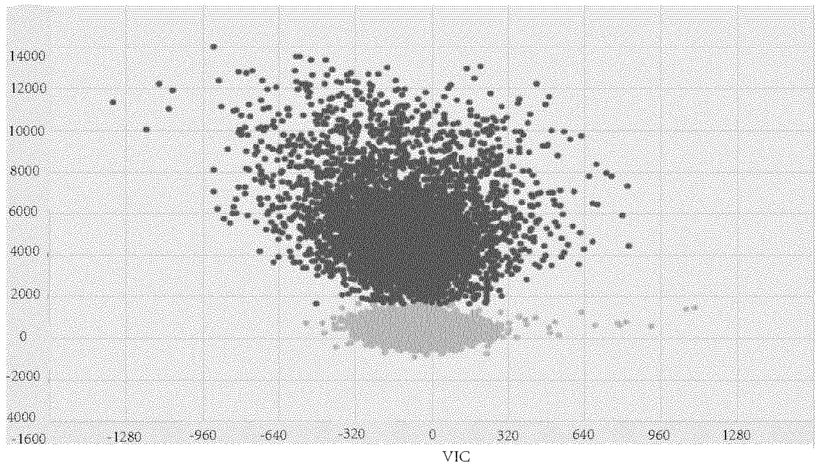
14000
12000
10000
8000
6000
4000
2000
0
-2000
4000
-1600
-1280
-960
-640
-320
0
320
640
960
1280
FAM
VIC

b

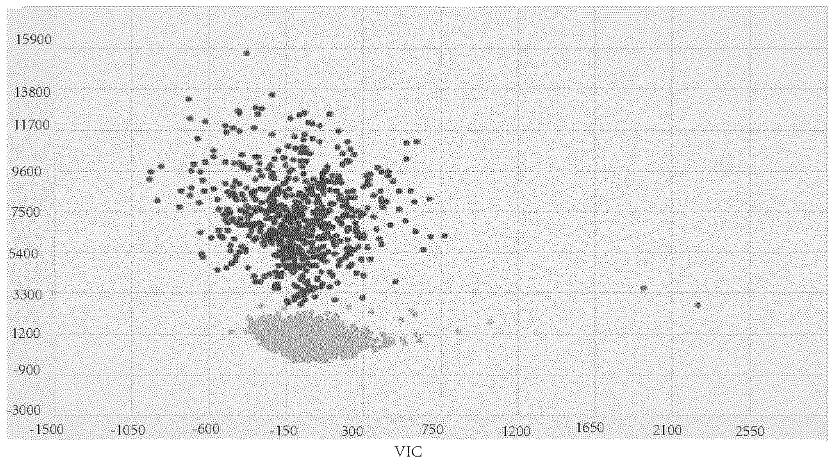
15900
13800
11700
9600
7500
5400
3300
1200
-900
-3000
-1500
-1050
-600
-150
300
750
1200
1650
2100
2550
FAM
VIC

Fig.16

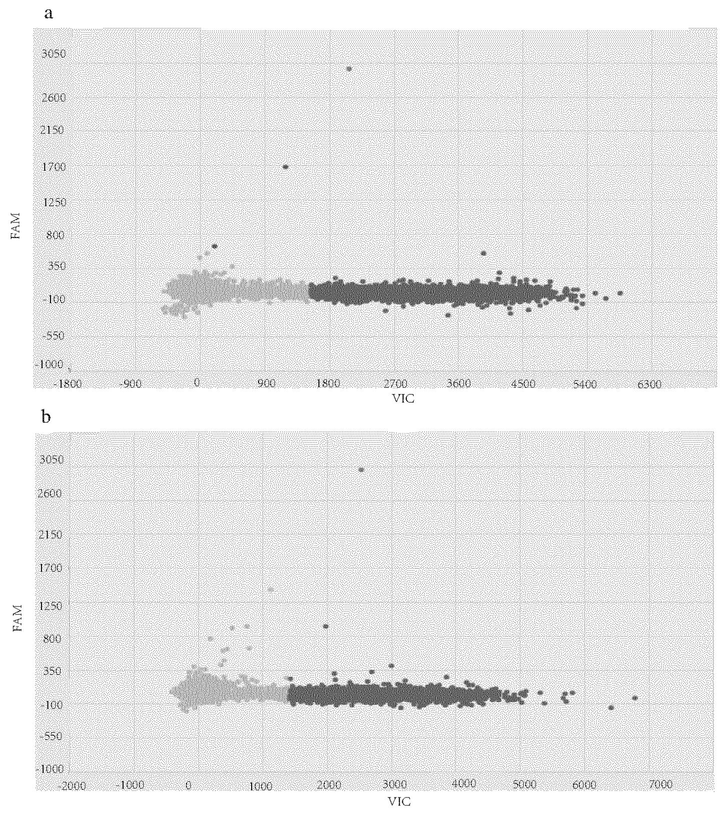
a
FAM
VIC
b
FAM
VIC

Fig.17

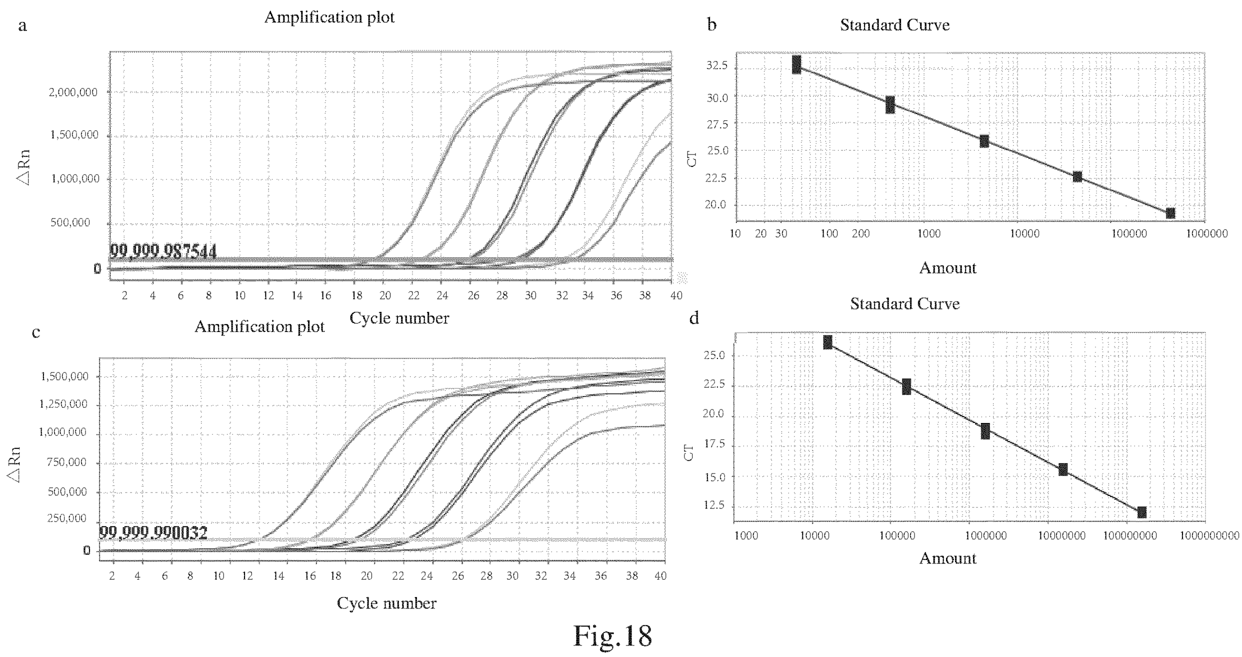
a
Amplification plot
△Rn
99,999.987544
Cycle number
b
Standard Curve
CT
Amount
c
Amplification plot
△Rn
99,999.990032
Cycle number
d
Standard Curve
CT
Amount

Fig.18

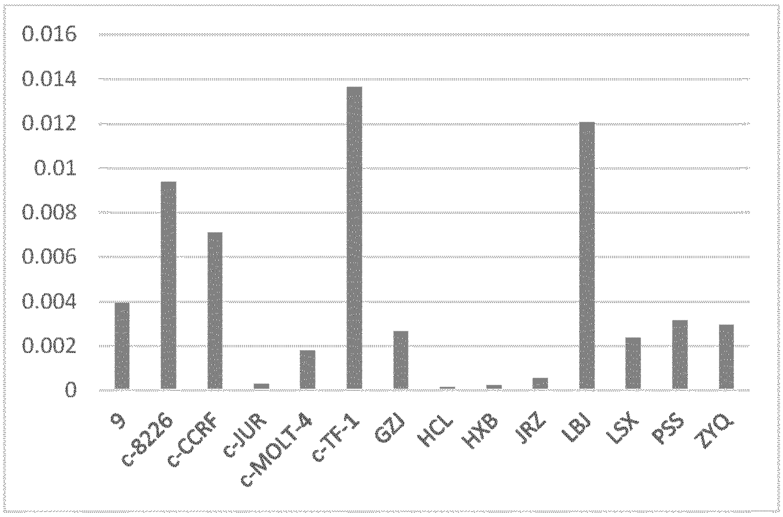
0.016
0.014
0.012
0.01
0.008
0.006
0.004
0.002
0
9
c-8226
c-CCRF
c-JUR
c-MOLT-4
c-TF-1
GZJ
HCL
HXB
JRZ
LBJ
LSX
PSS
ZYQ

Fig.19

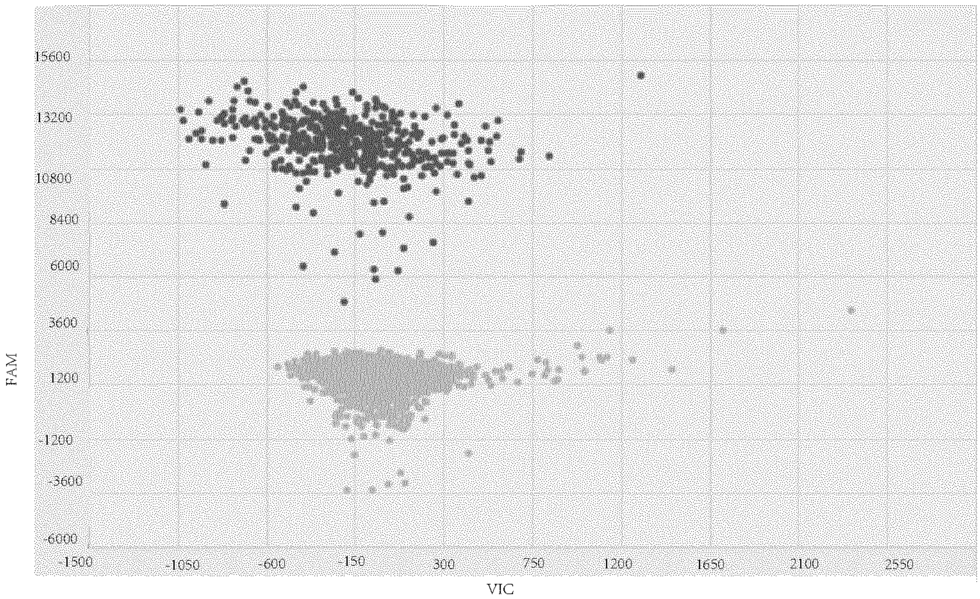
15600
13200
10800
8400
6000
3600
1200
-1200
-3600
-6000
FAM
-1500
-1050
-600
-150
300
750
1200
1650
2100
2550
VIC

Fig.20

**INTERNATIONAL SEARCH REPORT**

International application No. **PCT/CN2021/079299**

**A. CLASSIFICATION OF SUBJECT MATTER**

C12Q 1/6886(2018.01)i; G01N 33/68(2006.01)i; A61K 31/675(2006.01)i; A61P 35/00(2006.01)i

According to International Patent Classification (IPC) or to both national classification and IPC

**B. FIELDS SEARCHED**

Minimum documentation searched (classification system followed by classification symbols)

C12Q; G01N

Documentation searched other than minimum documentation to the extent that such documents are included in the fields searched

Electronic data base consulted during the international search (name of data base and, where practicable, search terms used)

CNABS, VEN(DWPI+SIPOABS), CNTXT, EPTXT, USTXT, WOTXT, CNKI, 百度学术搜索, BAIDU SCHOLAR SEARCH, ISI-WEB OF SCIENCE, Genbank or EMBL, 中国专利生物序列检索系统, Chinese Patent Biological Sequence Retrieval System: 引物, 探针, 深圳艾欣达伟, 肿瘤, AKR1C3, primers, probe, cancer, 对SEQ ID NOs:1-3和17-19的序列检索, sequence search for SEQ ID NOs:1-3 and 17-19

**C. DOCUMENTS CONSIDERED TO BE RELEVANT**

| Category* | Citation of document, with indication, where appropriate, of the relevant passages | Relevant to claim No. |
|---|---|---|
| X | CN 111876478 A (INSTITUTE OF MICROBIOLOGY, CHINESE ACADEMY OF SCIENCES) 03 November 2020 (2020-11-03)<br>see description, paragraphs 4-14 | 1 (in part), 3-11 (in part), 13-14 (in part), 16-26 (in part) |
| Y | CN 111876478 A (INSTITUTE OF MICROBIOLOGY, CHINESE ACADEMY OF SCIENCES) 03 November 2020 (2020-11-03)<br>see description, paragraphs 4-14 | 12 (in part), 15 (in part) |
| X | 陈宓等 (CHEN, Mi et al.). "非小细胞肺癌组织中AKR1C3 水平及其临床意义 (Expression of AKR1C3 and Clinical Significance in Non-small Cell Lung Cancer)"<br>*临床肿瘤学杂志 (Chinese Clinical Oncology),*<br>Vol. 22, No. 9, 30 September 2017 (2017-09-30),<br>pp. 787-791, see abstract, p. 788, left column, paragraph 1 to right column, paragraph 1 | 1 (in part), 3-11 (in part), 13-14 (in part), 16-26 (in part) |
| Y | 陈宓等 (CHEN, Mi et al.). "非小细胞肺癌组织中AKR1C3 水平及其临床意义 (Expression of AKR1C3 and Clinical Significance in Non-small Cell Lung Cancer)"<br>*临床肿瘤学杂志 (Chinese Clinical Oncology),*<br>Vol. 22, No. 9, 30 September 2017 (2017-09-30),<br>pp. 787-791, see abstract, p. 788, left column, paragraph 1 to right column, paragraph 1 | 12 (in part), 15 (in part) |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 November 2021** | **08 December 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/079299**

**C. DOCUMENTS CONSIDERED TO BE RELEVANT**

| Category* | Citation of document, with indication, where appropriate, of the relevant passages | Relevant to claim No. |
|---|---|---|
| X | SHIBA, M. et al. "Mefenamic acid enhances anticancer drug sensitivity via inhibition of aldo-keto reductase 1C enzyme activity" *ONCOLOGY REPORTS,* Vol. 37, 01 March 2017 (2017-03-01), pp. 2025-2032, see p. 2026, left column, paragraph 3 and p. 2028, table 1 | 1 (in part), 3-11 (in part), 13-14 (in part), 16-26 (in part) |
| Y | SHIBA, M. et al. "Mefenamic acid enhances anticancer drug sensitivity via inhibition of aldo-keto reductase 1C enzyme activity" *ONCOLOGY REPORTS,* Vol. 37, 01 March 2017 (2017-03-01), pp. 2025-2032, see p. 2026, left column, paragraph 3 and p. 2028, table 1 | 12 (in part), 15 (in part) |
| Y | CN 107530556 A (THRESHOLD PHARMACEUTICALS, INC.) 02 January 2018 (2018-01-02) see claims 2 and 14, and description paragraph 69 | 12 (in part), 15 (in part) |

Form PCT/ISA/210 (second sheet) (January 2015)

**INTERNATIONAL SEARCH REPORT**

International application No.
**PCT/CN2021/079299**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

INTERNATIONAL SEARCH REPORT

International application No. PCT/CN2021/079299

**Box No. III Observations where unity of invention is lacking (Continuation of item 3 of first sheet)**

This International Searching Authority found multiple inventions in this international application, as follows:

[1] Invention 1: claims 1 (in part) and 3-26 (in part) relate to a primer probe composition comprising an upstream primer AKR1C3-F1, a downstream primer AKR1C3-R1, and a probe AKR1C3-P1.

[2] Invention 2: claims 1 (in part) and 3-26 (in part) relate to a primer probe composition comprising an upstream primer AKR1C3-F2, a downstream primer AKR1C3-R2, and a probe AKR1C3-P1.

[3] Invention 3: claims 1-26 (in part) relate to a primer probe composition comprising an upstream primer AKR1C3-F2, a downstream primer AKR1C3-R6, and a probe AKR1C3-P1.

[4] Invention 4: claims 1-26 (in part) relate to a primer probe composition comprising an upstream primer AKR1C3-F6, a downstream primer AKR1C3-R2, and a probe AKR1C3-P1.

[5] Invention 5: claims 1-26 (in part) relate to a primer probe composition comprising an upstream primer AKR1C3-F6, a downstream primer AKR1C3-R6, and a probe AKR1C3-P1.

[6] Invention 6: claims 1 (in part) and 3-26 (in part) relate to a primer probe composition comprising an upstream primer AKR1C3-F5, a downstream primer AKR1C3-R5, and a probe AKR1C3-P2.

[7] Invention 7: claims 1 (in part) and 3-26 (in part) relate to a primer probe composition comprising an upstream primer AKR1C3-F3, a downstream primer AKR1C3-R3, and a probe AKR1C3-P1.

[8] Invention 8: claims 1 (in part) and 3-26 (in part) relate to a primer probe composition comprising an upstream primer AKR1C3-F4, a downstream primer AKR1C3-R3, and a probe AKR1C3-P2.

[9] Invention 9: claims 1 (in part) and 3-26 (in part) relate to a primer probe composition comprising an upstream primer AKR1C3-F7, a downstream primer AKR1C3-R7, and a probe AKR1C3-P3.

[10] These primers or probes have different sequences and do not have a common structure, and therefore, the same or corresponding technical feature among these inventions is the primer probe composition used for amplification of AKR1C3. However, WO2006092610A2 (08 September 2006 (08.09.2006)) discloses a melanoma marker and a diangostic method for a melanoma marker. In this method, the expression level of AKR1C3 is detected using RT-PCR (see description, p. 2, last paragraph and p. 7, last paragraph). A person skilled in the art would easily conceive of using AKR1C3 as a target to design appropriate primers and probes to perform detection, so as to diagnose whether melanoma is present. Therefore, the described same or corresponding technical feature cannot constitute a special technical feature that makes a contribution over the prior art. The nine inventions comprised in the present application do not belong to a single general concept and do not comply with PCT Rule 13.1.

INTERNATIONAL SEARCH REPORT

International application No.
**PCT/CN2021/079299**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1 (in part) and 3-26 (in part), relating to a primer probe composition comprising an upstream primer AKR1C3-F1, a downstream primer AKR1C3-R1, and a probe AKR1C3-P1.**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/CN2021/079299**

| Patent document cited in search report | | | Publication date (day/month/year) | Patent family member(s) | | | Publication date (day/month/year) |
|---|---|---|---|---|---|---|---|
| CN | 111876478 | A | 03 November 2020 | None | | | |
| CN | 107530556 | A | 02 January 2018 | SG | 11201707293V | A | 30 October 2017 |
| | | | | EP | 3277380 | A4 | 01 August 2018 |
| | | | | JP | 6704421 | B2 | 03 June 2020 |
| | | | | KR | 20190072688 | A | 25 June 2019 |
| | | | | KR | 102034618 | B1 | 21 October 2019 |
| | | | | SG | 10201913462 X | A | 30 March 2020 |
| | | | | WO | 2016145092 | A1 | 15 September 2016 |
| | | | | AU | 2016229136 | B2 | 09 August 2018 |
| | | | | ES | 2828026 | T3 | 25 May 2021 |
| | | | | EP | 3747508 | A1 | 09 December 2020 |
| | | | | CN | 107530556 | B | 10 April 2020 |
| | | | | KR | 20170128280 | A | 22 November 2017 |
| | | | | US | 10364261 | B2 | 30 July 2019 |
| | | | | EP | 3277380 | B1 | 16 September 2020 |
| | | | | IL | 254377 | D0 | 30 November 2017 |
| | | | | KR | 20190072689 | A | 25 June 2019 |
| | | | | BR | 112017019287 | A2 | 02 May 2018 |
| | | | | JP | 2018513876 | A | 31 May 2018 |
| | | | | AU | 2016229136 | A1 | 05 October 2017 |
| | | | | US | 2019225633 | A1 | 25 July 2019 |
| | | | | TW | 201706262 | A | 16 February 2017 |
| | | | | CA | 2979251 | C | 23 March 2021 |
| | | | | US | 2018044360 | A1 | 15 February 2018 |
| | | | | CA | 2979251 | A1 | 15 September 2016 |
| | | | | EP | 3277380 | A1 | 07 February 2018 |
| | | | | KR | 102189066 | B1 | 09 December 2020 |
| | | | | US | 10766914 | B2 | 08 September 2020 |
| | | | | TW | I674258 | B | 11 October 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2016021581 W
- WO 2016145092 A1
- CN 2016800150788
- CN 107530556 A
- US 2016025665 W
- WO 2016161342 A
- CN 2016800446081
- CN 108290911 A
- US 2016062114 W
- WO 2017087428 A
- CN 2016800200132
- CN 108136214 A
- NZ 2019050030 W
- WO 2019190331 A
- CN 201980023423
- CN 111918864 A
- WO 2019062919 A1
- WO 2018123764 A1
- WO 2017087428 A1

### Non-patent literature cited in the description


- **NOGRADY.** Medicinal Chemistry A Biochemical Approach. Oxford University Press, 1985, 388-392
- The Selection Of Reference Gene in Real-time Quantitative PCR [J. **DONG ENNI ; LIANG QING ; LI LI et al.** China Animal Industry. vol. 49, 92-96
- **ZHAO WEN-JING ; XU JIE ; BAO QIUHUA et al.** Selection of Reference Genes for Real-time Quantitative PCR [J. *Microbiology China,* 2010, (12), 1825-1829
- **WU, X. ; BLACKBURN, P. ; TSCHUMPER, R. et al.** TALEN-mediated genetic tailoring as a tool to analyze the function of acquired mutations in multiple myeloma cells. *Blood Cancer Journal,* 2014, vol. 4, e210, https://doi.org/10.1038/bcj.2014.32
- **WERNER KEMPF ; MARSHALL E. KADIN ; ANN M. DVORAK ; CAROL C. LORD ; GÜNTER BURG ; NORMAN L. LETVIN ; IGOR J. KORALNIK.** Endogenous retroviral elements, but not exogenous retroviruses, are detected in CD30-positive lymphoproliferative disorders of the skin. *Carcinogenesis,* 02 February 2003, vol. 24, 301-306, https://doi.org/10.1093/carcin/24.2.301.
- **STECKER C ; JOHANN A ; HERZBERG C ; AVERHOFF B ; GOTTSCHALK G.** Complete nucleotide sequence and genetic organization of the 210-kilobase linear plasmid of Rhodococcus erythropolis BD2. *J Bacteriol.,* 2003, vol. 185 (17), 5269-5274
- **LOCK RB ; LIEM N ; FARNSWORTH ML ; MILROSS CG ; XUE C ; TAJBAKHSH M et al.** The non-obese diabetic/severe combined immunodeficient (NOD/SCID) mouse model of childhood acute lymphoblastic leukemia reveals intrinsic differences in biologic characteristics at diagnosis and relapse. *Blood,* 2002, vol. 99, 4100-8
- **JAMIESON SM ; GU Y ; MANESH DM ; EL-HOSS J ; JING D ; MACKENZIE KL et al.** A novel fluorometric assay for aldo-keto reductase 1C3 predicts metabolic activation of the nitrogen mustard prodrug PR-104A in human leukaemia cells. *Biochem Pharmacol,* 2014, vol. 88, 36-45
- **LOCK RB ; LIEM N ; FARNSWORTH ML ; MILROSS CG ; XUE C ; TAJBAKHSH M et al.** The non-obese diabetic/severe combined immunodeficient (NOD/SCID) mouse model of childhood acute lymphoblastic leukemia reveals intrinsic differences in biologic characteristics at diagnosis and relapse. *Blood,* 2002, vol. 99, 4100-8
- **DOBIN A ; DAVIS CA ; SCHLESINGER F ; DRENKOW J ; ZALESKI C ; JHA S et al.** STAR: ultrafast universal RNA-seq aligner. *Bioinformatics,* 2013, vol. 29, 15-21
- **ANDERS S ; PYL PT ; HUBER W.** HTSeq-a Python framework to work with highthroughput sequencing data. *Bioinformatics,* 2015, vol. 31, 166-9
- *Genome Biol,* 2010, vol. 11, R106
- **YANLAN WANG ; YUE LIU ; CHANGHUA ZHOU et al.** An AKR1C3-specific prodrug with potent antitumor activities against T-ALL. [J. *Leukemia&Lymphoma,* February 2020

- **DONYA MORADI MANESH ; JAD EL-HOSS ; KATHRYN EVANS et al.** Growth factor AKR1C3 is a biomarker of sensitivity to PR-104 in preclinical models of T-cell acute lymphoblastic leukemiaas treatment targets in clinical oncology.[J]. *BLOOD,* 2015, vol. 125, 1193-1201
- **YUANTONG TIAN ; LIJING ZHAO ; HAITAO ZHANG et al.** AKR1C3 overexpression may serve as a promising biomarker for prostate cancer progression. *Diagnostic Pathology,* 2014, vol. 9, 42